# EUROPEAN PATENT SPECIFICATION

(11) **EP 2 433 124 B1**
(45) Date of publication and mention of the grant of the patent: **01.03.2017**
(21) Application number: 10721223.5
(22) Date of filing: 19.05.2010
(51) Int. Cl.: G01N 33/50

(54) **METHODS FOR PROVIDING PERSONALIZED MEDICINE TESTS EX VIVO FOR HEMATOLOGICAL NEOPLASMS**
VERFAHREN FÜR PERSONALISIERTE MEDIZINISCHE EX-VIVO-TESTS FÜR HÄMATOLOGISCHE NEOPLASMEN
PROCÉDÉS PERMETTANT DE FOURNIR DES ESSAIS DE MÉDICAMENTS PERSONNALISÉS EX VIVO POUR DES TUMEURS HÉMATOLOGIQUES

(30) Priority: 19.05.2009 US 179685 P
(43) Date of publication of application: 28.03.2012
(73) Proprietor: Vivia Biotech S.L., 28760 Tres Cantos (Madrid) (ES)
(72) Inventor: BALLESTEROS, Juan, Tres Cantos 28760 (ES); BENNETT, Teresa, 28760 Tres Cantos (ES); PRIMO, Daniel, 28760 Tres Cantos (ES); ORFAO, Alberto, 28760 Tres Cantos (ES); JACKSON, Coyt, 28760 Tres Cantos (ES); LAGO, Santiago, 28760 Tres Cantos (ES); MATOSES, Maria, 28760 Tres Cantos (ES); SUAREZ, Lilia, 28760 Tres Cantos (ES); SAPIA, Sandra, 28760 Tres Cantos (ES); BOSANQUET, Andrew, 28760 res Cantos (ES); GORROCHATEGUI, Julian, 28760 Tres Cantos (ES); TUDELA, Consuelo, 28760 Tres Cantos (ES); HERNANDEZ, Pilar, 28760 Tres Cantos (ES); CAVEDA, Luis Ignacio, 28760 Tres Cantos (ES)
(74) Representative: Illescas, Manuel
(86) International application number: PCT/US2010/035474
(87) International publication number: WO 2010/135468

(56) References cited:
- WO-A1-89/06162
- WO-A1-93/25705
- WO-A1-96/22085
- WO-A2-2006/031867
- WO-A2-2007/015926
- WO-A2-2010/058974
- US-A- 5 474 909
- US-B1- 6 448 030
- BENNETT T ET AL: "DEVELOPMENT OF A PERSONALIZED MEDICINE TEST FOR HAEMATOLOGICAL MALIGNANCIES" HAEMATOLOGICA-THE HEMATOLOGY JOURNAL, vol. 94, no. Suppl. 2, June 2009 (2009-06) , pages 233-234 URL, XP002594677 & 14TH ANNUAL MEETING OF THE EUROPEAN-HEMATOLOGY-ASSOCIATION; BERLIN, GERMANY; JUNE 04 -07, 2009
- BENNETT T ET AL: "UTILIZING CELL-BASED SCREENING TO IDENTIFY NOVEL USES FOR EXISTING DRUGS: INDUCING SPECIFIC APOPTOSIS OF B-CELL LYMPHOCYTES" HAEMATOLOGICA-THE HEMATOLOGY JOURNAL, vol. 94, no. Suppl. 2, June 2009 (2009-06) , pages 67-68 URL, XP002594678 & 14TH ANNUAL MEETING OF THE EUROPEAN-HEMATOLOGY-ASSOCIATION; BERLIN, GERMANY; JUNE 04 -07, 2009
- BENNETT TERESA A ET AL: "Personalized Medicine Test of Multi-Drug Protocols Ex Vivo for Hematological Malignancies." BLOOD, vol. 114, no. 22, November 2009 (2009-11), page 1041, XP002594679 & 51ST ANNUAL MEETING OF THE AMERICAN-SOCIETY-OF-HEMATOLOGY; NEW ORLEANS, LA, USA; DECEMBER 05 -08, 2009
- Andrew G. Bosanquet ET AL: "Drug cross-resistance and therapy-induced resistance in chronic lymphocytic leukaemia by an enhanced method of individualised tumour response testing", British Journal of Haematology, vol. 146, no. 4, 1 August 2009 (2009-08-01), pages 384-395, XP055210037, ISSN: 0007-1048, DOI: 10.1111/j.1365-2141.2009.07741.x
- Andrew G Bosanquet ET AL: "ENHANCED EX Vivo DRUG SENSITIVITY TESTING OF CHRONIC LYMPHOCYTIC LEUKAEMIA USING REFINED DiSC ASSAY METHODOLOGY", Leukemia Research Vol 20, 1 January 1996 (1996-01-01), pages 143-153, XP055210039, Retrieved from the Internet: URL:http://ac.els-cdn.com/0145212695001271 /1-s2.0-0145212695001271-main.pdf?_tid=051 25dfe-4d5d-11e5-8da3-00000aacb362&acdnat=1 440749941_71463f085a622db5a65b5f434fdca969 [retrieved on 2015-08-28]
- WEISENTHAL L M ET AL: "A novel dye exclusion method for testing in vitro chemosensitivity of human tumors", CANCER RESEARCH, AMERICAN ASSOCIATION FOR CANCER RESEARCH, US, vol. 43, no. 2, 1 January 1983 (1983-01-01), pages 749-757, XP009158589, ISSN: 0008-5472

## Description

### BACKGROUND OF THE INVENTION

This invention relates to the use of a screening platform to determine a cytotoxic drug sensitivity profile for multiple drugs and drug combinations using specimens from cancer patients. Described herein is a cell-based screening platform that incorporates both automated sample preparation and automated evaluation by flow cytometry that is useful as a personalized medicine test because of its rapid data acquisition, analysis, and reporting of results, even from very large numbers of drugs and drug combinations. Also disclosed are particular combinations of drugs useful in the treatment of proliferative lymphoid disease.

### Description of the Related Art

There are many methods available to evaluate the cytotoxic drug sensitivity profiles of tumor cells in ex vivo samples taken from cancer patients. Ex vivo assays for detecting cell death in hematological neoplasms have been developed during the past 40 years, resulting in a number of assays to identify chemosensitivity. The term Individualized Tumor Response Test/Testing (ITRT) has recently been proposed for these methods to describe the "effect of anticancer treatments on whole living tumor cells freshly removed from cancer patients." (Bosanquet et al., G. J. Kaspers, B. Coiffier, M. C. Heinrich and E. H. Estey. New York, NY, 2008, Informa Healthcare: 23-44). Initial ITRTs designed to study the ability of a drug to slow or arrest neoplastic cell growth (e.g., clonogenic assays) did not work well. However, in the 1980s, a number of ITRTs of cell death were developed that have consistently shown good comparisons between assay results and clinical outcomes (*i*.*e*., clinical correlations).

Even with good clinical correlations, currently available ITRTs of cell death suffer from undesirable limitations that restrict their use as personalized medicine tests. For example, clonogenic assays generally require weeks rather than days to generate results, restricting their clinical usefulness (Hamburger et al., Science 1977, 197:461-463; Marie et al., Br J Haematol 1983, 55:427-437; Selby et al., New Engl J Med 1983, 308:129-134). Also, the majority of ITRTs measure total cell death to evaluate the effect of incubating samples with drugs ex vivo. Measuring total cell death limits the ability of an ITRT to distinguish between a drug's effect on tumor cells versus normal cells. The ITRTs that are currently available differ from one another mainly with respect to the methodology used to determine the percentage of live cells or live tumor cells at the end of an assay.

While some ITRTs measure cells directly, the majority evaluate cell death indirectly using surrogate markers. For example, the MTT (methyl-thiazolyl tetrazolium) assay estimates the number of live cells by measuring mitochondrial reduction of MTT to formazan, eliciting a change in color that can be quantified using a spectrophotometer (Pieters et al., Blood 1990, 76:2327-2336; Sargent et al., Br J Cancer 1989, 60:206-210; Carmichael et al., Cancer Res 1987, 47:936-942). Other ITRTs use fluorescein diacetate hydrolysis (*e*.*g*., the fluorometric microculture cytotoxicity assay (FMCA)) or cellular ATP levels as indirect markers of cellular viability (Rhedin et al., Leuk Res 1993, 17:271-276; Larsson et al., Int J Cancer 1992, 50:177-185). The DiSC (Differential Staining Cytotoxicity) assay and more recently, the TRAC (Tumor Response to Antineoplastic Compounds) assay use staining methods to determine live tumor cells by microscopy (Bosanquet et al., Br J Haematol 2009, 146:384-395; Bosanquet et al., Leuk Res 1996, 20:143-153; Weisenthal et al., Cancer Res 1983, 43:749-757).

The above-mentioned ITRTs require incubation of a patient's neoplastic cells with cytotoxic drugs for a period of at least 4 to 5 days. However, hematological cells start to lose important properties after only 24 to 48 hours outside the human body. Shorter incubation periods would allow for the evaluation of ex vivo cytotoxicity profiles prior to the start of patient treatment, thereby increasing their clinical utility and allowing for a more effective application as personalized medicine tests.

Cytotoxic drugs have been shown to eliminate malignant cells by inducing apoptosis (Aragane et al., J Cell Biol 1998, 140:171-182; Hannun et al., Blood 1997, 89:1845-1853). Apoptosis is a type of cellular death, commonly referred to in the art as "programmed cell death," which the art defines according to morphological and antigenic features. Apoptosis commonly starts within hours of a drug coming into contact with target cells (Del Bino et al., Cell Prolif 1999, 32:25-37). There are many assays for apoptosis based on markers that reflect different aspects of the apoptotic process, such as: 1) changes in the mitochondrial potential membrane using DiOC6 or JC-1 (Tabrizi et al., Leukemia 2002, 16:1154-1159; Liu et al., Leukemia 2002, 16:223-232); 2) fragmentation of internucleosomic DNA identified by Tdt in the terminal deoxynucleotidyl transferase (TUNEL) assay (Liu et al., Leukemia 2002, 16:223-232) using electrophoresis or labeling with acridine orange (Tabrizi et al., Leukemia 2002, 16(6):1154-9; Kim et al., Exp Mol Med 2000, 32:197-203; Konstantinov et al., J Cancer Res Clin Oncol 2002, 128:271-278; Ofir et al., Cell Death Differ 2002, 9:636-642); or 3) identification of proteolytic fragments of either poly-ADP-ribose polymerase (PARP) or caspase-3 using specific antibodies (Konstantinov et al., J Cancer Res Clin Oncol 2002, 128(5):271-8; Ofir et al., Cell Death Differ 2002, 9(6):636-42; Byrd et al., Blood 2002, 99:1038-1043; Hasenjäger et al., Oncogene 2004, 23:4523-4535; Prokop et al., Oncogene 2003, 22:9107-9120).

Another assay of apoptosis is based on the detection by flow cytometry of Annexin V conjugated to a fluorescent marker (*i*.*e*., a fluorochrome). Annexin V binds to externalized phosphatidylserine residues that only appear on the surface membrane of cells undergoing apoptosis (Tabrizi et al., Leukemia 2002, 16(6):1154-9; Nimmanapalli et al., Cancer Res 2002, 62:5761-5769). The measurement of apoptosis can be evaluated according to the percentage of cells that bind an Annexin V-fluorescent conjugate, as detected by flow cytometry. Additionally, several monoclonal antibody combinations that are used for the identification of tumor cells (versus normal cells) are known in the art. Table 1 summarizes various monoclonal antibody combinations that, when conjugated to a fluorochrome, could be used to identify hematological tumor cells using various spectroscopic detection methods.

**Table 1: Monoclonal Antibody Combinations for Tumor Cell Identification**

| **Hematological Neoplasm** | **AcM-Fluorochrome Conjugate** |
|---|---|
| ALL, CLL, NHL | CD19-PE, CD45-APC |
| MM | CD38-PE, CD45-APC |
| AML | CD34-PE, CD45-APC |

| | |
|---|---|
| ALL = Acute Lymphocytic Leukemia; CLL = Chronic Lymphocytic Leukemia; NHL = Non-Hodgkin's Lymphoma; MM = Multiple Myeloma; AML = Acute Myeloblastic Leukemia | |

Some ITRTs, particularly the DiSC and TRAC assays, allow for the simultaneous measurement of cytotoxicity in tumor cells and normal cells, allowing for the determination of a therapeutic index (Bosanquet et al., Leuk. Res. 1996; 20: 143-53; Bosanquet et al., J Exp Ther Oncol 2004; 4: 145-54).

Researchers have shown the predictive capacity of ITRTs in several scientific reviews. A review of 1929 clinical correlations in hematological malignancies (Bosanquet et al. in Kaspers et al. (eds.), 2008) and other reviews (e.g., Kaspers GJ., Methods Mol Med. 2005; 110:49-57) indicate a high percentage of positive predictive efficacy, particularly with respect to drug resistance. Integrating results from multiple articles (Table 2), Nagourney found the positive predictive efficacy with respect to drug sensitivity was 81.8%, and the negative predictive efficacy with respect to drug resistance was 83.3% (adapted from http://www.rationaltherapeutics.com/physicians/contentl.aspx?rid= 35 and bibliographic references therein (visited 7 May 2010)).

**Table 2: List of Published Clinical Correlations that Support the Predictive Capacity of ITRTs**

| **Hematological Cancer** | **N** | **TP** | **TN** | **FP** | **FN** | **Ref.** |
|---|---|---|---|---|---|---|
| ALL | 3 | 2 | 1 | 0 | 0 | 1 |
| ALL | 17 | 14 | 2 | 1 | 0 | 2 |
| ALL | 25 | 16 | 3 | 5 | 1 | 3 |
| ALL | 130 | 90 | 18 | 20 | 2 | 4 |
| ALL | 58 | 40 | 6 | 0 | 2 | 5 |
| ALL | 4 | 1 | 2 | 1 | 0 | 6 |
| ALL | 4 | 3 | 1 | 0 | 0 | 7 |
| ALL | 29 | 18 | 5 | 2 | 4 | 8 |
| ALL | 2 | 2 | 0 | 0 | 0 | 9 |
| ALL/CLL | 55 | 38 | 7 | 10 | 0 | 10 |
| AML | 4 | 0 | 1 | 2 | 1 | 2 |
| AML | 11 | 6 | 5 | 0 | 0 | 11 |
| AML | 21 | 11 | 8 | 2 | 0 | 6 |
| AML | 83 | 74 | 9 | 0 | 0 | 12 |
| AML | 27 | 6 | 13 | 0 | 8 | 13 |
| AML | 21 | 10 | 9 | 2 | 0 | 14 |
| AML | 33 | 11 | 8 | 4 | 10 | 15 |
| AML /ALL/ NHL | 73 | 45 | 16 | 9 | 3 | 16 |
| AML | 12 | 7 | 3 | 2 | 0 | 17 |
| AML | 14 | 9 | 1 | 2 | 2 | 3 |
| AML | 14 | 9 | 2 | 1 | 2 | 4 |
| AML | 17 | 11 | 4 | 1 | 1 | 7 |
| AML | 27 | 12 | 12 | 2 | 1 | 18 |
| AML | 34 | 20 | 11 | 2 | 1 | 19 |
| CLL | 80 | 12 | 48 | 18 | 2 | 2 |
| CLL | 34 | 26 | 6 | 2 | 0 | 20 |
| CLL | 1 | 1 | 0 | 0 | 0 | 6 |
| CLL | 15 | 11 | 3 | 0 | 1 | 21 |
| CLL | 15 | 9 | 4 | 1 | 1 | 8 |
| CLL | 3 | 2 | 1 | 0 | 0 | 9 |
| CLL/ALL/NHL | 226 | 102 | 76 | 41 | 7 | 22 |
| CLL (blastic) | 9 | 2 | 6 | 1 | 0 | 8 |
| NHL | 1 | 1 | 0 | 0 | 0 | 17 |
| NHL | 10 | 3 | 3 | 3 | 1 | 2 |
| NHL | 3 | 2 | 0 | 1 | 0 | 1 |
| NHL | 50 | 27 | 10 | 11 | 2 | 23 |
| NHL | 10 | 6 | 3 | 1 | 0 | 8 |
| NHL | 3 | 0 | 3 | 0 | 0 | 9 |
| **Total** | **1178** | **659** | **310** | **147** | **62** | |

| | | | | | | |
|---|---|---|---|---|---|---|
| N = number of cases; TP = True Positives; TN = True Negatives; FP = False Positives; FN = False Negatives; Ref. = Bibliography (see References at end of specification); ALL = Acute Lymphocytic Leukemia; CLL = Chronic Lymphocytic Leukemia; AML = Acute Myeloblastic Leukemia; NHL = Non-Hodgkin's Lymphoma | | | | | | |

A prospective randomized controlled clinical trial is currently being conducted in the United Kingdom using a large number of chronic lymphocytic leukemia patients (UK LRF CLL4 trial, Catovsky et al., Lancet 2007, 370: 230-39). The study entails the evaluation of an ITRT as an outcome factor related to patient response to treatment (Bosanquet et al: ASH Annual Meeting Abstracts Blood, 2006 108:94a: Abstract 303). The trial started in 1999 and included 777 patients with previously untreated CLL. The patients were treated with chlorambucil (Chl) or fludarabine +/cyclophosphamide (Flu or FluCy). In this study, the TRAC assay was used to evaluate the ex vivo sensitivity to drugs prior to patient treatment. For analysis, patients were divided into three groups depending upon their ITRT result: Drug Resistant (DR), Drug Sensitive (DS), or Drug Intermediate (DI). Table 3 summarizes the results.

**Table 3. Correlation of ITRT Result (DS, DI, and DR) and Response to the Same Drugs in Patients with Chronic Lymphocytic Leukemia**

| **Result** | **Ch1** | **Flu** | **FluCy** | **Total** |
|---|---|---|---|---|
| DS | 85.1 (94) | 90.7 (54) | 95.7 (70) | 89.9 (218) |
| DI | 66.3 (92) | 79.2 (53) | 97.3 (37) | 76.4(182) |
| DR | 37.6 (24) | 21.4(14) | 25.0 (4) | 31.0 (42) |
| **Total** | **71.5 (210)** | **77.7 (121)** | **93.7 (111)** | **78.7 (442)** |

| | | | | |
|---|---|---|---|---|
| Results are represented as % of patients responding (with the number of patients in parentheses) for each drug (Chl and Flu) and for the drug combination (FluCy) | | | | |

As shown in Table 3, ITRT results correlate well with patient clinical responses. Among the 49% of patients that were DS, most of them (90%) responded to the chemotherapy treatment, whereas among the 9.5 % of patients that were DR, only 31% responded to chemotherapy. Among the 24 patients that were DR to Chl, 71% were DS or DI to Flu, and all showed either DS or DI to the FluCy combination. Among the 14 patients DR to Flu, only 36% were DS or DI to the FluCy combination. These results suggest that using ITRT results could have guided more effective treatments resulting in better clinical outcomes.

WO2007/015926 is concerned with assessing drug response in a sample of a CLL patient to develop personalized therapeutic strategies using a method of flow cytometry measuring apoptosis in response to drugs and drug combinations.

Given the tremendous therapeutic potential of personalized medicine tests, there exists an urgent need in the art for the development of an ITRT using shorter incubation times. Use of such an assay to assist in treatment choices could potentially increase the response rate, the progression-free survival time, and the overall survival time of patients afflicted with cancer. Preferably, the assay would use flow cytometry to allow for the evaluation of individual tumor cell death and reduce the assay incubation time to achieve a cytotoxicity profile in a short amount of time. Also desirable is an ITRT that would provide more extensive information regarding a larger numbers of drugs and concentrations of drugs that could be efficacious, either alone or in combination.

### SUMMARY OF THE INVENTION

In its broadest sense, the invention is defined by the subject-matter of the appended claims and the following aspects:
1. A method for analyzing cellular responsiveness to drugs, comprising: a. obtaining a sample of whole blood, whole peripheral blood or whole bone marrow that has been withdrawn from a patient with a hematological neoplasm; b. dividing the whole sample into at least 35 aliquots; c. combining each of the at least 35 aliquots with a drug composition; and d. measuring apoptosis or cell depletion in each of the at least 35 aliquots by flow cytometry.
2. The method of aspect 1, wherein the drug composition in each aliquot differs from the drug composition in all other aliquots by at least one drug identity, concentration of a combination thereof, and wherein the drug compositions collectively include at least one non-cytotoxic drug.
3. The method of aspect 1 or 2, wherein the analysis is completed within 72 hours of combining the aliquots with a drug composition.
4. The method of any of aspects 1 to 3, wherein the number of aliquots combined with a drug composition is at least 96.
5. The method of any of aspects 1 to 4, wherein at least one of the drug compositions comprises a non-cytotoxic drug.
6. The method of any of aspects 1 to 5, wherein at least one of the drug compositions combines a non-cytotoxic drug and a cytotoxic drug.
7. The method of any of aspects 1 to 6, wherein the whole sample comprises cells from a hematological neoplasm selected from the group consisting of chronic lymphocytic leukemia, adult acute lymphoblastic leukemia, pediatric acute lymphoblastic leukemia, multiple myeloma, myelodysplastic syndrome, non-M3 acute myeloblastic leukemia, acute myeloblastic leukemia M3, non-Hodgkin's lymphoma, Hodgkin's lymphoma, and chronic myeloid leukemia.
8. The method of any of aspects 1 to 7, wherein the drug composition comprises a compound selected from the group consisting of 5-Azacitidine, alemtuzumab, aminopterin, Amonafide, Amsacrine, CAT-8015, Bevacizumab, ARR Y520, arsenic trioxide, AS1413, Atra, AZD 6244, AZD1152, Banoxantrone, Behenoylara-C, Bendamustine, Bleomycin, Blinatumomab, Bortezomib, Busulfan, carboplatin, CEP-701, Chlorambucil, Chloro Deoxiadenosine, Cladribine, clofarabine, CPX-351, Cyclophosphamide, Cyclosporine, Cytarabine, Cytosine Arabinoside, Dasatinib, Daunorubicin, decitabine, Deglycosylated-ricin-A chain-conjugated anti-CD19/anti-CD22 immunotoxins, Dexamethasone, Doxorubicine, Elacytarabine, entinostat, epratuzumab, Erwinase, Etoposide, everolimus, Exatecan mesilate, flavopiridol, fludarabine, forodesine, Gemcitabine, Gemtuzumab-ozogamicin, Homoharringtonine, Hydrocortisone, Hydroxycarbamide, Idarubicin, Ifosfamide, Imatinib, interferon alpha 2a, iodine 1131 monoclonal antibody BC8, Iphosphamide, isotretinoin, Laromustine, L-Asparaginase, Lenalidomide, Lestaurtinib, Maphosphamide, Melphalan, Mercaptopurine, Methotrexate, Methylprednisolone, Methylprednisone, Midostaurin, Mitoxantrone, Nelarabine, Nilotinib, Oblimersen, Paclitaxel, panobinostat, Pegaspargase, Pentostatin, Pirarubicin, PKC412, Prednisolone, Prednisone, PSC-833, Rapamycin, Rituximab, Rivabirin, Sapacitabine, Dinaciclib, Sorafenib, Sorafenib, STA-9090, tacrolimus, tanespimycin, temsirolimus, Teniposide, Terameprocol, Thalidomide, Thioguanine, Thiotepa, Tipifarnib, Topotecan, Treosulfan, Troxacitabine, Vinblastine, Vincristine, Vindesine, Vinorelbine, Voreloxin, Vorinostat, Etoposide, Zosuquidar, and combinations thereof.
9. The method of any of aspects 1 to 8, wherein the drug composition comprises a compound selected from the group consisting of Aluminum Oxide Hydrate, Lorazepam, Amikacine, Meropenem, Cefepime, Vancomycin, Teicoplanin, Ondansetron, Dexamethasone, Amphotericin B (liposomal), Caspofugin, Itraconazole, Fluconazole, Voriconazole, Trimetoprime, sulfamethoxazole, G-CSF, Ranitidine, Rasburicase, Paracetamol, Metamizole, Morphine chloride, Omeprazole, Paroxetine, Fluoxetine, Sertraline and combinations thereof.
10. The method of any of aspects 1 to 9, wherein each of the at least 35 aliquots contains 500 or more diseased or neoplastic cells per well.
11. The method of any of aspects 1 to 9, wherein each of the at least 35 aliquots contains 5.000 or more diseased or neoplastic cells per well.
12. The method of any of aspects 1 to 9, wherein each of the at least 35 aliquots contains 10.000 or more diseased or neoplastic cells per well.
13. The method of any of aspects 1 to 9, wherein each of the at least 35 aliquots contains 20.000 or more diseased or neoplastic cells per well.
14. The method of any of aspects 1 to 9, wherein each of the at least 35 aliquots contains 40.000 or more diseased or neoplastic cells per well.

The present invention relates to the development of a personalized medicine test for a patient. In a general embodiment, the present invention is directed to methods for analyzing cellular responses to drugs using an ex vivo assay. Described herein are methods of analyzing whole blood samples, manipulating a large number of variables, and quickly completing analyses.

In addition to individual drug effects, detailed analyses of drug interactions, including the Combination Index and Dose Reduction Index, can be used to identify effective polytherapy regimens. Estimates of accuracy of both indexes can be calculated with accurate algebraic estimation algorithms (*i*.*e*., Monte Carlo simulations) based on the Median Effect methods described by Chou and Talalay (Chou et al., Adv Enzyme Regul 1984, 22:27-55). The Combination Index (CI) is a quantitative measure of the degree of drug interaction in terms of additive effect, where synergism is indicated by a CI <1, additive effect is indicated by a CI∼1, and antagonism is indicated by a CI>1. A dose-reduction index (DRI) is a measure of how much the dose of each drug in synergistic combination may be reduced at a given effect level compared with the dose of each drug alone. More recently, the MixLow method (Boik et al., BMC Pharmacol 2008, 8:13; Boik, Stat Med 2008, 27(7):1040-61) has been proposed as an alternative to the Median-Effect method of Chou and Talalay (Chou et al., Adv Enzyme Regul 1984, 22:27-55) for estimating drug interaction indices. One advantage of the MixLow method is that the nonlinear mixed-effects model used to estimate parameters of concentration-response curves can provide more accurate parameter estimates than the log linearization and least-squares analysis used in the Median-Effect method. One of skill in the art will know that these calculations and related methods can be used to analyze drug interactions for mixed drug treatments as described herein.

The limited amount of sample that can be extracted from patient limits the number of drug compositions that can be tested for the personalized medicine test. However, recent developments have provided mouse models that can propagate the primary cells of patients with hematological malignancies through multiple mice becoming a continuous source of patient cells (Pearson et al., Curr Top Microbiol Immunol. 2008, 324:25-51; Ito et al., Curr Top Microbiol Immunol. 2008, 324:53-76). These models may enable ex vivo sampling of many more drug compositions, and in particular drug combinations, than a recently extracted patient sample. It is contemplated that these models can be used in the methods described herein. For example, the samples may be drawn from an animal model, such as a mouse model. In particular, these models may enable exploring the efficacy of concomitant or adjuvant medicines, given to patients to palliate the effects of chemotherapy. These models may also enable exploration of the potential efficacy of approved non-cytotoxic safe drugs, which in the future could be added to treatments for an individual patient to increase the probability of therapeutic efficacy. Furthermore, the efficacy of any drug combination of a drug composition identified in ex vivo testing using human patient cells, directly from a patient sample or propagated by a mouse models, could be tested in mouse models in vivo.

As a personalized medicine test, it is desirable to provide patients and caregivers with summaries of cellular responses to drugs and drug combinations.

The present disclosure also includes particular drug combinations that are useful, for example, in treating AML, ALL, CLL, and NHL, and the use of those drug combinations in treating lymphoproliferative disease.

### BRIEF DESCRIPTION OF THE DRAWINGS

Figure 1 depicts the flow cytometric detection of phosphatidylserine expression on apoptotic cells using fluorescein labeled Annexin V.
Figure 2 depicts a precursor B-ALL adult case displaying BCR/ABL gene rearrangements [t(9;22)positive] and the detection of leukemic and normal cells among CD19 positive cells using quantitative flow cytometry.
Figure 3 illustrates a protocol for the ex vivo evaluation of peripheral blood (PB) or bone marrow (BM) in a sample from a chronic lymphocytic leukemia (CLL) patient.
Figure 4 depicts the ex vivo response to several drugs currently approved for CLL treatment in nine different patients.
Figure 5 depicts the number of desirable drug compositions to optimize a personalized medicine test treatment for an individual patient.
Figure 6 depicts several non-cytotoxic drugs *(i.e.,* paroxetine, fluoxetine, sertaline, guanabenz, and astemizole) that induce apoptosis in malignant CLL samples with similar efficacy as cytotoxic drugs approved for CLL treatment *(i.e.,* fludarabine, chlormbucil, and mitroxantrone).
Figure 7 depicts a dose-response curve for paroxetine in a whole blood sample from a CLL patient and compares the apoptotic effects of paroxetine on leukemic, T, and NK cells.
Figure 8 depicts a kinetic difference on the induction of apoptosis in CLL patient whole blood samples by sertraline and three drugs currently used in CLL treatment (*i.e*., fludarabine, chlorambucil, and mitoxantrone).
Figure 9 depicts the differential efficacy of compounds in the same pharmacological class as paroxetine (*i.e*., SSRIs) in inducing apoptosis in CLL samples.
Figure 10 depicts the hit frequency expressed as the number of patient samples, out of 23 total patient samples, for which non-cytotoxic drugs eliminate leukemic CLL cells with the same efficiency as approved cytotoxic drugs, and illustrates that most non-cytotoxic drugs are effective in very few patients.
Figure 11 depicts the potentiation of the efficacy of the approved cytotoxic drug chlorambucil by the non-cytotoxic drug sertraline.
Figure 12 depicts the percentage of Annexin V positive cells induced by the cytotoxic drugs vincristine, mitoxantrone, and cyclophosphamide (which are used in CLL treatments) and the percentage of Annexin V positive cells induced by the non-cytotoxic drugs omeprazole and acyclovir (which are often prescribed to treat side effects caused by chemotherapy).
Figures 13A-C illustrate 96-well plate designs for the personalized medicine testing of patients with CLL.
Figures 14A-F illustrate a 96-well plate design for the personalized medicine testing of patients with Multiple Myeloma.

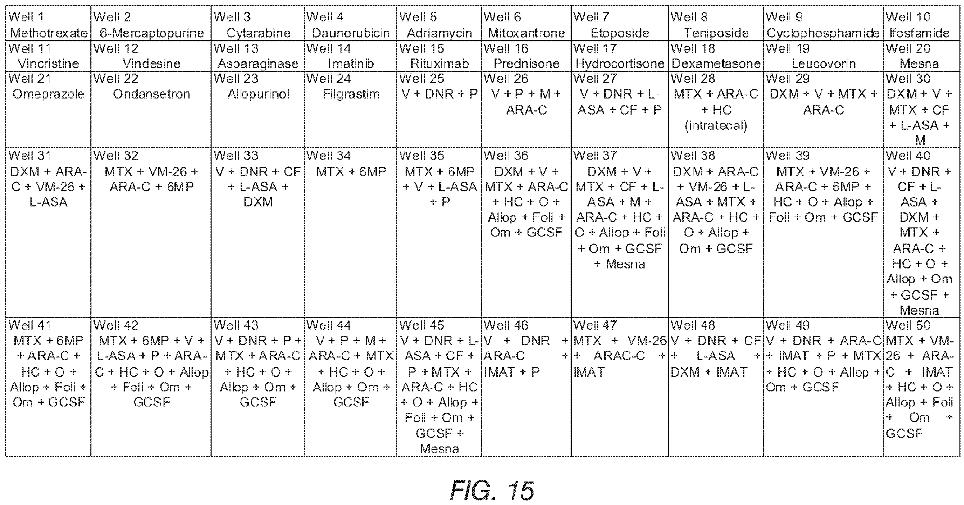

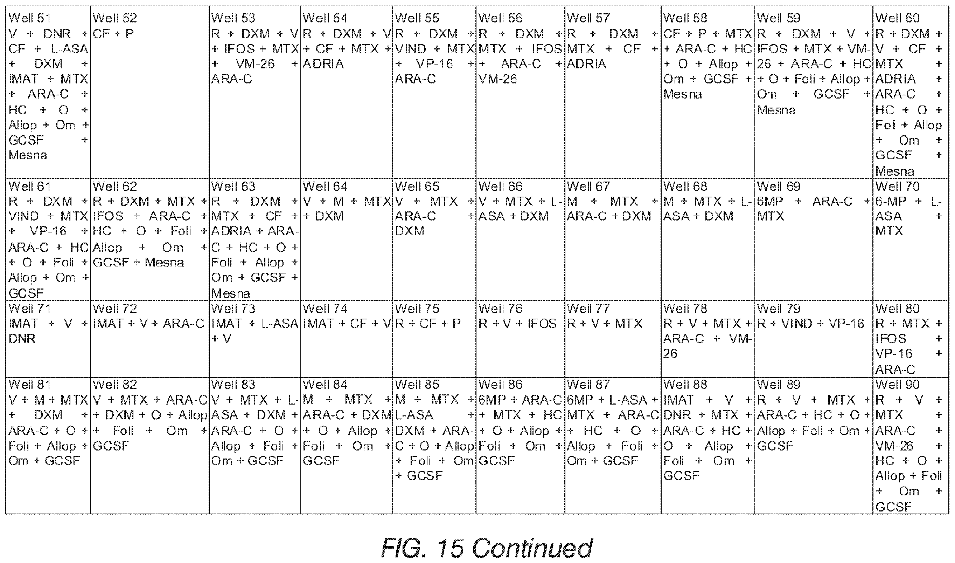

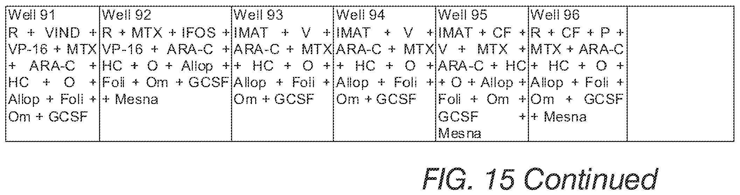

Figure 15 illustrates a 96-well plate design for the personalized medicine testing of patients with Acute Lymphoblastic Leukemia (ALL), including cytotoxic and non-cytotoxic drugs administered in the treatment protocols of PETHEMA. MTX: methotrexate; 6MP: 6-mercaptopurine; ARA-C: cytarabine; DNR: daunorubicin; ADRIA: adriamycin; M: mitoxantrone; VP-16: etoposide; VM-26: teniposide; CF: cyclophosphamide; IFOS: ifosfamide; V: vincristine; VIND: vindesine; L-ASA: asparaginase; IMAT: imatinib; R: rituximab; P: prednisone; HC: hydrocortisone; DXM: dexametasone; Foli: leucovorin; Mesna: mesna; Om: omeprazole; O: ondansetron; Allop: allopurinol; GCSF: filgrastim.
Figure 16 illustrates a 96-well plate design for the personalized medicine testing of patients with Myelodysplastic Syndrome, including cytotoxic and non-cytotoxic drugs administered in the treatment protocols of PETHEMA.
Figure 17 illustrates a 96-well plate design for the personalized medicine testing of patients with Acute Myeloblastic Leukemia (not M3), including cytotoxic and non-cytotoxic drugs administered in the treatment protocols of PETHEMA. Dauno: daunorubicin; Ida: idarubicin; ARA-C: citarabine; Mitox: mitoxantrone; VP16: etoposide; Fluda: fludarabine; GCSF: filgrastim; Ondans: ondansetron; Cotri: co-trimoxazol; AcF: folic acid; Alop: allopurinol; Om: omeprazol; Carbop: carboplatin; Dauno lipo: liposomal daunorubicin (Daunoxome®); AMSA: amsacrin; GO: gentuzumab ozogamicina.

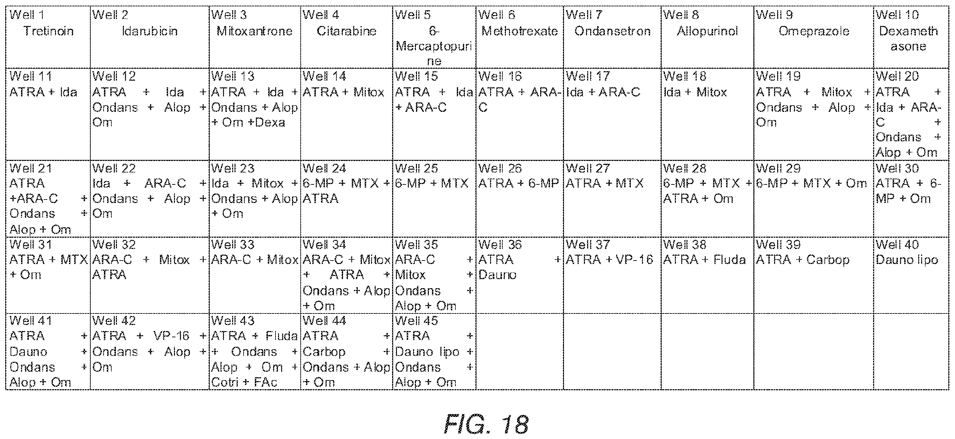

Figure 18 illustrates a 96-well plate design for the personalized medicine testing of patients with Acute Myeloblastic Leukemia M3 (Promyelocytic), including cytotoxic and non-cytotoxic drugs administered in the treatment protocols of PETHEMA. ATRA (all-trans retinoic acid): tretinoin; Ida: idarubicin; Mitox: mitoxantrone; ARA-C: citarabine; 6-MP: 6- mercaptopurine; MTX: methotrexate; Ondans: ondansetron; Alop: allopurinol; Om: omeprazole; Dexa: dexamethasone; VP-16: etoposide; Fluda: fludarabine; Carbop: carboplatin; Dauno lipo: liposomal daunorubicin; Dauno: daunorubicin; Cotri: co-trimoxazole; FAc: folic acid.
Figure 19 depicts the effect of sertraline on the inhibition of cell proliferation in TOM-1 and MOLT-4 cell lines.
Figure 20 depicts the effect of sertraline on the induction of apoptosis in TOM-1 and MOLT-4 cell lines at 24 hours.
Figure 21 depicts the effect of sertraline on the induction of active caspase-3 in TOM-1 and MOLT-4 cell lines at 24 hours.
Figure 22 depicts the ex vivo efficacy of individual drugs (*i.e*., rituxamib, fludarabine, mitoxantrone, and cyclophosphamide (maphosphamide)), and the most resistant and sensitive polytherapies with combinations of these individual drugs in a CLL sample.
Figure 23 depicts the results of the same experiment as Figure 22 with a 5-point dose response curve that characterizes the ex vivo efficacy of fludarabine, cyclophosphamide (maphosphamide), and their combination.
Figure 24 depicts the results of the same experiment as Figure 24 with a 5-point dose response curve that characterizes the ex vivo efficacy of fludarabine, cyclophosphamide (maphosphamide), mitoxantrone, and their combinations.
Figure 25 depicts the results of the same experiment as Figure 24 with a 5-point dose response curve that characterizes the ex vivo efficacy of fludarabine, cyclophosphamide (maphosphamide), rituximab, and their combinations.
Figure 26 depicts the effect of fludarabine and maphosphamide alone and in combination at five different concentrations in a clinical protocol for two patients, P2.0144 (left) and P2.0149 (right).
Figure 27 depicts a calculation of the synergism between fludarabine and maphosphamide (cyclophosphamide) found in CLL patient P2.0149 from Figure 26 using the Chou and Talalay method (Chou et al., Eur J Biochem 1981, 115(1):207-16; Chou et al., Adv Enzyme Regul 1984, 22:27-55).
Figure 28 depicts the effects of incubation time (both drug exposure time (0.5, 4, and 8 hours) and overall incubation time (24 or 48 hours)) on the efficacy of fludarabine and sertraline to induce apoptosis in malignant cells in CLL samples.
Figure 29 depicts a matrix for 2 drug combinations.
Figure 30 depicts a matrix for 3 drug combinations.
Figure 31 depicts a matrix for 4 drug combinations.
Figure 32 depicts a 3-color multiplexing of peripheral blood leukocytes using cell tracker dyes.
Figure 33 depicts fluorochrome dyes used to multiplex wells in a CLL sample distinguishing malignant cells and detecting apoptosis with Annexin V.

### DETAILED DESCRIPTION OF THE PREFERRED EMBODIMENT

The present invention provides meethods to evaluate the ex vivo apoptotic efficacy for multiple drug combinations using a screening platform. Specifically, the present invention provides a method to perform cell-based screening that incorporates both automated sample preparation and automated evaluation by flow cytometry that is geared for rapid data acquisition, analysis and reporting of results. The use of flow cytometry methods allows for the evaluation of individual cell death, whose single cell resolution can allow the shortening of the incubation time of ex vivo assays, and thereby provide a faster turnaround in cytotoxicity profiling. The cell-based screening platform can also be used to complete all screening and validation assays in 24 to 72 hours from sample extraction. This timeline enables the reporting of results to a medical doctor after diagnostics have been performed on the hematological neoplasm and prior to the start of treatment. Consequently, the methods described herein can be used for personalized medicine and to identify possible new indications for approved drugs.

In order that the present invention may be more readily understood, certain terms are first defined. Additional definitions are set forth throughout the detailed description.

As used herein, "EC₅₀" and "EC₉₀" refer to the drug concentrations required to elicit 50% and 90% of the maximal apoptosis, respectively.

As used herein, "ex vivo" refers to primary human patient cells in vitro, where the cells can be either recently extracted, cryopreserved, or frozen to preserve their state. In some embodiments, these cells are thawed for in vitro evaluation of drug effects.

As used herein, "ex vivo therapeutic index" refers to the ratio between neoplastic cell death and healthy cell death.

As used herein, "Exvitech" refers to an integrated platform that incorporates automated sample preparation, the EPS system for automated input to a flow cytometer, and automated bioinfomatic analysis.

As used herein, "hematological neoplasms," also called "hematological malignancies," refers to a group of diseases defined according to the World Health Organization classification (Swerdlow SH, Campo E, Harris NL, Jaffe ES, Pileri S, Stein H, Thiele J, Vardiman JW (Eds): WHO Classification of Tumors of Hematopoietic and Lymphoid Tissues. International Agent for Research of Cancer (IARC), Lyon. 4th Edition. Lyon 2008).

As used herein, Individualized Tumor Response Test/Testing (ITRT) refers to methods that describe the effect of anticancer treatments on whole living tumor cells freshly removed from cancer patients.

As used herein, "polytherapy" refers to treating a patient with multiple drugs.

As used herein, a "non-cytotoxic" compound or drug refers to a compound or drug that is not approved by a regulatory agency as a cytotoxic, chemotherapeutic, or antineoplastic agent.

As used herein, "aliquot" refers to a sample or fraction thereof that can be in separate containers or wells, or can be formed in tubing or another medium, wherein differences in drug content, drug identity, or drug concentration can be maintained even in congruent samples, whether the samples are continuous or are separated by a gas or immiscible liquid (e.g., oil).

As used herein, "drug composition" refers to the single drug, and various concentrations thereof, or combinations of drugs, and various concentrations thereof, administered to an aliquot for analysis or to a patient for treatment.

As used herein, "pharmaceutically acceptable salt," refers to a formulation of a compound that does not cause significant irritation to an organism to which it is administered and does not abrogate the biological activity and properties of the compound. Pharmaceutical salts can be obtained by routine experimentation.

As used herein, "well" or "chamber" refers to any structure with the capacity to hold a sample sufficient to perform the methods described herein. One of skill in the art will know that a "well" or a "chamber" can include, *e*.*g*., a recess in a plate, a spot on a glass slide created by surface tension, or a region of a microfluidic device.

Reference will now be made in detail to the presently preferred embodiments of the invention, examples of which are provided in the accompanying drawings. Wherever possible, the same or similar reference numbers are used in the drawings and the description to refer to the same or like parts. It should be noted that the drawings are in simplified form and are not to precise scale. Although the disclosure herein refers to certain illustrated embodiments, it is to be understood that these embodiments are presented by way of example and not by way of limitation. The disclosed methods may be utilized in conjunction with various medical procedures that are conventionally used in the art.

The disclosed methods have several advantages over that of the prior art that are described herein. One advantage is that the methods can analyze cellular responses to a large number of variables, including many drug compositions and different incubation times. Another advantage is the speed in which the methods analyze cellular responses to drugs. Another advantage is the capacity to analyze whole blood and thus more closely mimic the in vivo environment of a patient. Furthermore, the present methods are capable of generating dose response curves for the large number of drugs and drug compositions. Combined, these methods afford the advantage of developing a polytherapy regimen to treat patients. In a specific embodiment, the methods facilitate developing a polytherapy regimen to treat patients suffering from a hematological disorder.

The disclosed methods are amenable to the use of various types of equipment, including one or more sample preparation robots and one or more flow cytometers to analyze cellular responsiveness to drug compositions. Flow cytometry allows for single cell analysis at speeds far surpassing any other single cell analysis technology in the art. This enables a statistically significant number of cells to be analyzed faster than using other alternative techniques. In one embodiment, flow cytometry is used to analyze cellular responsiveness to drug compositions. In one embodiment, the analysis is completed within about 96 hours from the time that a sample is obtained. In another embodiment, the analysis is completed within about 72 hours from the time that a sample is obtained. In another embodiment, the analysis is completed within about 48 hours from the time that a sample is obtained. In another embodiment, the analysis is completed within about 24 hours from the time that a sample is obtained. One example of a flow cytometer useful for the methods described herein is provided in U.S. Patent No. 7,459,126.

Sample preparation robots and flow cytometers may be integrated with each other, or sample preparation robots and flow cytometers may not be integrated with each other. A flow cytometer is used without a sample preparation robot. In a specific embodiment, a CYAN™ cytometer (Beckman Coulter, Fullerton, CA) is used without a sample preparation robot. There are many different types of sample preparation robots and liquid handlers that are known in the art. In one embodiment, a flow cytometer is used with any suitable sample preparation robot or liquid handler that is known in the art. In another embodiment, a CYAN™ cytometer (Beckman Coulter, Fullerton, CA) is integrated with a small liquid handler, called EPS, to automate the delivery of the samples to the cytometer. In a specific embodiment, the EPS is a Tecan 360 liquid handler (Tecan, Männedorf, Switzerland). In another embodiment, the EPS is customized with syringe pumps and an interface switching valve that allows for the contents of each well to be aspirated through a fixed tip, transferred to a holding loop, and injected into the cytometer. In another embodiment, sample preparation and compound plating can be completed with a BIOMEK® 3000 liquid handler (Beckman Coulter, Fullerton, CA). Any number of well plates can be used, and one particularly useful well plate is a 96 well plate. Various other plate sizes are also contemplated, including those with 24, 48, 384, 1536, 3456, or 9600 wells. The sample preparation units can be encased within a flow cabinet that allows for the compounds and samples to remain sterile while being manipulated. Upon completion of assay setup, the plates are loaded onto the sample analysis system.

The CYAN™ cytometer (Beckman Coulter, Fullerton, CA) is a three laser, nine detector instrument, and methodologies that are known in the art have been developed to take full advantage of the multi-laser, and consequently, multiparametric measurement capacities of such modern flow cytometers. In one embodiment, a single laser flow cytometer is used for the analyzing step. In another embodiment, a multi-laser flow cytometer is used for the analyzing step. The development and optimization of an extensive set of fluorochromes and conjugating chemistries allows for a variety of ligands, such as immunoglobulins and small molecules, to be conjugated to the fluorochromes. Lasers with emission lines ranging from the ultraviolet to the red region of the light spectrum can excite these fluorochromes. Consequently, a large number of spectrally distinct reagents can be used to label cells for study with fluorescence-based instrumentation such as flow cytometry. These reagents are well known in the art. In one embodiment, one or more fluorochromes are used during the analyzing step. In some embodiments, one or more stains are used in the analysis of cellular responses to drug compositions.

There are several methods known in the art that minimize inadvertent sample mixing in tubing prior to analysis. One such method that is known to minimize inadvertent sample mixing during the ex vivo assay is a positive displacement pump that allows for all tubing to be washed between wells to eliminate cell carryover. Another method that is useful to minimize inadvertent sample mixing is an endpoint assay. An endpoint assay is advantageous because compound carryover is not an issue. In one embodiment, the assay comprises an endpoint assay.

There are several methods known in the art to collect and store data obtained from assays using flow cytometers. In one embodiment, software incorporated with the EPS records timing information on the injection and incubation times for each well. When a screening assay is run, two acquisition files can be collected. In one embodiment, one file, located in the cytometer software, contains actual data for each cell analyzed by the instrument. In another embodiment, a second file is a timing file, located in the EPS software or in the cytometer software, which contains actual data for each cell analyzed by the instrument. Each of these files can be named according to a bar code scanned from the well plate, *e*.*g*., a 96 well plate, at the start of an assay run.

A user can load all of the files from both instruments into an analysis software program, such as an EPS Analyzer. This program is designed to separate the acquired data from the cytometer into groups, and assign the well numbers of the compounds that were mixed with the cells in each group. Another use of the program involves gating the individual populations based on fluorescent readouts so that each individual population can be discretely analyzed. An analysis marker, included in the assay setup, is also evaluated. In one embodiment, for the screening and validation assays, Annexin V FITC, a marker of apoptosis conjugated to a fluorophore, is used to discriminate live cells from those entering the apoptotic pathway.

After completion of an analysis, files are uploaded into a database. In one embodiment, the database is ACTIVITYBASE™ from IDBS (Guildford, UK). Uploading files into a database allows for the rapid evaluation of the data to determine the compounds that are active for each patient sample. As data is accumulated, bioinformatics tools can be constructed and developed to facilitate data interpretation. As an example, pharmacological criteria such as EC₅₀, EC₉₀, maximum apoptosis, etc., from acquired data can be compared across many patient samples and correlated with immunophenotyping results and genetic information. Considering the large amounts of data acquired with each assay screen, a flexible database management system is important to the screening process.

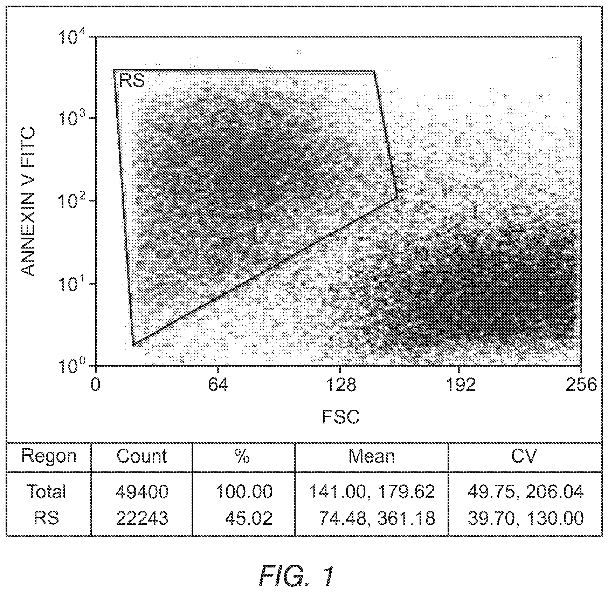

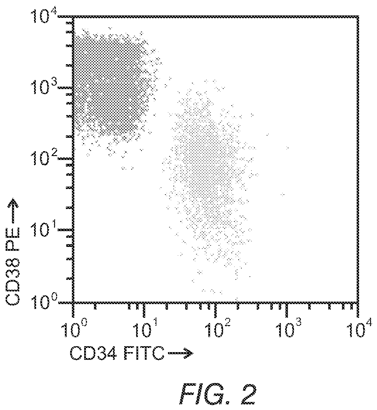

This system can determine the ex vivo therapeutic index by measuring the ability of a drug composition to induce apoptosis. Figures 1 and 2 depict the ability to detect apoptotic cells and differentiate between normal and tumor phenotypes using flow cytometry. In one embodiment, the method uses flow cytometry to differentiate between normal and tumor phenotypes. In another embodiment, the method uses flow cytometry and monoclonal antibodies to differentiate between normal and tumor phenotypes. In another embodiment, the method uses flow cytometry to detect apoptotic cells. In a specific embodiment, the method uses Annexin V coupled to Fluorescein Isothiocyanate (FITC) to detect phosphatidylserine expression on apoptotic cells. The simultaneous use of appropriate combinations of monoclonal antibodies that are known in the art with multiparametric analysis strategies allows for the discrimination of leukemic cells from residual normal cells present in samples from patients with hematological disorders. In one embodiment, the method allows for the discrimination between malignant cells and normal cells in either blood or bone marrow samples. In another embodiment, the discrimination between malignant and normal cells in either blood or bone marrow is performed according to the recent methodology developed by the Euroflow normative (EuroFlow Consortium, Cytometry A. 2008 Sep;73(9):834-46; van Dongen et al., 14th EHA Congress, Berlin, DE 4 June 2009: to be published in Leukemia 2010 (in press)).

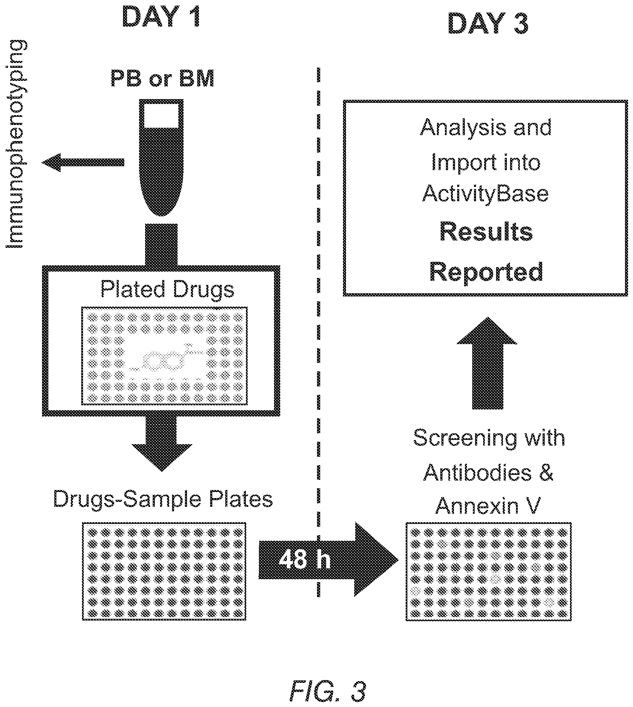

An ex vivo screening process for drug compositions is schematically shown in Figure 3. In Figure 3, the sample is prevented from coagulation by heparin, immunophenotyped, and counted. Then the sample is diluted to achieve a leukemic cell concentration of about 4,000 cells/µL. 45µl of the cell suspension are added to 96-well plates that contain the pharmacological agents in 5 different concentrations. After incubating the drugs and drug combinations with the sample for approximately 48 hours, the red blood cells are lysed and washed away to concentrate the leucocytes that contain the malignant cells. This speeds up the screening process by drastically reducing the volume and number of cells that need to be evaluated by the flow cytometer. Fluorescently labeled antibodies are added to distinguish malignant from healthy cells, and fluorescently labeled Annexin V is added to measure the level of apoptosis within each cell population, such as within the malignant cells. Screening is then performed, and the activity of each drug composition determined and the results are analyzed and reported.

In one embodiment, the method comprises splitting a sample into aliquots and distributing the aliquots into well plates. These well plates contain individual drugs or drug combinations at various concentrations. In one embodiment, the well plates contain individual drugs or combinations at various concentrations prior to the introduction of cell samples. In another embodiment, cell samples are introduced into the wells prior to the introduction of individual drugs or combinations at various concentrations. In another embodiment, an extensive library of compounds can be used, including about 20, 30, 50, 75, 100, 200, 300, 500, 700, 1000, or 2000 compounds, a range defined by any two of the preceding values, or a larger number of compounds.

In some embodiments, aliquots contain a detectable number of diseased cells per well. In one embodiment, aliquots contain about 500 or more diseased or neoplastic cells per well. In another embodiment, aliquots contain about 5,000 diseased or neoplastic cells per well. In another embodiment, aliquots contain about 10,000 or more diseased or neoplastic cells per well. In another embodiment, aliquots contain about 20,000 or more diseased or neoplastic cells per well. In another embodiment, aliquots contain about 40,000 or more diseased or neoplastic cells per well. Sample testing may be run in parallel. In one embodiment, at least two aliquots are tested in parallel to allow for immunophenotypic identification. In addition, control wells without any drug can be included (not shown) to identify the spontaneous level of apoptosis not associated with drug treatment. In one embodiment, the method uses control wells to identify the spontaneous level of apoptosis in a sample.

The time period for incubating different drug compositions with aliquots may vary. In one embodiment, the time period is up to about 24 hours. In another embodiment, the time period is up to about 48 hours. In another embodiment, the time period is up to about 72 hours. In another embodiment, the time period is up to about 96 hours. In another embodiment, the time period is up to about 120 hours. After incubation for a specified time, sample aliquots exposed to drug compositions can be treated with a buffer to lyse the erythrocyte population and concentrate the leukocyte population. In one embodiment, a buffer known in the art is used to lyse the erythrocyte population. Each well is then incubated with a reagent to detect apoptosis using flow cytometry. In one embodiment, the reagent is Annexin V.

It is possible to evaluate, using flow cytometry, the effect of each drug on each cell type and to quantify the level of selective cell death induced by each drug. Results can then be evaluated and, if desired, a new test can be started with an additional sample or aliquot in order to confirm the most relevant results in more deatil, such as the 10 best drug compositions and concentrations previously identified. Selection of the appropriate drug or drug composition that can selectively induce apoptosis in neoplastic cells, such as leukemia cells, can be made after the assay is performed for a patient sample. In one embodiment, about 5-20 drug compositions are identified and retested with fresh sample. In a specific embodiment, the five best drug compositions are identified and retested with fresh sample. In another specific embodiment, the ten best drug compositions are identified and retested with fresh sample. In another specific embodiment, the 20 best drug compositions are identified and retested with fresh sample.

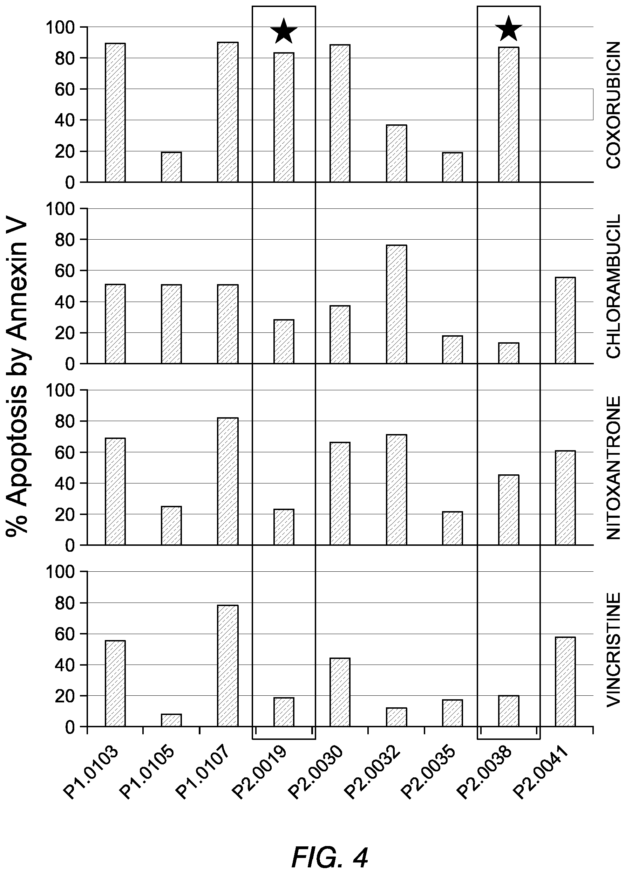

The methods provided herein have been used to analyze several drugs currently approved for chronic lymphocytic leukemia (CLL) in various patients. For example, the efficacy of the individually approved cytotoxic drugs in inducing apoptosis in malignant cells of ex vivo patient samples is provided in Figure 4. Figure 4 demonstrates that there is a high person-to-person variability in the drug responses, highlighting the potential for the methods described herein as personalized medicine tests.

In one embodiment, the method identifies drug compositions that induce greater than 90% apoptosis in patient samples. In another embodiment, the method identifies drug compositions that induce greater than 75% apoptosis in patient samples. In another embodiment, the method identifies drug compositions that induce greater than 50% apoptosis in patient samples.

Figure 4 demonstrates that the methods described herein can also detect drug compositions that generally do not induce apoptosis in patient samples. The inability to induce apoptosis may be a result of a patient's genetic predisposition to drug resistance or the neoplasm's inherent resistance to a drug. For either reason, the ability to predict the inability of a drug composition to induce apoptosis is desired. In one embodiment, the method identifies drug compositions that induce less than 90% apoptosis in patient samples. In another embodiment, the method identifies drug compositions that induce less than 75% apoptosis in patient samples. In another embodiment, the method identifies drug compositions that induce less than 50% apoptosis in patient samples. In another embodiment, the method identifies drug compositions that induce less than 30% apoptosis in patient samples.

The use of whole samples, such as whole peripheral blood or bone marrow samples, recently obtained and treated with heparin to avoid coagulation, and diluted as necessary, is an advantageous feature of the methods described herein. In one embodiment, the methods described herein use a whole blood sample. In another embodiment, the methods described herein use a whole peripheral blood sample. In another embodiment, the methods described herein use a bone marrow sample. In an embodiment, the methods described herein use samples drawn from animal models. In an embodiment, the methods described herein use samples drawn from a mouse model. Whole samples are advantageous because common in vitro assays only isolate the mononuclear fraction that contains tumor cells and discards the corresponding polymorphonuclear lymphocytes, erythrocytes, proteins, and other plasma elements through washes. This severely alters the biological context in which the effects of a drug are evaluated. In contrast, the methods described herein can maintain the erythrocytes in the plasma, as well as proteins such as albumin that typically bind about 90% to 98% of each drug.

Thus, the drug concentrations used in the assays described herein can be considered closer to the real drug concentrations existing in a patient's plasma. Using whole samples is also important because it facilitates one to observe the effects of antibodies such as Campath or rituximab on the induction of apoptosis in tumor cells. A different metric such as percentage of cell depletion rather than percentage of apoptosis may also be important. Although both metrics measure apoptosis, cell depletion counts the cells that are no longer alive relative to the control aliquots without drug. Direct apoptosis detection counts the cells that are undergoing apoptosis at the time of the measurement. The difference is the number of cells that, after apoptosis, enter necrosis and can no longer be detected by the flow cytometer. Depending on the time of the measurement, these two assays may report different results. For example, at shorter detection times *(e.g.,* 24 hours), cell depletion and cell apoptosis are similar. However, at longer detection times *(e.g.,* 48 to 72 hours), these measurements diverge, as the number of cells that first underwent apoptosis and become no longer detectable increases. For rituximab to induce apoptosis, it requires a complement found in the mononuclear fraction that is eliminated in common in vitro assays. Consequently, the methods described herein allow the original cellular microenvironment conditions to be maintained to a large extent in the analyzed samples. In one embodiment, the methods described herein substantially maintain the original cellular microenvironment.

The automated flow cytometry platform described herein is the first such platform capable of screening a large number of drug composition variables in ex vivo patient hematological samples. This platform enables the exploration of multiple drug combinations for the induction of apoptosis in an individual patient. Because hematologists generally utilize only drugs and drug combinations that are formally agreed upon in a treatment protocol (*e*.*g*., as validated through clinical trials), the methods and devices described herein preferably include the evaluation of drugs and drug combinations in existing treatment protocols. These treatment protocols can include protocols recognized in particular countries. These treatment protocols can also include older approved protocols, even though they are no longer the preferred treatment protocol. Newer experimental protocols (*e*.*g*., those still in clinical trials) are also included, including new drug compositions of approved drugs or drugs still in phase II or III clinical trials. The methods and devices described herein can also evaluate combinations of drugs for each indication of a hematological malignancy, including approved drugs and those in Phase II and III of clinical trials.

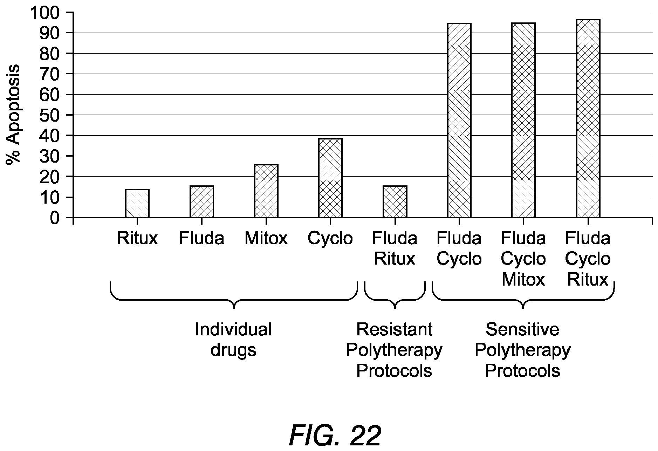

ITRT ex vivo tests previously used to guide personalized patient treatment were restricted to individual drugs, or a very small number of drug combinations. The significant benefit from evaluating multiple drug combinations, *e*.*g*., using the ExviTech platform, is demonstrated in Figure 22. For the CLL patient sample in Figure 22, individual CLL drugs (left) were ineffective, suggesting an ineffective treatment. However, these same drugs produced three combinations that were very effective at eliminating all leukemic cells (right), suggesting potential as a sensitive treatment. Thus, opposite predictions would have been made by evaluating only individual drugs or only drug combinations used in current treatment protocols. Figure 22 shows information that could be extremely important for the effective treatment of hematological neoplasms-resistant protocols that would predict lack of clinical response (center) and highly sensitive protocols that would predict a favorable clinical response (right).

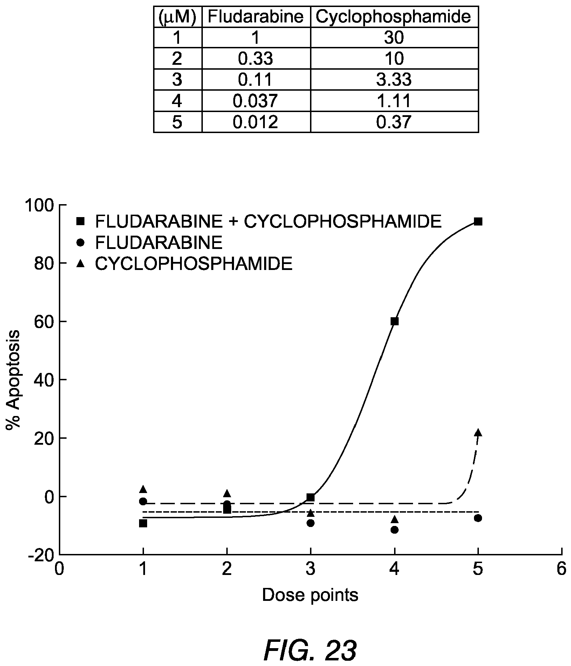

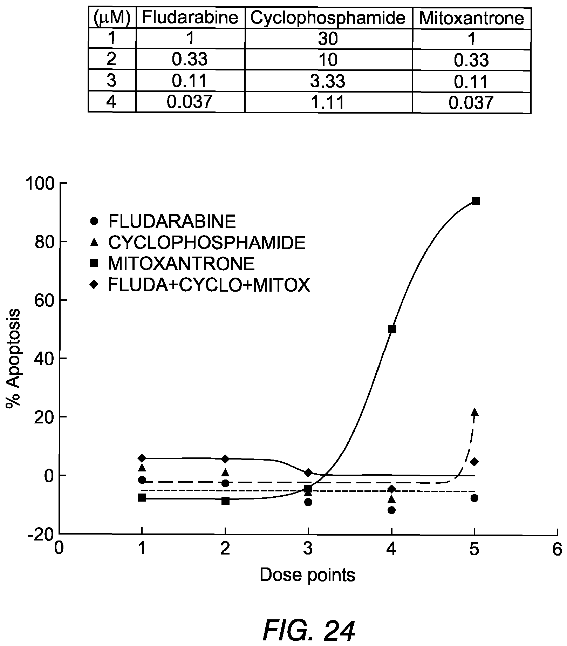

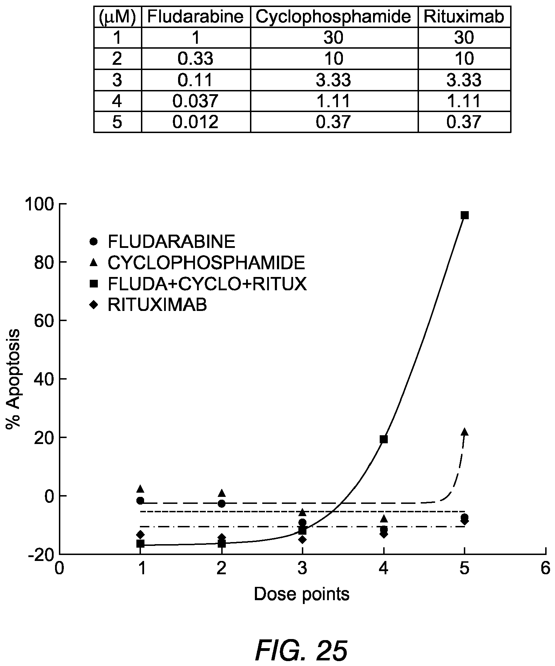

In some embodiments, the personalized medicine tests described herein evaluate five different concentrations of each drug or drug combination This enables a minimal dose-response curve to be determined that provides a more accurate pharmacological determination of efficacy than single dose data. It also facilitates a quality control by analyzing whether the five points fit to a sigmoid dose-response curve. The same data described in Figure 22 above for a CLL sample is shown in the 5-point dose-response curves in Figures 23-25.

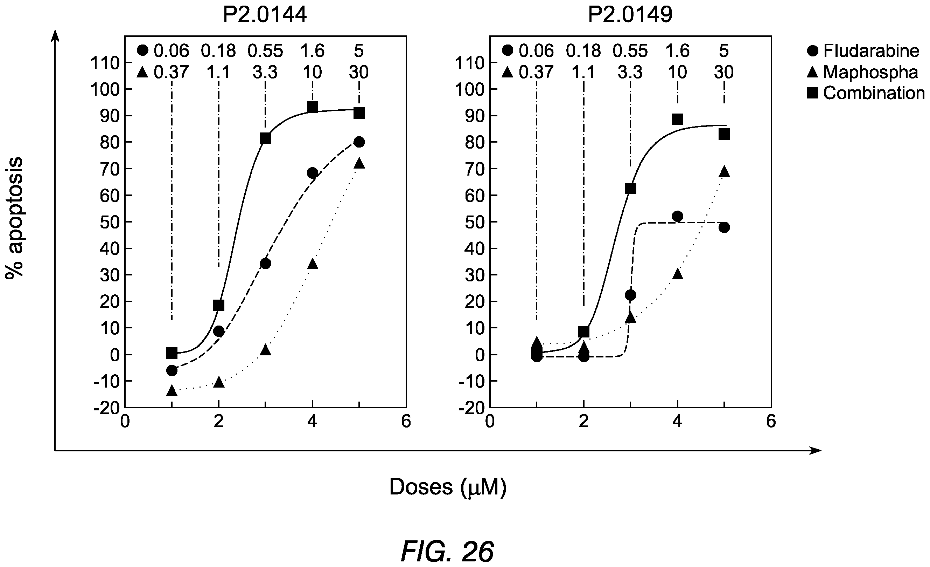

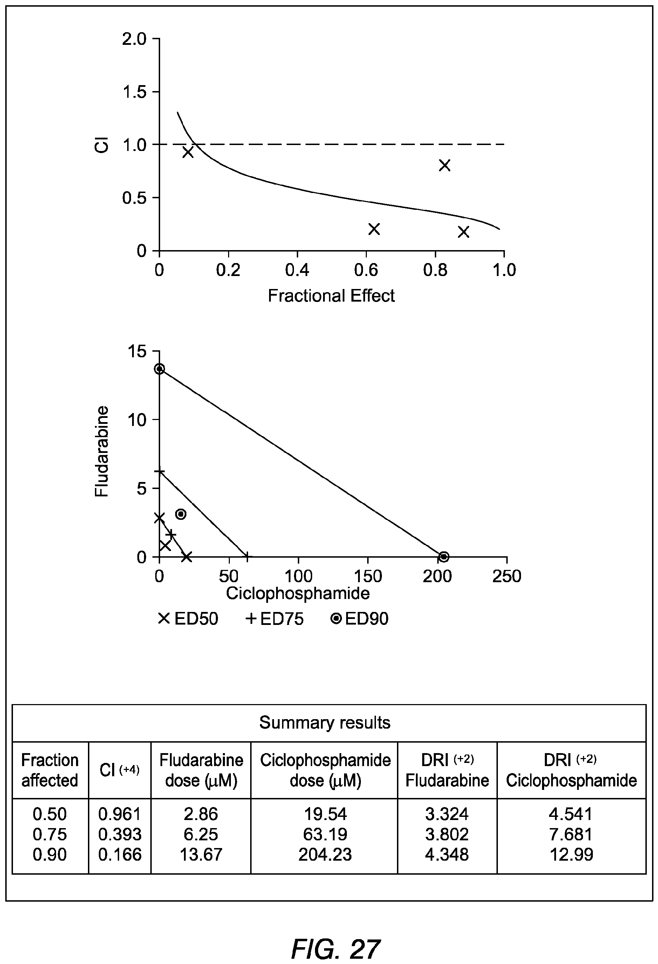

In the evaluation of drug combinations ex vivo, it is important to determine whether there is positive or negative cooperativity between combined drugs, also referred to as synergy. Such cooperation ex vivo is likely to be predictive of cooperation in vivo in the patient. Positive synergy between drugs indicates a likely increase in efficacy relative to toxicity that is a higher therapeutic index. Given the highly toxic nature of cytotoxic drugs, increasing their therapeutic index could be therapeutically important. Therefore, there have been several efforts to quantify drug synergism, and the most commonly used method is that of Chou and Talalay (Chou et al., Adv Enzyme Regul 1984, 22:27-55). Figure 26 shows the synergistic combination of fludarabine and maphosphamide (the metabolite and active ingredient of cyclophosphamide) in two CLL patient samples, where the Cooperative Index (CI) calculated using the program Calcusyn (Chou et al., Adv Enzyme Regul 1984, 22:27-55) to characterize potential synergy for the combinations. Figure 27 depicts a more elaborate calculation of the synergism found in patient P2.0149 from Figure 26 using the Chou and Talalay method.

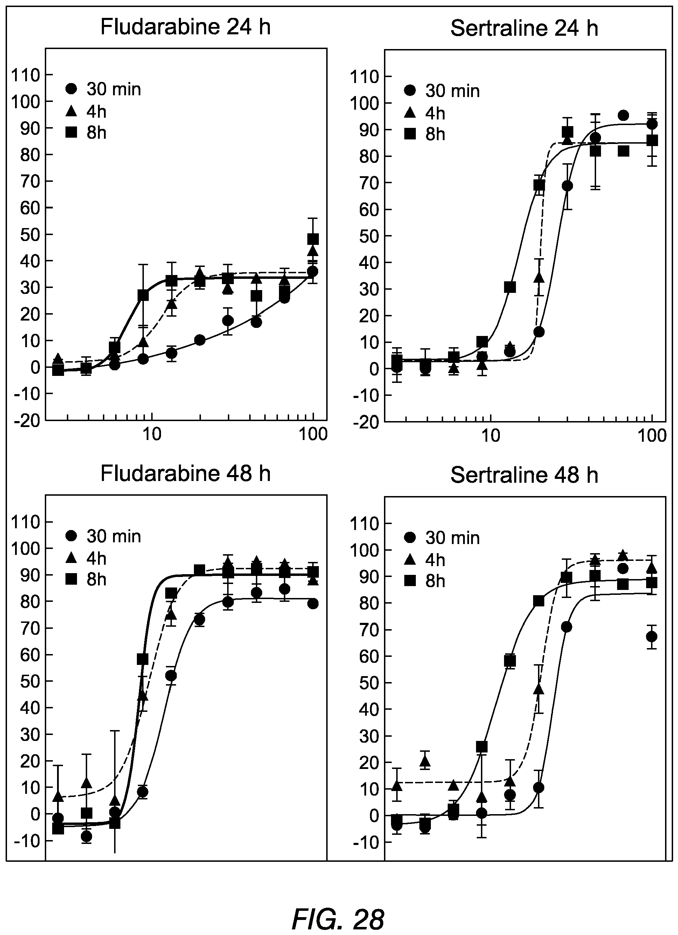

The efficacy of drugs and drug combinations may also be affected by their kinetics. Figure 28 show different kinetic behavior in a CLL sample with the approved cytotoxic drug fludarabine and the non-cytotoxic antidepressant drug sertraline. Sertraline (right panels) eliminates all malignant cells within 24 hours (right top panel), while fludarabine requires 48 hours (left bottom panel). However, both drugs require only 30 minutes of incubation with the sample to induce maximal apoptosis. This indicates that although apoptosis measured by Annexin V requires 24 or 48 hours to be fully detectable, malignant cells are programmed for apoptosis within a short period of incubation, In one embodiment, drug compositions are incubated at time periods of about 10 minutes, 15 minutes, 20 minutes, 30 minutes, 1 hour, 2 hours, 4 hours, 8 hours, or a range defined by any two of the preceding values. In another embodiment, apoptosis is measured at time points at about 24 hours, 48 hours, or 72 hours after the start of incubation, or a range defined by any two of the preceding values.

As the methodology described herein demonstrates, this platform enables the evaluation of hundreds to thousands of individual wells containing hematological samples mixed with drugs representing different compositions and concentrations. The limit of drug compositions is dictated by the volume and cellularity of the hematological sample obtained from the patient rather than the throughput of the platform. Because of the small volume used for each drug composition, such as about 20,000 cells per well, it is possible to evaluate up to about 10,000 or more drug compositions per sample obtained or up to 20,000 or more drug compositions per sample in samples with higher than usual volumes of sample. Such a number of combinations is sufficient to evaluate the alternative polytherapy drug compositions that can be administered to an individual patient. In one embodiment, the screens are performed with a minimum of about 500 neoplastic cells per well. In another embodiment, the screens are performed with about 1,000 neoplastic cells per well. In another embodiment, the screens are performed with about 20,000 total cells per well. In another embodiment, the screens are performed with about 50,000 total cells per well. Malignant cell numbers per patient sample may vary from virtually zero to over a billion, and thus their relative proportions to total number of cells may also vary.

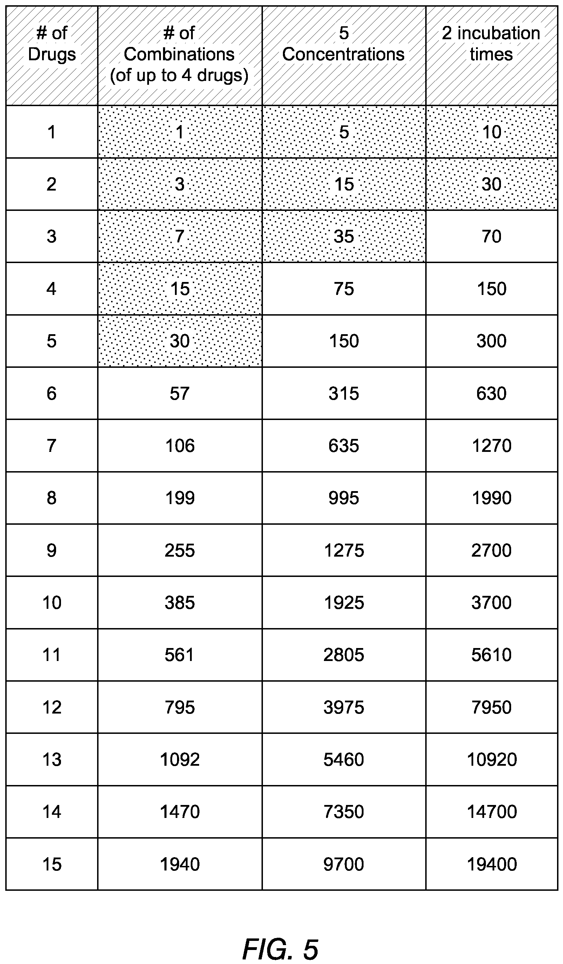

Figure 5 illustrates the number of potential drug compositions that can be explored to identify an optimal polytherapy treatment for an individual patient. Hypothetically, up to 15 drugs approved for a particular indication are considered in the first column on the left-hand side Figure 5. There are many drugs in the pipeline for hematological neoplasms, with several newer drugs expected to be approved in the coming years, There are also a number of non-cytotoxic drugs given to patients of hematological neoplasms to palliate the effects of the cytotoxic treatment (*i.e*., concomitant medicines) whose range is commonly from 5 to 10 drugs per patient. These drugs vary from gastric protectors to antiemetics for the nauseas to antibiotics and antivirals to prevent infections. The 15 drugs chosen in Figure 5 represents as a high number of approved cytotoxic drugs to be considered for a given indication, and can thus be considered as representative of certain clinical practices. The selection of 15 drugs in Figure 5 is merely illustrative and should in no way be construed as a limitation of the present invention. In Figure 5, a combination of up to 4 different drugs (2nd column) has been contemplated as a representative average number, even though there are protocols that combine 5 and 6 drugs. The design of well plates described herein illustrates the use of up to 22 different drugs in a single 96 well plate. Furthermore, different numbers of drugs can be analyzed with plates having different numbers of wells. In one embodiment, about 5 drugs are selected for analysis. In another embodiment, about 10 drugs are selected for analysis. In another embodiment, about 20 drugs are selected for analysis. In another embodiment, about 40 drugs are selected for analysis..

As illustrated in Figure 5, the number of different combinations for the 15 drugs in the second column is 1940, and would be 1470 for 14 drugs, etc. Because these results might be used to inform treatment decisions, they are preferably performed in five concentrations per drug or drug combination (3rd column). Further, evaluation of at least 2 incubation times would allow for the evaluation of kinetic parameters (4^{th} column). However, the performance of the analysis in five doses and/or more than one incubation time should not be construed as a limitation.

Even with all of the variables discussed in the preceding paragraph (*i*.*e*., number of different drugs, multiple measurements, varying drug concentrations, and varying incubation times), an automated platform capable of evaluating up to about 10,000 or 20,000 drug compositions would cover all of the hypothetical scenarios, illustrated as the non-shaded region in Figure 5. The therapeutic space enables one to explore the area shaded in gray in Figure 5 with current methods. The drug compositions that can be explored with current methods, up to 30 or 35, is shaded in gray. The rest of the table represents the novel space of drug compositions that can be explored enabled by the ExviTech platform. Because currently available manual platforms are only capable of evaluating up to about 35 individual conditions, their potential use as a personalized medicine test is limited. It follows that the automation of drug effects in ex vivo hematological samples is both favorable and innovative for a personalized medicine application. In one embodiment, the method analyzes less than 1,000 drug compositions. In another embodiment, the method analyzes about 10,000 drug compositions. In another embodiment, the method analyzes less than 20,000 drug compositions. In some embodiments, the methods described herein allow the analysis of up to about 20,000 drug compositions, which cover 1, 2, 3, or 4 drug combinations of up to 15 drugs. In some embodiments, incubation times are also varied. For example, as shown in Figure 5 (4^{th} column), including more than one incubation time increases the number of combinations tested. In some embodiments, the use of ExviTech enables the measurement of the non-shaded area in Figure 5, which represents the majority of the drug compositions required to individualize treatment to a patient.

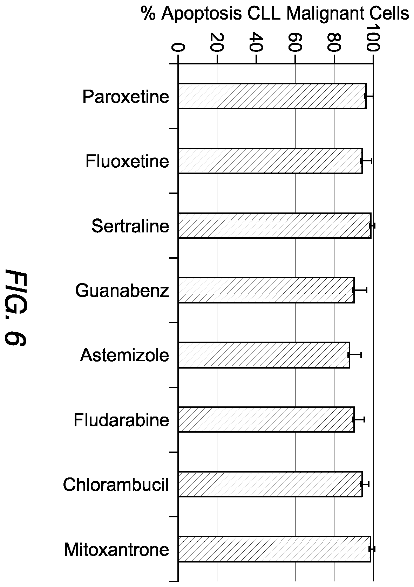

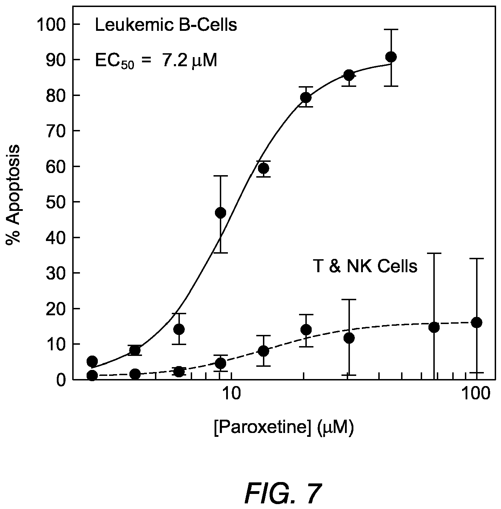

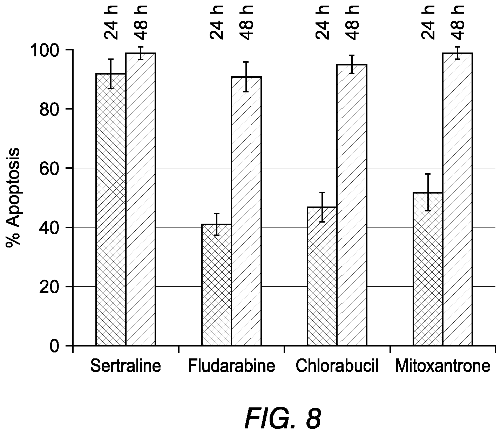

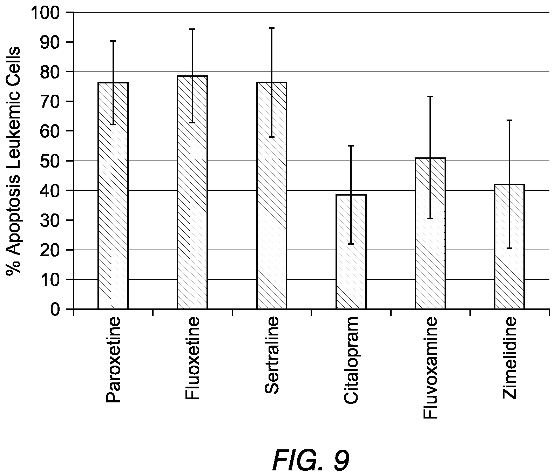

The ExviTech platform can be also used to screen thousands of drugs, and in particular about 1,000 approved drugs per patient sample to search for drugs that selectively induce apoptosis in malignant cells. Surprisingly, a significant number of approved non-cytotoxic drugs were shown induce apoptosis in malignant cells with the same efficacy as the approved cytotoxic drugs for each indication. Figure 6 shows how 5 non-cytotoxic drugs (left) not approved for hematological malignancies eliminate CLL malignant cells similar efficacy as 3 approved cytotoxic CLL drugs (right). Figure 7 shows dose responses of one of these drugs, the antidepressant paroxetine, demonstrating that the drug induces apoptosis preferentially in malignant B cells versus healthy T and NK cells. Figure 8 shows how one of these non-cytotoxic drugs, sertraline, eliminates malignant CLL cells faster than the approved cytotoxic CLL drugs (24 versus 48 hours) (left). Three of the five most effective non-cytotoxic drugs are the antidepressants paroxetine, fluoxetine, and sertraline-drugs that belong to the same pharmacological family. Figure 9 shows how only 3 out of 6 serotonin reuptake inhibitors are effective in inducing apoptosis in malignant CLL cells. This demonstrates that these effects are not necessarily related to a pharmacological class of drugs, and that the ex vivo personalized medicine test proposed herein in can be used to identify these activities.

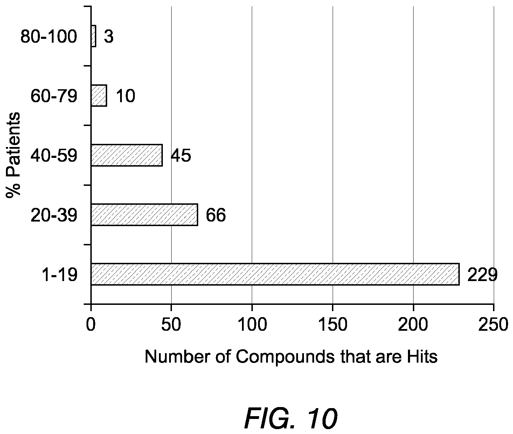

The unexpected finding that multiple safe non-cytotoxic approved drugs could be efficacious against tumor cells prompted a broader evaluation. First, only a few such drugs were effective in any given sample, discarding a non-selective effect. Figure 10, derived from a screening of 2,000 drugs in 23 CLL samples, shows how the efficacy of these approved non-cytotoxic drugs can vary tremendously from patient to patient. Drugs were defined as effective if they killed more than 80% of malignant cells, a standard similar to most effective cytotoxic drugs. While only 3 drugs were effective in more than 80% of the patients, 229 drugs were effective in less than 20% of the patient samples. This indicates that non-cytotoxic drugs can be effective against malignant cells ex vivo, but they show a very large degree of patient-to-patient variability. Nonetheless, in almost every CLL patient sample, 5-10 non-cytotoxic drugs were found effective against malignant cells ex vivo. Although the predictability of this effect in vivo is unknown, with pharmacokinetics and other factors such as formulation potentially playing a role, the effect of 5-10 such non-cytotoxic drugs administered to a patient could represent a significant therapeutic benefit.

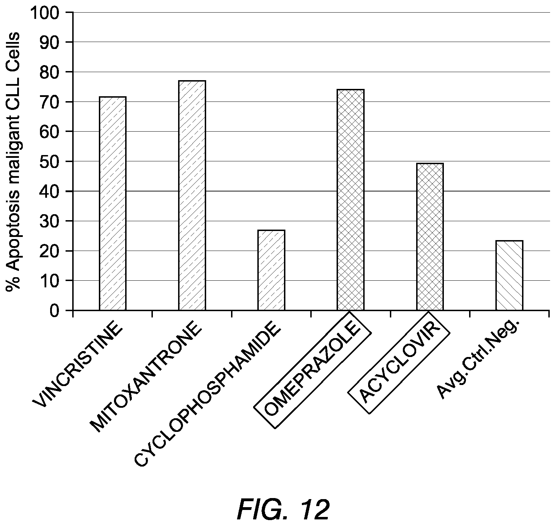

Some of the non-cytotoxic drugs that are effective ex vivo are drugs used to palliate the effects of the cytotoxic drugs that are administered to patients with a hematological malignancy (*i*.*e*., concomitant drugs). Figure 12 shows an example of a CLL sample for which the proton pump inhibitor omeprazole and the antiviral acyclovir showed significant efficacy against malignant cells ex vivo, similar to the efficacy of cytotoxic drugs. Table 4 lists some of these concomitant drugs

**Table 4. Concomitant Drugs**

| **Drug** | **Indication** |
|---|---|
| Aluminum Oxide Hydrate | Antacid |
| Lorazepam | Anti-anxiety agent |
| Amikacin | Antibiotic (Aminoglucoside) |
| Meropenem | Antibiotic (Betalactamic) |
| Cefepime | Antibiotic (Cephalosporin) |
| Vancomycin | Antibiotic (Glycopeptide) |
| Teicoplanin | Antibiotic (Glycopeptide) |
| Ondansetron | Antiemetic |
| Dexamethasone | Anti-inflammatory, Immunosupressor, Glucocorticoid |
| Amphotericin B (liposomal) | Antimycotic |
| Caspofungin | Antimycotic |
| Itraconazole | Antimycotic |
| Fluconazole | Antimycotic |
| Voriconazole | Antimycotic |
| Trimethoprim & Sulfamethoxazole | Bacteriostatic |
| G-CSF | Granulocyte colony-stimulating factor |
| Ranitidine | Histamine H2-receptor antagonist |
| Rasburicase | Hyperuricemia treatment |
| Paracetamol | Non-steroidal anti-inflammatory |
| Metamizole | Non-steroidal anti-inflammatory |
| Morphine chloride | Opiate analgesic |
| Omeprazole | Proton pump inhibitor |
| Paroxetine | Antidepressant |
| Fluoxetine | Antidepressant |
| Sertraline | Antidepressant |

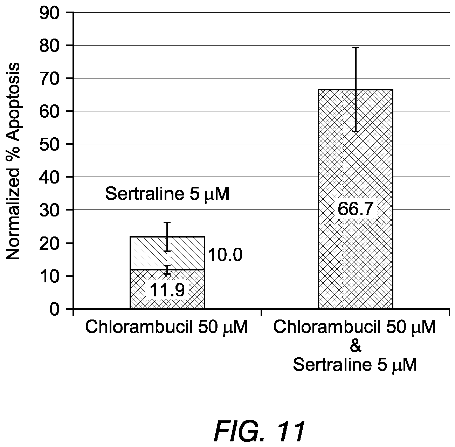

Some of the non-cytotoxic approved drugs could be therapeutically beneficial for potentiating the effect of cytotoxic drugs (*i*.*e*., as chemosensitizing agents). An example is shown in Figure 11, where low concentrations of the antidepressant sertraline potentiated the efficacy of low concentrations of the cytotoxic drug chlorambucil.

Because the present methods are intended to analyze large numbers of variables, 96-well plates have been designed to explore potential variations in polytherapy treatments. Other plates, including plates with larger or smaller numbers of wells, can also be used. In one embodiment, 1536 well plates are used. In another embodiment, 384 well plates are used. In another embodiment, 96 well plates are used.

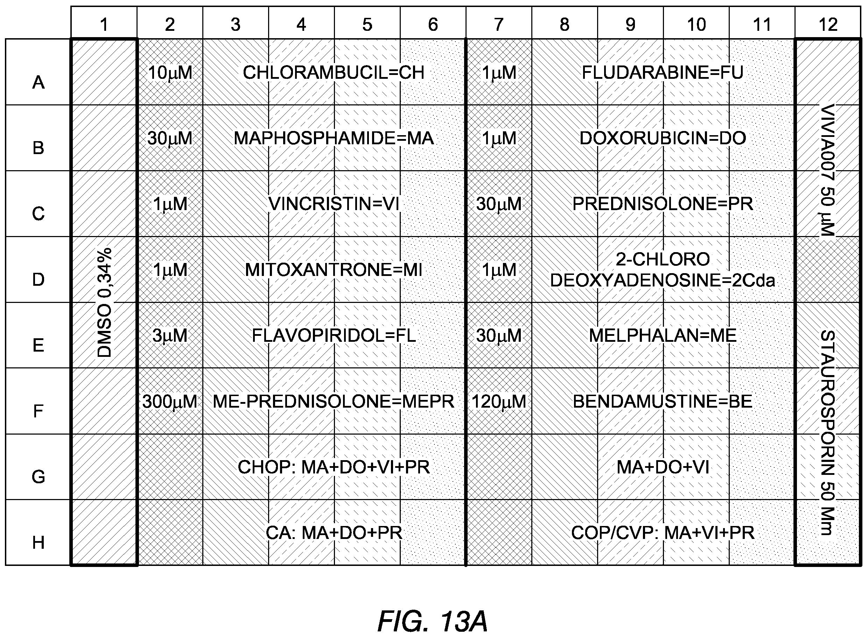

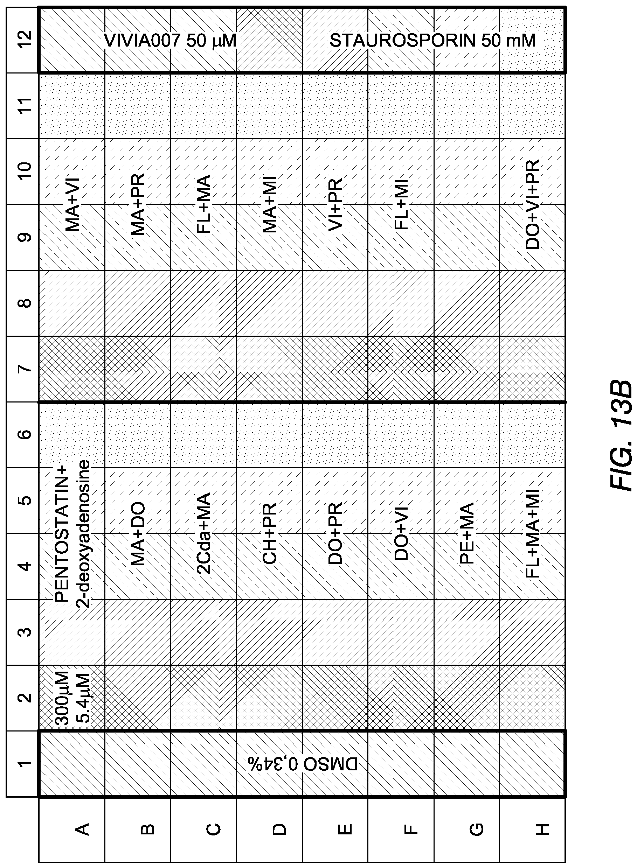

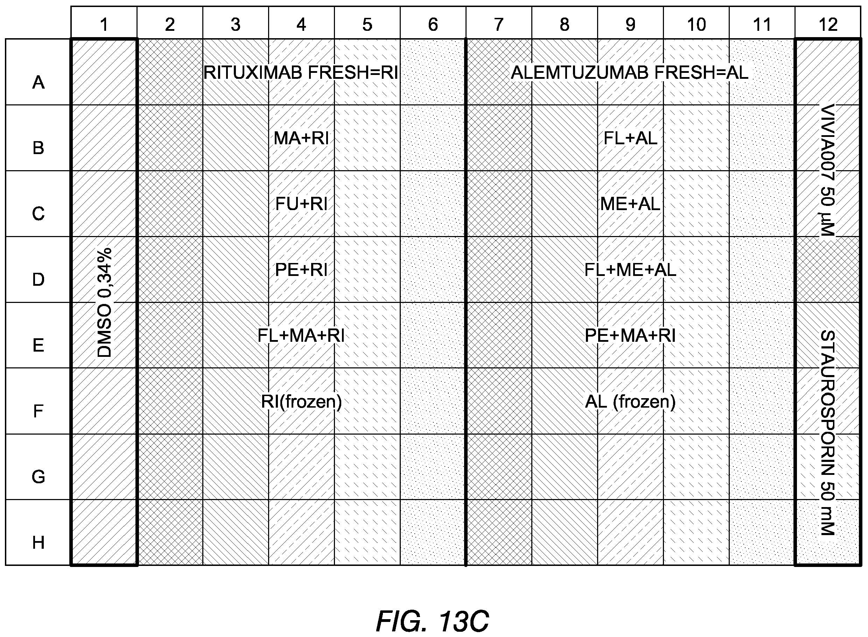

Figures 13-18 and Examples 9-14 illustrate the use of a 96 well plate format for the analysis of patient samples for the following indications: chronic lymphocytic leukemia, acute lymphoblastic leukemia, multiple myeloma, myelodysplastic syndrome, acute myeloblastic leukemia (not M3), and acute myeloblastic leukemia M3. The plate design for each indication comprises the drugs currently meeting the Spanish Program for the Treatment of Hematological Malignancies (Programa para el Tratamiento de Hemopatías Malignas (PETHEMA)) treatment protocol for the indication.

In one embodiment, the method analyzes drugs selected from the approved protocols of a clinical authority. In a specific embodiment, the method analyzes drugs selected from the PETHEMA treatment protocol. The well design utilizes drugs prescribed for monotherapy under the PETHEMA treatment protocol and also utilizes combinations of monotherapy drugs. Additionally, the design utilizes drugs prescribed to palliate side effects of the PETHEMA treatment protocol and also utilizes combinations of these drugs.

In an embodiment, the method analyzes cytotoxic drugs, including approved drugs and drugs not yet approved in clinical trials. In another embodiment, the method analyzes combinations of cytotoxic drugs. In a further embodiment, the method analyzes drugs prescribed to treat side effects of cytotoxic drugs. In a further embodiment, the method analyzes combinations of drugs prescribed to treat side effects of cytotoxic drugs. Furthermore, the well design utilizes combinations of cytotoxic drugs and drugs prescribed to treat side effects of cytotoxic drugs. In an embodiment, the method analyzes combinations of cytotoxic drugs and drugs prescribed to treat side effects of cytotoxic drugs. In another embodiment, the method analyzes any and all non-cytotoxic drugs, approved or in clinical trials, prescribed for any and all indications. In a further embodiment, the method analyzes combinations of non-cytotoxic drugs. For example, the plate design can utilize combinations of cytotoxic drugs and non-cytotoxic drugs. In an embodiment, the method analyzes combinations of cytotoxic drugs and non-cytotoxic drugs.

Treatments for hematological neoplasms are dictated by a certain limited number of treatment protocols agreed upon by hematologists. These protocols define the polytherapy regimen for both cytotoxic and additional combination drugs, including dosage and timing of each drug. The protocols differ depending upon variables such as the age, well-being, and disease state of each patient. Protocols can also vary from country to country, but are typically well followed within a country. There are still significant variations within these protocols in terms of ranges of dosages and different drug compositions that require tens to hundreds of conditions to be explored. In one embodiment, clinically validated reagents are used to evaluate cellular apoptosis. In another embodiment, clinically validated reagents are used in combination with antibodies to identify subtypes of tumor cells. In another embodiment, the reagents used to identify subtypes of tumor cells are defined according to the recent Euroflow normative (van Dongen et al., EuroFlow antibody panels for standardized n-dimensional flow cytometric immunophenotyping of normal, reactive and malignant leukocytes, 14th EHA Congress, Berlin, DE 4 June 2009: to be published in Leukemia 2010 (in press)). In another embodiment, drug compositions are selected from a protocol for hematological treatment used in a particular country. In another embodiment, drug compositions are selected from an older protocol for hematological treatment used in a particular country. In another embodiment, drug compositions are selected from an experimental protocol used in a particular country, defined as a new combination of approved drugs, for hematological treatment. In another embodiment, drug compositions are selected from a protocol, including drugs being evaluated in a clinical trial for hematological treatment.

The effect of each drug used in a treatment protocol should preferably be explored individually. However, this exploration should not be construed as a limitation. Not only should monotherapy drugs be explored individually using the methods described herein, but also drugs typically administered only in combinations. For example, individual screening of drugs that are typically administered only in combination can provide data allowing for the determination of the individual effects of these drugs.

In one embodiment, monotherapy drugs are individually analyzed. In another embodiment, drugs typically administered only in a combination are individually analyzed. Non-cytotoxic drugs commonly used in conjunction with cytotoxic drugs should also be explored (*e*.*g*., as in Figure 12). This includes antibiotics, antiemetics (anti-nauseas), antacids, antivirals, etc. In one embodiment, the method analyzes the ability of omeprazole to induce apoptosis in a patient sample. In another embodiment, the method analyzes the ability of acyclovir to induce apoptosis in a patient sample.

Indeed, the methods described herein have been used to demonstrate that some non-cytotoxic drugs, such as paroxetine and sertraline, can modulate the effect of cytotoxic drugs such as fludarabine and chlorambucil, respectively-potentiating their efficacy (*e*.*g*., as shown in Figure 11). As Figure 6 indicates, some of these non-cytotoxic drugs administered alone can induce apoptosis ex vivo in malignant cells with efficacy similar to that of approved cytotoxic drugs. In one embodiment, the method uses non-cytotoxic drugs to induce apoptosis in a patient sample. In another embodiment, the method uses combinations of non-cytotoxic drugs to induce apoptosis in a patient sample. In another embodiment, the method uses combinations of cytotoxic and non-cytotoxic drugs to induce apoptosis in a patient sample.

Unexpectedly, certain non-cytotoxic drugs eliminate malignant cells without damaging healthy cells, indicating that such drugs selectively attack the malignant cells (*e*.*g*., as seen in Figure 12). Such an unexpected result may have far-reaching implications for the treatment of hematological neoplasms. In one embodiment, the method uses non-cytotoxic drugs to selectively induce apoptosis in neoplastic cells. In an embodiment, the ex vivo therapeutic index is greater than about 1. In another embodiment, the ex vivo therapeutic index is greater than about 5. In another embodiment, the ex vivo therapeutic index is greater than about 10. In some embodiments, the methods described herein allow for the discrimination between leukemic cells and normal cells in tissues involved in a hematological neoplasms, such as blood, bone marrow, lymph node, or spleen samples.

Additionally, the ability of non-cytotoxic drugs to induce apoptosis varies within pharmacological classes of drugs (*e*.*g*., as seen in Figure 9), as well as between pharmacological classes of drugs. In one embodiment, the method analyzes drugs selected from the same pharmacological class as the drugs administered to a patient for the treatment of a certain indication or to palliate the side effects of treatment of a certain indication. In a specific embodiment, the method analyzes selective serotonin reuptake inhibitors. Furthermore, the methods described herein are not limited to the analysis of only cytotoxic drugs or only non-cytotoxic drugs. Indeed, there are instances in which the combination of a non-cytotoxic drug with a cytotoxic drug is desirable because the combination can have a greater ability to induce apoptosis in a patient sample relative to the ability of the cytotoxic drug alone (*e*.*g*., as seen in Figure 11).

The present system is fully capable of analyzing combinations of two classes of drugs, such as cytotoxic and non-cytotoxic drugs that are typically administered together. In one embodiment, non-cytotoxic drugs that are prescribed for patients who are administered cytotoxic drugs are analyzed. For example, the methods described herein can be used to analyze a patient sample treated with the cytotoxic drug fludarabine and a non-cytotoxic selective serotonin reuptake inhibitor. In a specific embodiment, the method is used to analyze a patient sample treated with the cytotoxic drug fludarabine and the non-cytotoxic drug paroxetine. For hematological neoplasms, patient drug regimens can include multiple drugs combinations. In one embodiment, drugs prescribed for hematological indications are analyzed in various combinations. For example, each patient could be administered from 8 to 10 drugs on average. In one embodiment, 5 or more drug compositions are analyzed. Preferable designs of plates for some of the major indications are shown in the Examples below.

The current strategy of protocol-based treatments for hematological neoplasms is a consequence of drug development stagnation. This stagnation has enabled hematologists to familiarize themselves with particular drugs and to develop a reasonable estimate of each drug's best combinations. In one embodiment, the methods described herein are used to validate current scientific expectations for drug compositions. However, two factors are dramatically changing current strategy of protocol-based treatments. First, as depicted in Figure 4, the realization that each patient responds differently to chemotherapy has recently brought personalized medicine to the forefront of medical research. The revolution brought by molecular biology techniques and the decoding of the human genome has generated a major focus on genomic analysis of patient samples with hematological neoplasms. However, 10 to 15 years of genomic research has enabled the stratification of patient in risk subpopulations, but has not been capable of personalizing the treatment to individual patients. The consequence of this realization creates a desire to match individual patients with their optimal treatment using a personalized medicine test. However, current protocols are estimated to explore less than 1-5% of the available therapeutic space that the platform described herein can explore (as depicted in Figure 5). Second, there are a significant number of new drugs recently approved for hematological neoplasms, and several late stage clinical candidates also exist. Consequently, these diseases are quickly transitioning from a scenario of the same old drugs prescribed for many years to a scenario with many new drugs being approved in a few years. In one embodiment, the methods described herein are used to evaluate old drugs, new drugs, late stage clinical candidates, or combinations thereof.

The methods described herein are useful for selecting drugs on an individualized patient basis and for identifying trends in treatment protocols that will be useful for selecting drugs for patients having similar indications and responses to current drug regimens. Every patient will have these compounds at selected concentrations in their bloodstream and bone marrow in order to eliminate malignant cells. One advantage of the polytherapy personalized medicine test described herein is the ability to explore many different drug compositions, sometimes reaching 8 to 10 drugs administered concurrently. In one embodiment, multiple drugs are administered concurrently to a patient. In another embodiment, multiple drugs are administered in series to a patient. Many of the drugs provided herein have not been evaluated for administration in combination. As shown below in the Examples, clear and dramatic effects on the induction of apoptosis for these drugs in combination can been observed.

Another advantage is the ability to determine optimal drug compositions on a personalized basis. As indicated in Figure 10, there is a large amount of variability for a patient's response to a certain drug compositions. In fact, only three drugs induced apoptosis in greater than 80% of the neoplastic cells for greater than 80% of the 23 patient samples. In contrast, 229 different drugs induced apoptosis in greater than 80% of the neoplastic cells for less than 20% (1-4) patient samples. This suggests that most non-cytotoxic drugs are effective in very few patients and demonstrates a larger degree of person-to-person variation than for cytotoxic drugs. However, patients with hematological neoplasms are commonly administered 5-10 non-cytotoxic concomitant drugs to palliate the effect of the cytotoxic drugs. Thus, the additive effect of selecting among these concomitant medicines a subgroup that shows significant efficacy in inducing apoptosis of malignant cells ex vivo, such as in Figure 12, can be significant.

In addition to identifying the potentially most efficacious drugs for an individual patient, these results also enable the stratification of patients into subgroups, and the possibility of new treatment protocols for these subgroups, including for cytotoxic and non-cytotoxic drugs. In one embodiment, a drug treatment protocol is selected on an individual patient basis. In another embodiment, a drug treatment protocol is selected based on its efficacy in 1-4 patient samples. In another embodiment, a drug treatment protocol is selected based on its efficacy in 5-9 patient samples. In another embodiment, a drug treatment protocol is selected based on its efficacy 10-14 patient samples. In another embodiment, a drug treatment protocol is selected based on its efficacy in 15-19 patient samples. In another embodiment, a drug treatment protocol is selected based on its efficacy in greater than 20 patient samples. The methods described herein afford more choices for treatment protocols than are currently available.

One advantage of a personalized medicine test is its ability to optimize a particular drug regimen on an individual basis. In a polytherapy regimen, where several different drugs are administered in combination to a patient, the pharmacokinetics and typical dose response curves of an individual drug may be unconventional. Using the methods described herein, optimal dosages may be observed for both neoplastic and normal cells based upon the recognition of optima in a dose response curve for a particular patient.

Various drug and drug combinations can be utilized in the methods and devices described herein. For example a drug combination comprising cytotoxic drugs can be used. Also, a drug combination comprising non-cytotoxic drugs can be used. Furthermore, a drug combination of cytotoxic and non-cytotoxic drugs can be used.

Some examples of cytotoxic compounds that can be used alone or in combination with other compounds include fludarabine (designated as "1"), chlorambucil (designated as "2"), mitoxantrone (designated as "3"), vincristine (designated as "4"), mitoxantrone (designated as "5"), cyclophosphamide (designated as "6"), adriamycin (designated as "7"), and doxorubicin (designated as "8").

Some examples of non-cytotoxic compounds that can be used alone or in combination with other compounds include 5-Azacitidine (designated as "1"), alemtuzumab (designated as "2"), aminopterin (designated as "3"), Amonafide (designated as "4"), Amsacrine (designated as "5"), CAT-8015 (designated as "6"), Bevacizumab (designated as "7"), ARR Y520 (designated as "8"), arsenic trioxide (designated as "9"), AS1413 (designated as "10"), Atra (designated as "11"), AZD 6244 (designated as "12"), AZD1152 (designated as "13"), Banoxantrone (designated as "14"), Behenoylara-C (designated as "15"), Bendamustine (designated as "16"), Bleomycin (designated as "17"), Blinatumomab (designated as "18"), Bortezomib (designated as "19"), Busulfan (designated as "20"), carboplatin (designated as "21"), CEP-701 (designated as "22"), Chlorambucil (designated as "23"), Chloro Deoxiadenosine (designated as "24"), Cladribine (designated as "25"), clofarabine (designated as "26"), CPX-351 (designated as "27"), Cyclophosphamide (designated as "28"), Cyclosporine (designated as "29"), Cytarabine (designated as "30"), Cytosine Arabinoside (designated as "31"), Dasatinib (designated as "32"), Daunorubicin (designated as "33"), decitabine (designated as "34"), Deglycosylated-ricin-A chain-conjugated anti-CD19/anti-CD22 immunotoxins (designated as "35"), Dexamethasone (designated as "36"), Doxorubicine (designated as "37"), Elacytarabine (designated as "38"), entinostat (designated as "39"), epratuzumab (designated as "40"), Erwinase (designated as "41"), Etoposide (designated as "42"), everolimus (designated as "43"), Exatecan mesilate (designated as "44"), flavopiridol (designated as "45"), fludarabine (designated as "46"), forodesine (designated as "47"), Gemcitabine (designated as "48"), Gemtuzumab-ozogamicin (designated as "49"), Homoharringtonine (designated as "50"), Hydrocortisone (designated as "51"), Hydroxycarbamide (designated as "52"), Idarubicin (designated as "53"), Ifosfamide (designated as "54"), Imatinib (designated as "55"), interferon alpha 2a (designated as "56"), iodine I 131 monoclonal antibody BC8 (designated as "57"), Iphosphamide (designated as "58"), isotretinoin (designated as "59"), Laromustine (designated as "60"), L-Asparaginase (designated as "61"), Lenalidomide (designated as "62"), Lestaurtinib (designated as "63"), Maphosphamide (designated as "64"), Melphalan (designated as "65"), Mercaptopurine (designated as "66"), Methotrexate (designated as "67"), Methylprednisolone (designated as "68"), Methylprednisone (designated as "69"), Midostaurin (designated as "70"), Mitoxantrone (designated as "71"), Nelarabine (designated as "72"), Nilotinib (designated as "73"), Oblimersen (designated as "74"), Paclitaxel (designated as "75"), panobinostat (designated as "76"), Pegaspargase (designated as "77"), Pentostatin (designated as "78"), Pirarubicin (designated as "79"), PKC412 (designated as "80"), Prednisolone (designated as "81"), Prednisone, PSC-833 (designated as "82"), Rapamycin (designated as "83"), Rituximab (designated as "84"), Rivabirin (designated as "85"), Sapacitabine (designated as "86"), Dinaciclib (designated as "87"), Sorafenib (designated as "88"), Sorafenib (designated as "89"), STA-9090 (designated as "90"), tacrolimus (designated as "91"), tanespimycin (designated as "92"), temsirolimus (designated as "93"), Teniposide (designated as "94"), Terameprocol (designated as "95"), Thalidomide (designated as "96"), Thioguanine (designated as "97"), Thiotepa (designated as "98"), Tipifarnib (designated as "99"), Topotecan (designated as "100"), Treosulfan (designated as "101"), Troxacitabine (designated as "102"), Vinblastine (designated as "103"), Vincristine (designated as "104"), Vindesine (designated as "105"), Vinorelbine (designated as "106"), Voreloxin (designated as "107"), Vorinostat (designated as "108"), Etoposide (designated as "109"), and Zosuquidar (designated as "110").

Some examples of non-cytotoxic compounds that can be used alone or in combination with other compounds include Aluminum Oxide Hydrate (designated as "111"), Lorazepam (designated as "112"), Amikacine (designated as "113"), Meropenem (designated as "114"), Cefepime (designated as "115"), Vancomycin (designated as "116"), Teicoplanin (designated as "117"), Ondansetron (designated as "118"), Dexamethasone (designated as "119"), Amphotericin B (liposomal) (designated as "120"), Caspofugin (designated as "121"), Itraconazole (designated as "122"), Fluconazole (designated as "123"), Voriconazole (designated as "124"), Trimetoprime (designated as "125"), sulfamethoxazole (designated as "126"), G-CSF (designated as "127"), Ranitidine (designated as "128"), Rasburicase (designated as "129"), Paracetamol (designated as "130"), Metamizole (designated as "131"), Morphine chloride (designated as "132"), Omeprazole (designated as "133"), Paroxetine (designated as "134"), Fluoxetine (designated as "135"), and Sertraline (designated as "136").

In addition, in most countries, particular drug combinations represent the preferred or standard cytotoxic therapies for treatment of AML, ALL, CLL, and NHL. These existing therapies can be assigned numerical designators, and in the following combinations, can be used in further combination with additional drugs.

Using the numerical designations set forth above in a #.# format, examples of two-compound combinations comprising at least one cytotoxic compounds are listed below, which may or may not further comprise other compounds in the combination:
1.2, 1.3, 1.4, 1.5, 1.6, 1.7, 1.8, 1.9, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28; 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, 1.93, 1.94, 1.95, 1.96, 1.97, 1.98, 1.99, 1.100, 1.101, 1.102, 1.103, 1.104, 1.105, 1.106, 1.107, 1.108, 1.109, 1.110, 1.111, 1.112, 1.113, 1.114, 1.115, 1.116, 1.117, 1.118, 1.119, 1.120, 1.121, 1.122, 1.123, 1.124, 1.125, 1.126, 1.127, 1.128, 1.129, 1.130, 1.131, 1.132, 1.133, 1.134, 1.135, 1.136; 2.3, 2.4, 2.5, 2.6, 2.7, 2.8, 2.9, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28; 2.29, 2.30, 2.31, 2.32, 2.33, 2.34, 2.35, 2.36, 2.37, 2.38, 2.39, 2.40, 2.41, 2.42, 2.43, 2.44, 2.45, 2.46, 2.47, 2.48, 2.49, 2.50, 2.51, 2.52, 2.53, 2.54, 2.55, 2.56, 2.57, 2.58, 2.59, 2.60, 2.61, 2.62, 2.63, 2.64, 2.65, 2.66, 2.67, 2.68, 2.69, 2.70, 2.71, 2.72, 2.73, 2.74, 2.75, 2.76, 2.77, 2.78, 2.79, 2.80, 2.81, 2.82, 2.83, 2.84, 2.85, 2.86, 2.87, 2.88, 2.89, 2.90, 2.91, 2.92, 2.93, 2.94, 2.95, 2.96, 2.97, 2.98, 2.99, 2.100, 2.101, 2.102, 2.103, 2.104, 2.105, 2.106, 2.107, 2.108, 2.109, 2.110, 2.111, 2.112, 2.113, 2.114, 2.115, 2.116, 2.117, 2.118, 2.119, 2.120, 2.121, 2.122, 2.123, 2.124, 2.125, 2.126, 2.127, 2.128, 2.129, 2.130, 2.131, 2.132, 2.133, 2.134, 2.135, 2.136; 3.4, 3.5, 3.6, 3.7, 3.8, 3.9, 3.10, 3.11, 3.12, 3.13, 3.14, 3.15, 3.16, 3.17, 3.18, 3.19, 3.20, 3.21, 3.22, 3.23, 3.24, 3.25, 3.26, 3.27, 3.28; 3.29, 3.30, 3.31, 3.32, 3.33, 3.34, 3.35, 3.36, 3.37, 3.38, 3.39, 3.40, 3.41, 3.42, 3.43, 3.44, 3.45, 3.46, 3.47, 3.48, 3.49, 3.50, 3.51, 3.52, 3.53, 3.54, 3.55, 3.56, 3.57, 3.58, 3.59, 3.60, 3.61, 3.62, 3.63, 3.64, 3.65, 3.66, 3.67, 3.68, 3.69, 3.70, 3.71, 3.72, 3.73, 3.74, 3.75, 3.76, 3.77, 3.78, 3.79, 3.80, 3.81, 3.82, 3.83, 3.84, 3.85, 3.86, 3.87, 3.88, 3.89, 3.90, 3.91, 3.92, 3.93, 3.94, 3.95, 3.96, 3.97, 3.98, 3.99, 3.100, 3.101, 3.102, 3.103, 3.104, 3.105, 3.106, 3.107, 3.108, 3.109, 3.110, 3.111, 3.112, 3.113, 3.114, 3.115, 3.116, 3.117, 3.118, 3.119, 3.120, 3.121, 3.122, 3.123, 3.124, 3.125, 3.126, 3.127, 3.128, 3.129, 3.130, 3.131, 3.132, 3.133, 3.134, 3.135, 3.136; 4.5, 4.6, 4.7, 4.8, 4.9, 4.10, 4.11, 4.12, 4.13, 4.14, 4.15, 4.16, 4.17, 4.18, 4.19, 4.20, 4.21, 4.22, 4.23, 4.24, 4.25, 4.26, 4.27, 4.28; 4.29, 4.30, 4.31, 4.32, 4.33, 4.34, 4.35, 4.36, 4.37, 4.38, 4.39, 4.40, 4.41, 4.42, 4.43, 4.44, 4.45, 4.46, 4.47, 4.48, 4.49, 4.50, 4.51, 4.52, 4.53, 4.54, 4.55, 4.56, 4.57, 4.58, 4.59, 4.60, 4.61, 4.62, 4.63, 4.64, 4.65, 4.66, 4.67, 4.68, 4.69, 4.70, 4.71, 4.72, 4.73, 4.74, 4.75, 4.76, 4.77, 4.78, 4.79, 4.80, 4.81, 4.82, 4.83, 4.84, 4.85, 4.86, 4.87, 4.88, 4.89, 4.90, 4.91, 4.92, 4.93, 4.94, 4.95, 4.96, 4.97, 4.98, 4.99, 4.100, 4.101, 4.102, 4.103, 4.104, 4.105, 4.106, 4.107, 4.108, 4.109, 4.110, 4.111, 4.112, 4.113, 4.114, 4.115, 4.116, 4.117, 4.118, 4.119, 4.120, 4.121, 4.122, 4.123, 4.124, 4.125, 4.126, 4.127, 4.128, 4.129, 4.130, 4.131, 4.132, 4.133, 4.134, 4.135, 4.136; 5.6, 5.7, 5.8, 5.9, 5.10, 5.11, 5.12, 5.13, 5.14, 5.15, 5.16, 5.17, 5.18, 5.19, 5.20, 5.21, 5.22, 5.23, 5.24, 5.25, 5.26, 5.27, 5.28; 5.29, 5.30, 5.31, 5.32, 5.33, 5.34, 5.35, 5.36, 5.37, 5.38, 5.39, 5.40, 5.41, 5.42, 5.43, 5.44, 5.45, 5.46, 5.47, 5.48, 5.49, 5.50, 5.51, 5.52, 5.53, 5.54, 5.55, 5.56, 5.57, 5.58, 5.59, 5.60, 5.61, 5.62, 5.63, 5.64, 5.65, 5.66, 5.67, 5.68, 5.69, 5.70, 5.71, 5.72, 5.73, 5.74, 5.75, 5.76, 5.77, 5.78, 5.79, 5.80, 5.81, 5.82, 5.83, 5.84, 5.85, 5.86, 5.87, 5.88, 5.89, 5.90, 5.91, 5.92, 5.93, 5.94, 5.95, 5.96, 5.97, 5.98, 5.99, 5.100, 5.101, 5.102, 5.103, 5.104, 5.105, 5.106, 5.107, 5.108, 5.109, 5.110, 5.111, 5.112, 5.113, 5.114, 5.115, 5.116, 5.117, 5.118, 5.119, 5.120, 5.121, 5.122, 5.123, 5.124, 5.125, 5.126, 5.127, 5.128, 5.129, 5.130, 5.131, 5.132, 5.133, 5.134, 5.135, 5.136; 6.7, 6.8, 6.9, 6.10, 6.11, 6.12, 6.13, 6.14, 6.15, 6.16, 6.17, 6.18, 6.19, 6.20, 6.21, 6.22, 6.23, 6.24, 6.25, 6.26, 6.27, 6.28; 6.29, 6.30, 6.31, 6.32, 6.33, 6.34, 6.35, 6.36, 6.37, 6.38, 6.39, 6.40, 6.41, 6.42, 6.43, 6.44, 6.45, 6.46, 6.47, 6.48, 6.49, 6.50, 6.51, 6.52, 6.53, 6.54, 6.55, 6.56, 6.57, 6.58, 6.59, 6.60, 6.61, 6.62, 6.63, 6.64, 6.65, 6.66, 6.67, 6.68, 6.69, 6.70, 6.71, 6.72, 6.73, 6.74, 6.75, 6.76, 6.77, 6.78, 6.79, 6.80, 6.81, 6.82, 6.83, 6.84, 6.85, 6.86, 6.87, 6.88, 6.89, 6.90, 6.91, 6.92, 6.93, 6.94, 6.95, 6.96, 6.97, 6.98, 6.99, 6.100, 6.101, 6.102, 6.103, 6.104, 6.105, 6.106, 6.107, 6.108, 6.109, 6.110, 6.111, 6.112, 6.113, 6.114, 6.115, 6.116, 6.117, 6.118, 6.119, 6.120, 6.121, 6.122, 6.123, 6.124, 6.125, 6.126, 6.127, 6.128, 6.129, 6.130, 6.131, 6.132, 6.133, 6.134, 6.135, 6.136; 7.8, 7.9, 7.10, 7.11, 7.12, 7.13, 7.14, 7.15, 7.16, 7.17, 7.18, 7.19, 7.20, 7.21, 7.22, 7.23, 7.24, 7.25, 7.26, 7.27, 7.28; 7.29, 7.30, 7.31, 7.32, 7.33, 7.34, 7.35, 7.36, 7.37, 7.38, 7.39, 7.40, 7.41, 7.42, 7.43, 7.44, 7.45, 7.46, 7.47, 7.48, 7.49, 7.50, 7.51, 7.52, 7.53, 7.54, 7.55, 7.56, 7.57, 7.58, 7.59, 7.60, 7.61, 7.62, 7.63, 7.64, 7.65, 7.66, 7.67, 7.68, 7.69, 7.70, 7.71, 7.72, 7.73, 7.74, 7.75, 7.76, 7.77, 7.78, 7.79, 7.80, 7.81, 7.82, 7.83, 7.84, 7.85, 7.86, 7.87, 7.88, 7.89, 7.90, 7.91, 7.92, 7.93, 7.94, 7.95, 7.96, 7.97, 7.98, 7.99, 7.100, 7.101, 7.102, 7.103, 7.104, 7.105, 7.106, 7.107, 7.108, 7.109, 7.110, 7.111, 7.112, 7.113, 7.114, 7.115, 7.116, 7.117, 7.118, 7.119, 7.120, 7.121, 7.122, 7.123, 7.124, 7.125, 7.126, 7.127, 7.128, 7.129, 7.130, 7.131, 7.132, 7.133, 7.134, 7.135, 7.136; 8.9, 8.10, 8.11, 8.12, 8.13, 8.14, 8.15, 8.16, 8.17, 8.18, 8.19, 8.20, 8.21, 8.22, 8.23, 8.24, 8.25, 8.26, 8.27, 8.28; 8.29, 8.30, 8.31, 8.32, 8.33, 8.34, 8.35, 8.36, 8.37, 8.38, 8.39, 8.40, 8.41, 8.42, 8.43, 8.44, 8.45, 8.46, 8.47, 8.48, 8.49, 8.50, 8.51, 8.52, 8.53, 8.54, 8.55, 8.56, 8.57, 8.58, 8.59, 8.60, 8.61, 8.62, 8.63, 8.64, 8.65, 8.66, 8.67, 8.68, 8.69, 8.70, 8.71, 8.72, 8.73, 8.74, 8.75, 8.76, 8.77, 8.78, 8.79, 8.80, 8.81, 8.82, 8.83, 8.84, 8.85, 8.86, 8.87, 8.88, 8.89, 8.90, 8.91, 8.92, 8.93, 8.94, 8.95, 8.96, 8.97, 8.98, 8.99, 8.100, 8.101, 8.102, 8.103, 8.104, 8.105, 8.106, 8.107, 8.108, 8.109, 8.110, 8.111, 8.112, 8.113, 8.114, 8.115, 8.116, 8.117, 8.118, 8.119, 8.120, 8.121, 8.122, 8.123, 8.124, 8.125, 8.126, 8.127, 8.128, 8.129, 8.130, 8.131, 8.132, 8.133, 8.134, 8.135, 8.136; 9.10, 9.11, 9.12, 9.13, 9.14, 9.15, 9.16, 9.17, 9.18, 9.19, 9.20, 9.21, 9.22, 9.23, 9.24, 9.25, 9.26, 9.27, 9.28; 9.29, 9.30, 9.31, 9.32, 9.33, 9.34, 9.35, 9.36, 9.37, 9.38, 9.39, 9.40, 9.41, 9.42, 9.43, 9.44, 9.45, 9.46, 9.47, 9.48, 9.49, 9.50, 9.51, 9.52, 9.53, 9.54, 9.55, 9.56, 9.57, 9.58, 9.59, 9.60, 9.61, 9.62, 9.63, 9.64, 9.65, 9.66, 9.67, 9.68, 9.69, 9.70, 9.71, 9.72, 9.73, 9.74, 9.75, 9.76, 9.77, 9.78, 9.79, 9.80, 9.81, 9.82, 9.83, 9.84, 9.85, 9.86, 9.87, 9.88, 9.89, 9.90, 9.91, 9.92, 9.93, 9.94, 9.95, 9.96, 9.97, 9.98, 9.99, 9.100, 9.101, 9.102, 9.103, 9.104, 9.105, 9.106, 9.107, 9.108, 9.109, 9.110, 9.111, 9.112, 9.113, 9.114, 9.115, 9.116, 9.117, 9.118, 9.119, 9.120, 9.121, 9.122, 9.123, 9.124, 9.125, 9.126, 9.127, 9.128, 9.129, 9.130, 9.131, 9.132, 9.133, 9.134, 9.135, 9.136; 10.11, 10.12, 10.13, 10.14, 10.15, 10.16, 10.17, 10.18, 10.19, 10.20, 10.21, 10.22, 10.23, 10.24, 10.25, 10.26, 10.27, 10.28; 10.29, 10.30, 10.31, 10.32, 10.33, 10.34, 10.35, 10.36, 10.37, 10.38, 10.39, 10.40, 10.41, 10.42, 10.43, 10.44, 10.45, 10.46, 10.47, 10.48, 10.49, 10.50, 10.51, 10.52, 10.53, 10.54, 10.55, 10.56, 10.57, 10.58, 10.59, 10.60, 10.61, 10.62, 10.63, 10.64, 10.65, 10.66, 10.67, 10.68, 10.69, 10.70, 10.71, 10.72, 10.73, 10.74, 10.75, 10.76, 10.77, 10.78, 10.79, 10.80, 10.81, 10.82, 10.83, 10.84, 10.85, 10.86, 10.87, 10.88, 10.89, 10.90, 10.91, 10.92, 10.93, 10.94, 10.95, 10.96, 10.97, 10.98, 10.99, 10.100, 10.101, 10.102, 10.103, 10.104, 10.105, 10.106, 10.107, 10.108, 10.109, 10.110, 10.111, 10.112, 10.113, 10.114, 10.115, 10.116, 10.117, 10.118, 10.119, 10.120, 10.121, 10.122, 10.123, 10.124, 10.125, 10.126, 10.127, 10.128, 10.129, 10.130, 10.131, 10.132, 10.133, 10.134, 10.135, 10.136; 11.12, 11.13, 11.14, 11.15, 11.16, 11.17, 11.18, 11.19, 11.20, 11.21, 11.22, 11.23, 11.24, 11.25, 11.26, 11.27, 11.28; 11.29, 11.30, 11.31, 11.32, 11.33, 11.34, 11.35, 11.36, 11.37, 11.38, 11.39, 11.40, 11.41, 11.42, 11.43, 11.44, 11.45, 11.46, 11.47, 11.48, 11.49, 11.50, 11.51, 11.52, 11.53, 11.54, 11.55, 11.56, 11.57, 11.58, 11.59, 11.60, 11.61, 11.62, 11.63, 11.64, 11.65, 11.66, 11.67, 11.68, 11.69, 11.70, 11.71, 11.72, 11.73, 11.74, 11.75, 11.76, 11.77, 11.78, 11.79, 11.80, 11.81, 11.82, 11.83, 11.84, 11.85, 11.86, 11.87, 11.88, 11.89, 11.90, 11.91, 11.92, 11.93, 11.94, 11.95, 11.96, 11.97, 11.98, 11.99, 11.100, 11.101, 11.102, 11.103, 11.104, 11.105, 11.106, 11.107, 11.108, 11.109, 11.110, 11.111, 11.112, 11.113, 11.114, 11.115, 11.116, 11.117, 11.118, 11.119, 11.120, 11.121, 11.122, 11.123, 11.124, 11.125, 11.126, 11.127, 11.128, 11.129, 11.130, 11.131, 11.132, 11.133, 11.134, 11.135, 11.136; 12.13, 12.14, 12.15, 12.16, 12.17, 12.18, 12.19, 12.20, 12.21, 12.22, 12.23, 12.24, 12.25, 12.26, 12.27, 12.28; 12.29, 12.30, 12.31, 12.32, 12.33, 12.34, 12.35, 12.36, 12.37, 12.38, 12.39, 12.40, 12.41, 12.42, 12.43, 12.44, 12.45, 12.46, 12.47, 12.48, 12.49, 12.50, 12.51, 12.52, 12.53, 12.54, 12.55, 12.56, 12.57, 12.58, 12.59, 12.60, 12.61, 12.62, 12.63, 12.64, 12.65, 12.66, 12.67, 12.68, 12.69, 12.70, 12.71, 12.72, 12.73, 12.74, 12.75, 12.76, 12.77, 12.78, 12.79, 12.80, 12.81, 12.82, 12.83, 12.84, 12.85, 12.86, 12.87, 12.88, 12.89, 12.90, 12.91, 12.92, 12.93, 12.94, 12.95, 12.96, 12.97, 12.98, 12.99, 12.100, 12.101, 12.102, 12.103, 12.104, 12.105, 12.106, 12.107, 12.108, 12.109, 12.110, 12.111, 12.112, 12.113, 12.114, 12.115, 12.116, 12.117, 12.118, 12.119, 12.120, 12.121, 12.122, 12.123, 12.124, 12.125, 12.126, 12.127, 12.128, 12.129, 12.130, 12.131, 12.132, 12.133, 12.134, 12.135, 12.136; 13.14, 13.15, 13.16, 13.17, 13.18, 13.19, 13.20, 13.21, 13.22, 13.23, 13.24, 13.25, 13.26, 13.27, 13.28; 13.29, 13.30, 13.31, 13.32, 13.33, 13.34, 13.35, 13.36, 13.37, 13.38, 13.39, 13.40, 13.41, 13.42, 13.43, 13.44, 13.45, 13.46, 13.47, 13.48, 13.49, 13.50, 13.51, 13.52, 13.53, 13.54, 13.55, 13.56, 13.57, 13.58, 13.59, 13.60, 13.61, 13.62, 13.63, 13.64, 13.65, 13.66, 13.67, 13.68, 13.69, 13.70, 13.71, 13.72, 13.73, 13.74, 13.75, 13.76, 13.77, 13.78, 13.79, 13.80, 13.81, 13.82, 13.83, 13.84, 13.85, 13.86, 13.87, 13.88, 13.89, 13.90, 13.91, 13.92, 13.93, 13.94, 13.95, 13.96, 13.97, 13.98, 13.99, 13.100, 13.101, 13.102, 13.103, 13.104, 13.105, 13.106, 13.107, 13.108, 13.109, 13.110, 13.111, 13.112, 13.113, 13.114, 13.115, 13.116, 13.117, 13.118, 13.119, 13.120, 13.121, 13.122, 13.123, 13.124, 13.125, 13.126, 13.127, 13.128, 13.129, 13.130, 13.131, 13.132, 13.133, 13.134, 13.135, 13.136; 14.15, 14.16, 14.17, 14.18, 14.19, 14.20, 14.21, 14.22, 14.23, 14.24, 14.25, 14.26, 14.27, 14.28; 14.29, 14.30, 14.31, 14.32, 14.33, 14.34, 14.35, 14.36, 14.37, 14.38, 14.39, 14.40, 14.41, 14.42, 14.43, 14.44, 14.45, 14.46, 14.47, 14.48, 14.49, 14.50, 14.51, 14.52, 14.53, 14.54, 14.55, 14.56, 14.57, 14.58, 14.59, 14.60, 14.61, 14.62, 14.63, 14.64, 14.65, 14.66, 14.67, 14.68, 14.69, 14.70, 14.71, 14.72, 14.73, 14.74, 14.75, 14.76, 14.77, 14.78, 14.79, 14.80, 14.81, 14.82, 14.83, 14.84, 14.85, 14.86, 14.87, 14.88, 14.89, 14.90, 14.91, 14.92, 14.93, 14.94, 14.95, 14.96, 14.97, 14.98, 14.99, 14.100, 14.101, 14.102, 14.103, 14.104, 14.105, 14.106, 14.107, 14.108, 14.109, 14.110, 14.111, 14.112, 14.113, 14.114, 14.115, 14.116, 14.117, 14.118, 14.119, 14.120, 14.121, 14.122, 14.123, 14.124, 14.125, 14.126, 14.127, 14.128, 14.129, 14.130, 14.131, 14.132, 14.133, 14.134, 14.135, 14.136; 15.16, 15.17, 15.18, 15.19, 15.20, 15.21, 15.22, 15.23, 15.24, 15.25, 15.26, 15.27, 15.28; 15.29, 15.30, 15.31, 15.32, 15.33, 15.34, 15.35, 15.36, 15.37, 15.38, 15.39, 15.40, 15.41, 15.42, 15.43, 15.44, 15.45, 15.46, 15.47, 15.48, 15.49, 15.50, 15.51, 15.52, 15.53, 15.54, 15.55, 15.56, 15.57, 15.58, 15.59, 15.60, 15.61, 15.62, 15.63, 15.64, 15.65, 15.66, 15.67, 15.68, 15.69, 15.70, 15.71, 15.72, 15.73, 15.74, 15.75, 15.76, 15.77, 15.78, 15.79, 15.80, 15.81, 15.82, 15.83, 15.84, 15.85, 15.86, 15.87, 15.88, 15.89, 15.90, 15.91, 15.92, 15.93, 15.94, 15.95, 15.96, 15.97, 15.98, 15.99, 15.100, 15.101, 15.102, 15.103, 15.104, 15.105, 15.106, 15.107, 15.108, 15.109, 15.110, 15.111, 15.112, 15.113, 15.114, 15.115, 15.116, 15.117, 15.118, 15.119, 15.120, 15.121, 15.122, 15.123, 15.124, 15.125, 15.126, 15.127, 15.128, 15.129, 15.130, 15.131, 15.132, 15.133, 15.134, 15.135, 15.136; 16.17, 16.18, 16.19, 16.20, 16.21, 16.22, 16.23, 16.24, 16.25, 16.26, 16.27, 16.28; 16.29, 16.30, 16.31, 16.32, 16.33, 16.34, 16.35, 16.36, 16.37, 16.38, 16.39, 16.40, 16.41, 16.42, 16.43, 16.44, 16.45, 16.46, 16.47, 16.48, 16.49, 16.50, 16.51, 16.52, 16.53, 16.54, 16.55, 16.56, 16.57, 16.58, 16.59, 16.60, 16.61, 16.62, 16.63, 16.64, 16.65, 16.66, 16.67, 16.68, 16.69, 16.70, 16.71, 16.72, 16.73, 16.74, 16.75, 16.76, 16.77, 16.78, 16.79, 16.80, 16.81, 16.82, 16.83, 16.84, 16.85, 16.86, 16.87, 16.88, 16.89, 16.90, 16.91, 16.92, 16.93, 16.94, 16.95, 16.96, 16.97, 16.98, 16.99, 16.100, 16.101, 16.102, 16.103, 16.104, 16.105, 16.106, 16.107, 16.108, 16.109, 16.110, 16.111, 16.112, 16.113, 16.114, 16.115, 16.116, 16.117, 16.118, 16.119, 16.120, 16.121, 16.122, 16.123, 16.124, 16.125, 16.126, 16.127, 16.128, 16.129, 16.130, 16.131, 16.132, 16.133, 16.134, 16.135, 16.136; 17.18, 17.19, 17.20, 17.21, 17.22, 17.23, 17.24, 17.25, 17.26, 17.27, 17.28; 17.29, 17.30, 17.31, 17.32, 17.33, 17.34, 17.35, 17.36, 17.37, 17.38, 17.39, 17.40, 17.41, 17.42, 17.43, 17.44, 17.45, 17.46, 17.47, 17.48, 17.49, 17.50, 17.51, 17.52, 17.53, 17.54, 17.55, 17.56, 17.57, 17.58, 17.59, 17.60, 17.61, 17.62, 17.63, 17.64, 17.65, 17.66, 17.67, 17.68, 17.69, 17.70, 17.71, 17.72, 17.73, 17.74, 17.75, 17.76, 17.77, 17.78, 17.79, 17.80, 17.81, 17.82, 17.83, 17.84, 17.85, 17.86, 17.87, 17.88, 17.89, 17.90, 17.91, 17.92, 17.93, 17.94, 17.95, 17.96, 17.97, 17.98, 17.99, 17.100, 17.101, 17.102, 17.103, 17.104, 17.105, 17.106, 17.107, 17.108, 17.109, 17.110, 17.111, 17.112, 17.113, 17.114, 17.115, 17.116, 17.117, 17.118, 17.119, 17.120, 17.121, 17.122, 17.123, 17.124, 17.125, 17.126, 17.127, 17.128, 17.129, 17.130, 17.131, 17.132, 17.133, 17.134, 17.135, 17.136; 18.19, 18.20, 18.21, 18.22, 18.23, 18.24, 18.25, 18.26, 18.27, 18.28; 18.29, 18.30, 18.31, 18.32, 18.33, 18.34, 18.35, 18.36, 18.37, 18.38, 18.39, 18.40, 18.41, 18.42, 18.43, 18.44, 18.45, 18.46, 18.47, 18.48, 18.49, 18.50, 18.51, 18.52, 18.53, 18.54, 18.55, 18.56, 18.57, 18.58, 18.59, 18.60, 18.61, 18.62, 18.63, 18.64, 18.65, 18.66, 18.67, 18.68, 18.69, 18.70, 18.71, 18.72, 18.73, 18.74, 18.75, 18.76, 18.77, 18.78, 18.79, 18.80, 18.81, 18.82, 18.83, 18.84, 18.85, 18.86, 18.87, 18.88, 18.89, 18.90, 18.91, 18.92, 18.93, 18.94, 18.95, 18.96, 18.97, 18.98, 18.99, 18.100, 18.101, 18.102, 18.103, 18.104, 18.105, 18.106, 18.107, 18.108, 18.109, 18.110, 18.111, 18.112, 18.113, 18.114, 18.115, 18.116, 18.117, 18.118, 18.119, 18.120, 18.121, 18.122, 18.123, 18.124, 18.125, 18.126, 18.127, 18.128, 18.129, 18.130, 18.131, 18.132, 18.133, 18.134, 18.135, 18.136; 19.20, 19.21, 19.22, 19.23, 19.24, 19.25, 19.26, 19.27, 19.28; 19.29, 19.30, 19.31, 19.32, 19.33, 19.34, 19.35, 19.36, 19.37, 19.38, 19.39, 19.40, 19.41, 19.42, 19.43, 19.44, 19.45, 19.46, 19.47, 19.48, 19.49, 19.50, 19.51, 19.52, 19.53, 19.54, 19.55, 19.56, 19.57, 19.58, 19.59, 19.60, 19.61, 19.62, 19.63, 19.64, 19.65, 19.66, 19.67, 19.68, 19.69, 19.70, 19.71, 19.72, 19.73, 19.74, 19.75, 19.76, 19.77, 19.78, 19.79, 19.80, 19.81, 19.82, 19.83, 19.84, 19.85, 19.86, 19.87, 19.88, 19.89, 19.90, 19.91, 19.92, 19.93, 19.94, 19.95, 19.96, 19.97, 19.98, 19.99, 19.100, 19.101, 19.102, 19.103, 19.104, 19.105, 19.106, 19.107, 19.108, 19.109, 19.110, 19.111, 19.112, 19.113, 19.114, 19.115, 19.116, 19.117, 19.118, 19.119, 19.120, 19.121, 19.122, 19.123, 19.124, 19.125, 19.126, 19.127, 19.128, 19.129, 19.130, 19.131, 19.132, 19.133, 19.134, 19.135, 19.136; 20.21, 20.22, 20.23, 20.24, 20.25, 20.26, 20.27, 20.28; 20.29, 20.30, 20.31, 20.32, 20.33, 20.34, 20.35, 20.36, 20.37, 20.38, 20.39, 20.40, 20.41, 20.42, 20.43, 20.44, 20.45, 20.46, 20.47, 20.48, 20.49, 20.50, 20.51, 20.52, 20.53, 20.54, 20.55, 20.56, 20.57, 20.58, 20.59, 20.60, 20.61, 20.62, 20.63, 20.64, 20.65, 20.66, 20.67, 20.68, 20.69, 20.70, 20.71, 20.72, 20.73, 20.74, 20.75, 20.76, 20.77, 20.78, 20.79, 20.80, 20.81, 20.82, 20.83, 20.84, 20.85, 20.86, 20.87, 20.88, 20.89, 20.90, 20.91, 20.92, 20.93, 20.94, 20.95, 20.96, 20.97, 20.98, 20.99, 20.100, 20.101, 20.102, 20.103, 20.104, 20.105, 20.106, 20.107, 20.108, 20.109, 20.110, 20.111, 20.112, 20.113, 20.114, 20.115, 20.116, 20.117, 20.118, 20.119, 20.120, 20.121, 20.122, 20.123, 20.124, 20.125, 20.126, 20.127, 20.128, 20.129, 20.130, 20.131, 20.132, 20.133, 20.134, 20.135, 20.136; 21.22, 21.23, 21.24, 21.25, 21.26, 21.27, 21.28; 21.29, 21.30, 21.31, 21.32, 21.33, 21.34, 21.35, 21.36, 21.37, 21.38, 21.39, 21.40, 21.41, 21.42, 21.43, 21.44, 21.45, 21.46, 21.47, 21.48, 21.49, 21.50, 21.51, 21.52, 21.53, 21.54, 21.55, 21.56, 21.57, 21.58, 21.59, 21.60, 21.61, 21.62, 21.63, 21.64, 21.65, 21.66, 21.67, 21.68, 21.69, 21.70, 21.71, 21.72, 21.73, 21.74, 21.75, 21.76, 21.77, 21.78, 21.79, 21.80, 21.81, 21.82, 21.83, 21.84, 21.85, 21.86, 21.87, 21.88, 21.89, 21.90, 21.91, 21.92, 21.93, 21.94, 21.95, 21.96, 21.97, 21.98, 21.99, 21.100, 21.101, 21.102, 21.103, 21.104, 21.105, 21.106, 21.107, 21.108, 21.109, 21.110, 21.111, 21.112, 21.113, 21.114, 21.115, 21.116, 21.117, 21.118, 21.119, 21.120, 21.121, 21.122, 21.123, 21.124, 21.125, 21.126, 21.127, 21.128, 21.129, 21.130, 21.131, 21.132, 21.133, 21.134, 21.135, 21.136; 22.23, 22.24, 22.25, 22.26, 22.27, 22.28; 22.29, 22.30, 22.31, 22.32, 22.33, 22.34, 22.35, 22.36, 22.37, 22.38, 22.39, 22.40, 22.41, 22.42, 22.43, 22.44, 22.45, 22.46, 22.47, 22.48, 22.49, 22.50, 22.51, 22.52, 22.53, 22.54, 22.55, 22.56, 22.57, 22.58, 22.59, 22.60, 22.61, 22.62, 22.63, 22.64, 22.65, 22.66, 22.67, 22.68, 22.69, 22.70, 22.71, 22.72, 22.73, 22.74, 22.75, 22.76, 22.77, 22.78, 22.79, 22.80, 22.81, 22.82, 22.83, 22.84, 22.85, 22.86, 22.87, 22.88, 22.89, 22.90, 22.91, 22.92, 22.93, 22.94, 22.95, 22.96, 22.97, 22.98, 22.99, 22.100, 22.101, 22.102, 22.103, 22.104, 22.105, 22.106, 22.107, 22.108, 22.109, 22.110, 22.111, 22.112, 22.113, 22.114, 22.115, 22.116, 22.117, 22.118, 22.119, 22.120, 22.121, 22.122, 22.123, 22.124, 22.125, 22.126, 22.127, 22.128, 22.129, 22.130, 22.131, 22.132, 22.133, 22.134, 22.135, 22.136; 23.24, 23.25, 23.26, 23.27, 23.28; 23.29, 23.30, 23.31, 23.32, 23.33, 23.34, 23.35, 23.36, 23.37, 23.38, 23.39, 23.40, 23.41, 23.42, 23.43, 23.44, 23.45, 23.46, 23.47, 23.48, 23.49, 23.50, 23.51, 23.52, 23.53, 23.54, 23.55, 23.56, 23.57, 23.58, 23.59, 23.60, 23.61, 23.62, 23.63, 23.64, 23.65, 23.66, 23.67, 23.68, 23.69, 23.70, 23.71, 23.72, 23.73, 23.74, 23.75, 23.76, 23.77, 23.78, 23.79, 23.80, 23.81, 23.82, 23.83, 23.84, 23.85, 23.86, 23.87, 23.88, 23.89, 23.90, 23.91, 23.92, 23.93, 23.94, 23.95, 23.96, 23.97, 23.98, 23.99, 23.100, 23.101, 23.102, 23.103, 23.104, 23.105, 23.106, 23.107, 23.108, 23.109, 23.110, 23.111, 23.112, 23.113, 23.114, 23.115, 23.116, 23.117, 23.118, 23.119, 23.120, 23.121, 23.122, 23.123, 23.124, 23.125, 23.126, 23.127, 23.128, 23.129, 23.130, 23.131, 23.132, 23.133, 23.134, 23.135, 23.136; 24.25, 24.26, 24.27, 24.28; 24.29, 24.30, 24.31, 24.32, 24.33, 24.34, 24.35, 24.36, 24.37, 24.38, 24.39, 24.40, 24.41, 24.42, 24.43, 24.44, 24.45, 24.46, 24.47, 24.48, 24.49, 24.50, 24.51, 24.52, 24.53, 24.54, 24.55, 24.56, 24.57, 24.58, 24.59, 24.60, 24.61, 24.62, 24.63, 24.64, 24.65, 24.66, 24.67, 24.68, 24.69, 24.70, 24.71, 24.72, 24.73, 24.74, 24.75, 24.76, 24.77, 24.78, 24.79, 24.80, 24.81, 24.82, 24.83, 24.84, 24.85, 24.86, 24.87, 24.88, 24.89, 24.90, 24.91, 24.92, 24.93, 24.94, 24.95, 24.96, 24.97, 24.98, 24.99, 24.100, 24.101, 24.102, 24.103, 24.104, 24.105, 24.106, 24.107, 24.108, 24.109, 24.110, 24.111, 24.112, 24.113, 24.114, 24.115, 24.116, 24.117, 24.118, 24.119, 24.120, 24.121, 24.122, 24.123, 24.124, 24.125, 24.126, 24.127, 24.128, 24.129, 24.130, 24.131, 24.132, 24.133, 24.134, 24.135, 24.136; 25.26, 25.27, 25.28; 25.29, 25.30, 25.31, 25.32, 25.33, 25.34, 25.35, 25.36, 25.37, 25.38, 25.39, 25.40, 25.41, 25.42, 25.43, 25.44, 25.45, 25.46, 25.47, 25.48, 25.49, 25.50, 25.51, 25.52, 25.53, 25.54, 25.55, 25.56, 25.57, 25.58, 25.59, 25.60, 25.61, 25.62, 25.63, 25.64, 25.65, 25.66, 25.67, 25.68, 25.69, 25.70, 25.71, 25.72, 25.73, 25.74, 25.75, 25.76, 25.77, 25.78, 25.79, 25.80, 25.81, 25.82, 25.83, 25.84, 25.85, 25.86, 25.87, 25.88, 25.89, 25.90, 25.91, 25.92, 25.93, 25.94, 25.95, 25.96, 25.97, 25.98, 25.99, 25.100, 25.101, 25.102, 25.103, 25.104, 25.105, 25.106, 25.107, 25.108, 25.109, 25.110, 25.111, 25.112, 25.113, 25.114, 25.115, 25.116, 25.117, 25.118, 25.119, 25.120, 25.121, 25.122, 25.123, 25.124, 25.125, 26.126, 26.127, 26.128, 26.129, 26.130, 26.131, 26.132, 26.133, 26.134, 26.135, 26.136; 26.27, 26.28; 26.29, 26.30, 26.31, 26.32, 26.33, 26.34, 26.35, 26.36, 26.37, 26.38, 26.39, 26.40, 26.41, 26.42, 26.43, 26.44, 26.45, 26.46, 26.47, 26.48, 26.49, 26.50, 26.51, 26.52, 26.53, 26.54, 26.55, 26.56, 26.57, 26.58, 26.59, 26.60, 26.61, 26.62, 26.63, 26.64, 26.65, 26.66, 26.67, 26.68, 26.69, 26.70, 26.71, 26.72, 26.73, 26.74, 26.75, 26.76, 26.77, 26.78, 26.79, 26.80, 26.81, 26.82, 26.83, 26.84, 26.85, 26.86, 26.87, 26.88, 26.89, 26.90, 26.91, 26.92, 26.93, 26.94, 26.95, 26.96, 26.97, 26.98, 26.99, 26.100, 26.101, 26.102, 26.103, 26.104, 26.105, 26.106, 26.107, 26.108, 26.109, 26.110, 26.111, 26.112, 26.113, 26.114, 26.115, 26.116, 26.117, 26.118, 26.119, 26.120, 26.121, 26.122, 26.123, 26.124, 26.125, 26.126, 26.127, 26.128, 26.129, 26.130, 26.131, 26.132, 26.133, 26.134, 26.135, 26.136; 27.28; 27.29, 27.30, 27.31, 27.32, 27.33, 27.34, 27.35, 27.36, 27.37, 27.38, 27.39, 27.40, 27.41, 27.42, 27.43, 27.44, 27.45, 27.46, 27.47, 27.48, 27.49, 27.50, 27.51, 27.52, 27.53, 27.54, 27.55, 27.56, 27.57, 27.58, 27.59, 27.60, 27.61, 27.62, 27.63, 27.64, 27.65, 27.66, 27.67, 27.68, 27.69, 27.70, 27.71, 27.72, 27.73, 27.74, 27.75, 27.76, 27.77, 27.78, 27.79, 27.80, 27.81, 27.82, 27.83, 27.84, 27.85, 27.86, 27.87, 27.88, 27.89, 27.90, 27.91, 27.92, 27.93, 27.94, 27.95, 27.96, 27.97, 27.98, 27.99, 27.100, 27.101, 27.102, 27.103, 27.104, 27.105, 27.106, 27.107, 27.108, 27.109, 27.110, 27.111, 27.112, 27.113, 27.114, 27.115, 27.116, 27.117, 27.118, 27.119, 27.120, 27.121, 27.122, 27.123, 27.124, 27.125, 27.126, 27.127, 27.128, 27.129, 27.130, 27.131, 27.132, 27.133, 27.134, 27.135, 27.136; 28.29, 28.30, 28.31, 28.32, 28.33, 28.34, 28.35, 28.36, 28.37, 28.38, 28.39, 28.40, 28.41, 28.42, 28.43, 28.44, 28.45, 28.46, 28.47, 28.48, 28.49, 28.50, 28.51, 28.52, 28.53, 28.54, 28.55, 28.56, 28.57, 28.58, 28.59, 28.60, 28.61, 28.62, 28.63, 28.64, 28.65, 28.66, 28.67, 28.68, 28.69, 28.70, 28.71, 28.72, 28.73, 28.74, 28.75, 28.76, 28.77, 28.78, 28.79, 28.80, 28.81, 28.82, 28.83, 28.84, 28.85, 28.86, 28.87, 28.88, 28.89, 28.90, 28.91, 28.92, 28.93, 28.94, 28.95, 28.96, 28.97, 28.98, 28.99, 28.100, 28.101, 28.102, 28.103, 28.104, 28.105, 28.106, 28.107, 28.108, 28.109, 28.110, 28.111, 28.112, 28.113, 28.114, 28.115, 28.116, 28.117, 28.118, 28.119, 28.120, 28.121, 28.122, 28.123, 28.124, 28.125, 28.126, 28.127, 28.128, 28.129, 28.130, 28.131, 28.132, 28.133, 28.134, 28.135, 28.136; 29.30, 29.31, 29.32, 29.33, 29.34, 29.35, 29.36, 29.37, 29.38, 29.39, 29.40, 29.41, 29.42, 29.43, 29.44, 29.45, 29.46, 29.47, 29.48, 29.49, 29.50, 29.51, 29.52, 29.53, 29.54, 29.55, 29.56, 29.57, 29.58, 29.59, 29.60, 29.61, 29.62, 29.63, 29.64, 29.65, 29.66, 29.67, 29.68, 29.69, 29.70, 29.71, 29.72, 29.73, 29.74, 29.75, 29.76, 29.77, 29.78, 29.79, 29.80, 29.81, 29.82, 29.83, 29.84, 29.85, 29.86, 29.87, 29.88, 29.89, 29.90, 29.91, 29.92, 29.93, 29.94, 29.95, 29.96, 29.97, 29.98, 29.99, 29.100, 29.101, 29.102, 29.103, 29.104, 29.105, 29.106, 29.107, 29.108, 29.109, 29.110, 29.111, 29.112, 29.113, 29.114, 29.115, 29.116, 29.117, 29.118, 29.119, 29.120, 29.121, 29.122, 29.123, 29.124, 29.125, 29.126, 29.127, 29.128, 29.129, 29.130, 29.131, 29.132, 29.133, 29.134, 29.135, 29.136; 30.31, 30.32, 30.33, 30.34, 30.35, 30.36, 30.37, 30.38, 30.39, 30.40, 30.41, 30.42, 30.43, 30.44, 30.45, 30.46, 30.47, 30.48, 30.49, 30.50, 30.51, 30.52, 30.53, 30.54, 30.55, 30.56, 30.57, 30.58, 30.59, 30.60, 30.61, 30.62, 30.63, 30.64, 30.65, 30.66, 30.67, 30.68, 30.69, 30.70, 30.71, 30.72, 30.73, 30.74, 30.75, 30.76, 30.77, 30.78, 30.79, 30.80, 30.81, 30.82, 30.83, 30.84, 30.85, 30.86, 30.87, 30.88, 30.89, 30.90, 30.91, 30.92, 30.93, 30.94, 30.95, 30.96, 30.97, 30.98, 30.99, 30.100, 30.101, 30.102, 30.103, 30.104, 30.105, 30.106, 30.107, 30.108, 30.109, 30.110, 30.111, 30.112, 30.113, 30.114, 30.115, 30.116, 30.117, 30.118, 30.119, 30.120, 30.121, 30.122, 30.123, 30.124, 30.125, 30.126, 30.127, 30.128, 30.129, 30.130, 30.131, 30.132, 30.133, 30.134, 30.135, 30.136; 31.32, 31.33, 31.34, 31.35, 31.36, 31.37, 31.38, 31.39, 31.40, 31.41, 31.42, 31.43, 31.44, 31.45, 31.46, 31.47, 31.48, 31.49, 31.50, 31.51, 31.52, 31.53, 31.54, 31.55, 31.56, 31.57, 31.58, 31.59, 31.60, 31.61, 31.62, 31.63, 31.64, 31.65, 31.66, 31.67, 31.68, 31.69, 31.70, 31.71, 31.72, 31.73, 31.74, 31.75, 31.76, 31.77, 31.78, 31.79, 31.80, 31.81, 31.82, 31.83, 31.84, 31.85, 31.86, 31.87, 31.88, 31.89, 31.90, 31.91, 31.92, 31.93, 31.94, 31.95, 31.96, 31.97, 31.98, 31.99, 31.100, 31.101, 31.102, 31.103, 31.104, 31.105, 31.106, 31.107, 31.108, 31.109, 31.110, 31.111, 31.112, 31.113, 31.114, 31.115, 31.116, 31.117, 31.118, 31.119, 31.120, 31.121, 31.122, 31.123, 31.124, 31.125, 31.126, 31.127, 31.128, 31.129, 31.130, 31.131, 31.132, 31.133, 31.134, 31.135, 31.136; 32.33, 32.34, 32.35, 32.36, 32.37, 32.38, 32.39, 32.40, 32.41, 32.42, 32.43, 32.44, 32.45, 32.46, 32.47, 32.48, 32.49, 32.50, 32.51, 32.52, 32.53, 32.54, 32.55, 32.56, 32.57, 32.58, 32.59, 32.60, 32.61, 32.62, 32.63, 32.64, 32.65, 32.66, 32.67, 32.68, 32.69, 32.70, 32.71, 32.72, 32.73, 32.74, 32.75, 32.76, 32.77, 32.78, 32.79, 32.80, 32.81, 32.82, 32.83, 32.84, 32.85, 32.86, 32.87, 32.88, 32.89, 32.90, 32.91, 32.92, 32.93, 32.94, 32.95, 32.96, 32.97, 32.98, 32.99, 32.100, 32.101, 32.102, 32.103, 32.104, 32.105, 32.106, 32.107, 32.108, 32.109, 32.110, 32.111, 32.112, 32.113, 32.114, 32.115, 32.116, 32.117, 32.118, 32.119, 32.120, 32.121, 32.122, 32.123, 32.124, 32.125, 32.126, 32.127, 32.128, 32.129, 32.130, 32.131, 32.132, 32.133, 32.134, 32.135, 32.136; 33.34, 33.35, 33.36, 33.37, 33.38, 33.39, 33.40, 33.41, 33.42, 33.43, 33.44, 33.45, 33.46, 33.47, 33.48, 33.49, 33.50, 33.51, 33.52, 33.53, 33.54, 33.55, 33.56, 33.57, 33.58, 33.59, 33.60, 33.61, 33.62, 33.63, 33.64, 33.65, 33.66, 33.67, 33.68, 33.69, 33.70, 33.71, 33.72, 33.73, 33.74, 33.75, 33.76, 33.77, 33.78, 33.79, 33.80, 33.81, 33.82, 33.83, 33.84, 33.85, 33.86, 33.87, 33.88, 33.89, 33.90, 33.91, 33.92, 33.93, 33.94, 33.95, 33.96, 33.97, 33.98, 33.99, 33.100, 33.101, 33.102, 33.103, 33.104, 33.105, 33.106, 33.107, 33.108, 33.109, 33.110, 33.111, 33.112, 33.113, 33.114, 33.115, 33.116, 33.117, 33.118, 33.119, 33.120, 33.121, 33.122, 33.123, 33.124, 33.125, 33.126, 33.127, 33.128, 33.129, 33.130, 33.131, 33.132, 33.133, 33.134, 33.135, 33.136; 34.35, 34.36, 34.37, 34.38, 34.39, 34.40, 34.41, 34.42, 34.43, 34.44, 34.45, 34.46, 34.47, 34.48, 34.49, 34.50, 34.51, 34.52, 34.53, 34.54, 34.55, 34.56, 34.57, 34.58, 34.59, 34.60, 34.61, 34.62, 34.63, 34.64, 34.65, 34.66, 34.67, 34.68, 34.69, 34.70, 34.71, 34.72, 34.73, 34.74, 34.75, 34.76, 34.77, 34.78, 34.79, 34.80, 34.81, 34.82, 34.83, 34.84, 34.85, 34.86, 34.87, 34.88, 34.89, 34.90, 34.91, 34.92, 34.93, 34.94, 34.95, 34.96, 34.97, 34.98, 34.99, 34.100, 34.101, 34.102, 34.103, 34.104, 34.105, 34.106, 34.107, 34.108, 34.109, 34.110, 34.111, 34.112, 34.113, 34.114, 34.115, 34.116, 34.117, 34.118, 34.119, 34.120, 34.121, 34.122, 34.123, 34.124, 34.125, 34.126, 34.127, 34.128, 34.129, 34.130, 34.131, 34.132, 34.133, 34.134, 34.135, 34.136; 35.36, 35.37, 35.38, 35.39, 35.40, 35.41, 35.42, 35.43, 35.44, 35.45, 35.46, 35.47, 35.48, 35.49, 35.50, 35.51, 35.52, 35.53, 35.54, 35.55, 35.56, 35.57, 35.58, 35.59, 35.60, 35.61, 35.62, 35.63, 35.64, 35.65, 35.66, 35.67, 35.68, 35.69, 35.70, 35.71, 35.72, 35.73, 35.74, 35.75, 35.76, 35.77, 35.78, 35.79, 35.80, 35.81, 35.82, 35.83, 35.84, 35.85, 35.86, 35.87, 35.88, 35.89, 35.90, 35.91, 35.92, 35.93, 35.94, 35.95, 35.96, 35.97, 35.98, 35.99, 35.100, 35.101, 35.102, 35.103, 35.104, 35.105, 35.106, 35.107, 35.108, 35.109, 35.110, 35.111, 35.112, 35.113, 35.114, 35.115, 35.116, 35.117, 35.118, 35.119, 35.120, 35.121, 35.122, 35.123, 35.124, 35.125, 35.126, 35.127, 35.128, 35.129, 35.130, 35.131, 35.132, 35.133, 35.134, 35.135, 35.136; 36.37, 36.38, 36.39, 36.40, 36.41, 36.42, 36.43, 36.44, 36.45, 36.46, 36.47, 36.48, 36.49, 36.50, 36.51, 36.52, 36.53, 36.54, 36.55, 36.56, 36.57, 36.58, 36.59, 36.60, 36.61, 36.62, 36.63, 36.64, 36.65, 36.66, 36.67, 36.68, 36.69, 36.70, 36.71, 36.72, 36.73, 36.74, 36.75, 36.76, 36.77, 36.78, 36.79, 36.80, 36.81, 36.82, 36.83, 36.84, 36.85, 36.86, 36.87, 36.88, 36.89, 36.90, 36.91, 36.92, 36.93, 36.94, 36.95, 36.96, 36.97, 36.98, 36.99, 36.100, 36.101, 36.102, 36.103, 36.104, 36.105, 36.106, 36.107, 36.108, 36.109, 36.110, 36.111, 36.112, 36.113, 36.114, 36.115, 36.116, 36.117, 36.118, 36.119, 36.120, 36.121, 36.122, 36.123, 36.124, 36.125, 36.126, 36.127, 36.128, 36.129, 36.130, 36.131, 36.132, 36.133, 36.134, 36.135, 36.136; 37.38, 37.39, 37.40, 37.41, 37.42, 37.43, 37.44, 37.45, 37.46, 37.47, 37.48, 37.49, 37.50, 37.51, 37.52, 37.53, 37.54, 37.55, 37.56, 37.57, 37.58, 37.59, 37.60, 37.61, 37.62, 37.63, 37.64, 37.65, 37.66, 37.67, 37.68, 37.69, 37.70, 37.71, 37.72, 37.73, 37.74, 37.75, 37.76, 37.77, 37.78, 37.79, 37.80, 37.81, 37.82, 37.83, 37.84, 37.85, 37.86, 37.87, 37.88, 37.89, 37.90, 37.91, 37.92, 37.93, 37.94, 37.95, 37.96, 37.97, 37.98, 37.99, 37.100, 37.101, 37.102, 37.103, 37.104, 37.105, 37.106, 37.107, 37.108, 37.109, 37.110, 37.111, 37.112, 37.113, 37.114, 37.115, 37.116, 37.117, 37.118, 37.119, 37.120, 37.121, 37.122, 37.123, 37.124, 37.125, 37.126, 37.127, 37.128, 37.129, 37.130, 37.131, 37.132, 37.133, 37.134, 37.135, 37.136; 38.39, 38.40, 38.41, 38.42, 38.43, 38.44, 38.45, 38.46, 38.47, 38.48, 38.49, 38.50, 38.51, 38.52, 38.53, 38.54, 38.55, 38.56, 38.57, 38.58, 38.59, 38.60, 38.61, 38.62, 38.63, 38.64, 38.65, 38.66, 38.67, 38.68, 38.69, 38.70, 38.71, 38.72, 38.73, 38.74, 38.75, 38.76, 38.77, 38.78, 38.79, 38.80, 38.81, 38.82, 38.83, 38.84, 38.85, 38.86, 38.87, 38.88, 38.89, 38.90, 38.91, 38.92, 38.93, 38.94, 38.95, 38.96, 38.97, 38.98, 38.99, 38.100, 38.101, 38.102, 38.103, 38.104, 38.105, 38.106, 38.107, 38.108, 38.109, 38.110, 38.111, 38.112, 38.113, 38.114, 38.115, 38.116, 38.117, 38.118, 38.119, 38.120, 38.121, 38.122, 38.123, 38.124, 38.125, 38.126, 38.127, 38.128, 38.129, 38.130, 38.131, 38.132, 38.133, 38.134, 38.135, 38.136; 39.40, 39.41, 39.42, 39.43, 39.44, 39.45, 39.46, 39.47, 39.48, 39.49, 39.50, 39.51, 39.52, 39.53, 39.54, 39.55, 39.56, 39.57, 39.58, 39.59, 39.60, 39.61, 39.62, 39.63, 39.64, 39.65, 39.66, 39.67, 39.68, 39.69, 39.70, 39.71, 39.72, 39.73, 39.74, 39.75, 39.76, 39.77, 39.78, 39.79, 39.80, 39.81, 39.82, 39.83, 39.84, 39.85, 39.86, 39.87, 39.88, 39.89, 39.90, 39.91, 39.92, 39.93, 39.94, 39.95, 39.96, 39.97, 39.98, 39.99, 39.100, 39.101, 39.102, 39.103, 39.104, 39.105, 39.106, 39.107, 39.108, 39.109, 39.110, 39.111, 39.112, 39.113, 39.114, 39.115, 39.116, 39.117, 39.118, 39.119, 39.120, 39.121, 39.122, 39.123, 39.124, 39.125, 39.126, 39.127, 39.128, 39.129, 39.130, 39.131, 39.132, 39.133, 39.134, 39.135, 39.136; 40.41, 40.42, 40.43, 40.44, 40.45, 40.46, 40.47, 40.48, 40.49, 40.50, 40.51, 40.52, 40.53, 40.54, 40.55, 40.56, 40.57, 40.58, 40.59, 40.60, 40.61, 40.62, 40.63, 40.64, 40.65, 40.66, 40.67, 40.68, 40.69, 40.70, 40.71, 40.72, 40.73, 40.74, 40.75, 40.76, 40.77, 40.78, 40.79, 40.80, 40.81, 40.82, 40.83, 40.84, 40.85, 40.86, 40.87, 40.88, 40.89, 40.90, 40.91, 40.92, 40.93, 40.94, 40.95, 40.96, 40.97, 40.98, 40.99, 40.100, 40.101, 40.102, 40.103, 40.104, 40.105, 40.106, 40.107, 40.108, 40.109, 40.110, 40.111, 40.112, 40.113, 40.114, 40.115, 40.116, 40.117, 40.118, 40.119, 40.120, 40.121, 40.122, 40.123, 40.124, 40.125, 40.126, 40.127, 40.128, 40.129, 40.130, 40.131, 40.132, 40.133, 40.134, 40.135, 40.136; 41.42, 41.43, 41.44, 41.45, 41.46, 41.47, 41.48, 41.49, 41.50, 41.51, 41.52, 41.53, 41.54, 41.55, 41.56, 41.57, 41.58, 41.59, 41.60, 41.61, 41.62, 41.63, 41.64, 41.65, 41.66, 41.67, 41.68, 41.69, 41.70, 41.71, 41.72, 41.73, 41.74, 41.75, 41.76, 41.77, 41.78, 41.79, 41.80, 41.81, 41.82, 41.83, 41.84, 41.85, 41.86, 41.87, 41.88, 41.89, 41.90, 41.91, 41.92, 41.93, 41.94, 41.95, 41.96, 41.97, 41.98, 41.99, 41.100, 41.101, 41.102, 41.103, 41.104, 41.105, 41.106, 41.107, 41.108, 41.109, 41.110, 41.111, 41.112, 41.113, 41.114, 41.115, 41.116, 41.117, 41.118, 41.119, 41.120, 41.121, 41.122, 41.123, 41.124, 41.125, 41.126, 41.127, 41.128, 41.129, 41.130, 41.131, 41.132, 41.133, 41.134, 41.135, 41.136; 42.43, 42.44, 42.45, 42.46, 42.47, 42.48, 42.49, 42.50, 42.51, 42.52, 42.53, 42.54, 42.55, 42.56, 42.57, 42.58, 42.59, 42.60, 42.61, 42.62, 42.63, 42.64, 42.65, 42.66, 42.67, 42.68, 42.69, 42.70, 42.71, 42.72, 42.73, 42.74, 42.75, 42.76, 42.77, 42.78, 42.79, 42.80, 42.81, 42.82, 42.83, 42.84, 42.85, 42.86, 42.87, 42.88, 42.89, 42.90, 42.91, 42.92, 42.93, 42.94, 42.95, 42.96, 42.97, 42.98, 42.99, 42.100, 42.101, 42.102, 42.103, 42.104, 42.105, 42.106, 42.107, 42.108, 42.109, 42.110, 42.111, 42.112, 42.113, 42.114, 42.115, 42.116, 42.117, 42.118, 42.119, 42.120, 42.121, 42.122, 42.123, 42.124, 42.125, 42.126, 42.127, 42.128, 42.129, 42.130, 42.131, 42.132, 42.133, 42.134, 42.135, 42.136; 43.44, 43.45, 43.46, 43.47, 43.48, 43.49, 43.50, 43.51, 43.52, 43.53, 43.54, 43.55, 43.56, 43.57, 43.58, 43.59, 43.60, 43.61, 43.62, 43.63, 43.64, 43.65, 43.66, 43.67, 43.68, 43.69, 43.70, 43.71, 43.72, 43.73, 43.74, 43.75, 43.76, 43.77, 43.78, 43.79, 43.80, 43.81, 43.82, 43.83, 43.84, 43.85, 43.86, 43.87, 43.88, 43.89, 43.90, 43.91, 43.92, 43.93, 43.94, 43.95, 43.96, 43.97, 43.98, 43.99, 43.100, 43.101, 43.102, 43.103, 43.104, 43.105, 43.106, 43.107, 43.108, 43.109, 43.110, 43.111, 43.112, 43.113, 43.114, 43.115, 43.116, 43.117, 43.118, 43.119, 43.120, 43.121, 43.122, 43.123, 43.124, 43.125, 43.126, 43.127, 43.128, 43.129, 43.130, 43.131, 43.132, 43.133, 43.134, 43.135, 43.136; 44.45, 44.46, 44.47, 44.48, 44.49, 44.50, 44.51, 44.52, 44.53, 44.54, 44.55, 44.56, 44.57, 44.58, 44.59, 44.60, 44.61, 44.62, 44.63, 44.64, 44.65, 44.66, 44.67, 44.68, 44.69, 44.70, 44.71, 44.72, 44.73, 44.74, 44.75, 44.76, 44.77, 44.78, 44.79, 44.80, 44.81, 44.82, 44.83, 44.84, 44.85, 44.86, 44.87, 44.88, 44.89, 44.90, 44.91, 44.92, 44.93, 44.94, 44.95, 44.96, 44.97, 44.98, 44.99, 44.100, 44.101, 44.102, 44.103, 44.104, 44.105, 44.106, 44.107, 44.108, 44.109, 44.110, 44.111, 44.112, 44.113, 44.114, 44.115, 44.116, 44.117, 44.118, 44.119, 44.120, 44.121, 44.122, 44.123, 44.124, 44.125, 44.126, 44.127, 44.128, 44.129, 44.130, 44.131, 44.132, 44.133, 44.134, 44.135, 44.136; 45.46, 45.47, 45.48, 45.49, 45.50, 45.51, 45.52, 45.53, 45.54, 45.55, 45.56, 45.57, 45.58, 45.59, 45.60, 45.61, 45.62, 45.63, 45.64, 45.65, 45.66, 45.67, 45.68, 45.69, 45.70, 45.71, 45.72, 45.73, 45.74, 45.75, 45.76, 45.77, 45.78, 45.79, 45.80, 45.81, 45.82, 45.83, 45.84, 45.85, 45.86, 45.87, 45.88, 45.89, 45.90, 45.91, 45.92, 45.93, 45.94, 45.95, 45.96, 45.97, 45.98, 45.99, 45.100, 45.101, 45.102, 45.103, 45.104, 45.105, 45.106, 45.107, 45.108, 45.109, 45.110, 45.111, 45.112, 45.113, 45.114, 45.115, 45.116, 45.117, 45.118, 45.119, 45.120, 45.121, 45.122, 45.123, 45.124, 45.125, 45.126, 45.127, 45.128, 45.129, 45.130, 45.131, 45.132, 45.133, 45.134, 45.135, 45.136; 46.47, 46.48, 46.49, 46.50, 46.51, 46.52, 46.53, 46.54, 46.55, 46.56, 46.57, 46.58, 46.59, 46.60, 46.61, 46.62, 46.63, 46.64, 46.65, 46.66, 46.67, 46.68, 46.69, 46.70, 46.71, 46.72, 46.73, 46.74, 46.75, 46.76, 46.77, 46.78, 46.79, 46.80, 46.81, 46.82, 46.83, 46.84, 46.85, 46.86, 46.87, 46.88, 46.89, 46.90, 46.91, 46.92, 46.93, 46.94, 46.95, 46.96, 46.97, 46.98, 46.99, 46.100, 46.101, 46.102, 46.103, 46.104, 46.105, 46.106, 46.107, 46.108, 46.109, 46.110, 46.111, 46.112, 46.113, 46.114, 46.115, 46.116, 46.117, 46.118, 46.119, 46.120, 46.121, 46.122, 46.123, 46.124, 46.125, 46.126, 46.127, 46.128, 46.129, 46.130, 46.131, 46.132, 46.133, 46.134, 46.135, 46.136; 47.48, 47.49, 47.50, 47.51, 47.52, 47.53, 47.54, 47.55, 47.56, 47.57, 47.58, 47.59, 47.60, 47.61, 47.62, 47.63, 47.64, 47.65, 47.66, 47.67, 47.68, 47.69, 47.70, 47.71, 47.72, 47.73, 47.74, 47.75, 47.76, 47.77, 47.78, 47.79, 47.80, 47.81, 47.82, 47.83, 47.84, 47.85, 47.86, 47.87, 47.88, 47.89, 47.90, 47.91, 47.92, 47.93, 47.94, 47.95, 47.96, 47.97, 47.98, 47.99, 47.100, 47.101, 47.102, 47.103, 47.104, 47.105, 47.106, 47.107, 47.108, 47.109, 47.110, 47.111, 47.112, 47.113, 47.114, 47.115, 47.116, 47.117, 47.118, 47.119, 47.120, 47.121, 47.122, 47.123, 47.124, 47.125, 47.126, 47.127, 47.128, 47.129, 47.130, 47.131, 47.132, 47.133, 47.134, 47.135, 47.136; 48.49, 48.50, 48.51, 48.52, 48.53, 48.54, 48.55, 48.56, 48.57, 48.58, 48.59, 48.60, 48.61, 48.62, 48.63, 48.64, 48.65, 48.66, 48.67, 48.68, 48.69, 48.70, 48.71, 48.72, 48.73, 48.74, 48.75, 48.76, 48.77, 48.78, 48.79, 48.80, 48.81, 48.82, 48.83, 48.84, 48.85, 48.86, 48.87, 48.88, 48.89, 48.90, 48.91, 48.92, 48.93, 48.94, 48.95, 48.96, 48.97, 48.98, 48.99, 48.100, 48.101, 48.102, 48.103, 48.104, 48.105, 48.106, 48.107, 48.108, 48.109, 48.110, 48.111, 48.112, 48.113, 48.114, 48.115, 48.116, 48.117, 48.118, 48.119, 48.120, 48.121, 48.122, 48.123, 48.124, 48.125, 48.126, 48.127, 48.128, 48.129, 48.130, 48.131, 48.132, 48.133, 48.134, 48.135, 48.136; 49.50, 49.51, 49.52, 49.53, 49.54, 49.55, 49.56, 49.57, 49.58, 49.59, 49.60, 49.61, 49.62, 49.63, 49.64, 49.65, 49.66, 49.67, 49.68, 49.69, 49.70, 49.71, 49.72, 49.73, 49.74, 49.75, 49.76, 49.77, 49.78, 49.79, 49.80, 49.81, 49.82, 49.83, 49.84, 49.85, 49.86, 49.87, 49.88, 49.89, 49.90, 49.91, 49.92, 49.93, 49.94, 49.95, 49.96, 49.97, 49.98, 49.99, 49.100, 49.101, 49.102, 49.103, 49.104, 49.105, 49.106, 49.107, 49.108, 49.109, 49.110, 49.111, 49.112, 49.113, 49.114, 49.115, 49.116, 49.117, 49.118, 49.119, 49.120, 49.121, 49.122, 49.123, 49.124, 49.125, 49.126, 49.127, 49.128, 49.129, 49.130, 49.131, 49.132, 49.133, 49.134, 49.135, 49.136; 50.51, 50.52, 50.53, 50.54, 50.55, 50.56, 50.57, 50.58, 50.59, 50.60, 50.61, 50.62, 50.63, 50.64, 50.65, 50.66, 50.67, 50.68, 50.69, 50.70, 50.71, 50.72, 50.73, 50.74, 50.75, 50.76, 50.77, 50.78, 50.79, 50.80, 50.81, 50.82, 50.83, 50.84, 50.85, 50.86, 50.87, 50.88, 50.89, 50.90, 50.91, 50.92, 50.93, 50.94, 50.95, 50.96, 50.97, 50.98, 50.99, 50.100, 50.101, 50.102, 50.103, 50.104, 50.105, 50.106, 50.107, 50.108, 50.109, 50.110, 50.111, 50.112, 50.113, 50.114, 50.115, 50.116, 50.117, 50.118, 50.119, 50.120, 50.121, 50.122, 50.123, 50.124, 50.125, 50.126, 50.127, 50.128, 50.129, 50.130, 50.131, 50.132, 50.133, 50.134, 50.135, 50.136; 51.52, 51.53, 51.54, 51.55, 51.56, 51.57, 51.58, 51.59, 51.60, 51.61, 51.62, 51.63, 51.64, 51.65, 51.66, 51.67, 51.68, 51.69, 51.70, 51.71, 51.72, 51.73, 51.74, 51.75, 51.76, 51.77, 51.78, 51.79, 51.80, 51.81, 51.82, 51.83, 51.84, 51.85, 51.86, 51.87, 51.88, 51.89, 51.90, 51.91, 51.92, 51.93, 51.94, 51.95, 51.96, 51.97, 51.98, 51.99, 51.100, 51.101, 51.102, 51.103, 51.104, 51.105, 51.106, 51.107, 51.108, 51.109, 51.110, 51.111, 51.112, 51.113, 51.114, 51.115, 51.116, 51.117, 51.118, 51.119, 51.120, 51.121, 51.122, 51.123, 51.124, 51.125, 51.126, 51.127, 51.128, 51.129, 51.130, 51.131, 51.132, 51.133, 51.134, 51.135, 51.136; 52.53, 52.54, 52.55, 52.56, 52.57, 52.58, 52.59, 52.60, 52.61, 52.62, 52.63, 52.64, 52.65, 52.66, 52.67, 52.68, 52.69, 52.70, 52.71, 52.72, 52.73, 52.74, 52.75, 52.76, 52.77, 52.78, 52.79, 52.80, 52.81, 52.82, 52.83, 52.84, 52.85, 52.86, 52.87, 52.88, 52.89, 52.90, 52.91, 52.92, 52.93, 52.94, 52.95, 52.96, 52.97, 52.98, 52.99, 52.100, 52.101, 52.102, 52.103, 52.104, 52.105, 52.106, 52.107, 52.108, 52.109, 52.110, 52.111, 52.112, 52.113, 52.114, 52.115, 52.116, 52.117, 52.118, 52.119, 52.120, 52.121, 52.122, 52.123, 52.124, 52.125, 52.126, 52.127, 52.128, 52.129, 52.130, 52.131, 52.132, 52.133, 52.134, 52.135, 52.136; 53.54, 53.55, 53.56, 53.57, 53.58, 53.59, 53.60, 53.61, 53.62, 53.63, 53.64, 53.65, 53.66, 53.67, 53.68, 53.69, 53.70, 53.71, 53.72, 53.73, 53.74, 53.75, 53.76, 53.77, 53.78, 53.79, 53.80, 53.81, 53.82, 53.83, 53.84, 53.85, 53.86, 53.87, 53.88, 53.89, 53.90, 53.91, 53.92, 53.93, 53.94, 53.95, 53.96, 53.97, 53.98, 53.99, 53.100, 53.101, 53.102, 53.103, 53.104, 53.105, 53.106, 53.107, 53.108, 53.109, 53.110, 53.111, 53.112, 53.113, 53.114, 53.115, 53.116, 53.117, 53.118, 53.119, 53.120, 53.121, 53.122, 53.123, 53.124, 53.125, 53.126, 53.127, 53.128, 53.129, 53.130, 53.131, 53.132, 53.133, 53.134, 53.135, 53.136; 54.55, 54.56, 54.57, 54.58, 54.59, 54.60, 54.61, 54.62, 54.63, 54.64, 54.65, 54.66, 54.67, 54.68, 54.69, 54.70, 54.71, 54.72, 54.73, 54.74, 54.75, 54.76, 54.77, 54.78, 54.79, 54.80, 54.81, 54.82, 54.83, 54.84, 54.85, 54.86, 54.87, 54.88, 54.89, 54.90, 54.91, 54.92, 54.93, 54.94, 54.95, 54.96, 54.97, 54.98, 54.99, 54.100, 54.101, 54.102, 54.103, 54.104, 54.105, 54.106, 54.107, 54.108, 54.109, 54.110, 54.111, 54.112, 54.113, 54.114, 54.115, 54.116, 54.117, 54.118, 54.119, 54.120, 54.121, 54.122, 54.123, 54.124, 54.125, 54.126, 54.127, 54.128, 54.129, 54.130, 54.131, 54.132, 54.133, 54.134, 54.135, 54.136; 55.56, 55.57, 55.58, 55.59, 55.60, 55.61, 55.62, 55.63, 55.64, 55.65, 55.66, 55.67, 55.68, 55.69, 55.70, 55.71, 55.72, 55.73, 55.74, 55.75, 55.76, 55.77, 55.78, 55.79, 55.80, 55.81, 55.82, 55.83, 55.84, 55.85, 55.86, 55.87, 55.88, 55.89, 55.90, 55.91, 55.92, 55.93, 55.94, 55.95, 55.96, 55.97, 55.98, 55.99, 55.100, 55.101, 55.102, 55.103, 55.104, 55.105, 55.106, 55.107, 55.108, 55.109, 55.110, 55.111, 55.112, 55.113, 55.114, 55.115, 55.116, 55.117, 55.118, 55.119, 55.120, 55.121, 55.122, 55.123, 55.124, 55.125, 55.126, 55.127, 55.128, 55.129, 55.130, 55.131, 55.132, 55.133, 55.134, 55.135, 55.136; 56.57, 56.58, 56.59, 56.60, 56.61, 56.62, 56.63, 56.64, 56.65, 56.66, 56.67, 56.68, 56.69, 56.70, 56.71, 56.72, 56.73, 56.74, 56.75, 56.76, 56.77, 56.78, 56.79, 56.80, 56.81, 56.82, 56.83, 56.84, 56.85, 56.86, 56.87, 56.88, 56.89, 56.90, 56.91, 56.92, 56.93, 56.94, 56.95, 56.96, 56.97, 56.98, 56.99, 56.100, 56.101, 56.102, 56.103, 56.104, 56.105, 56.106, 56.107, 56.108, 56.109, 56.110, 56.111, 56.112, 56.113, 56.114, 56.115, 56.116, 56.117, 56.118, 56.119, 56.120, 56.121, 56.122, 56.123, 56.124, 56.125, 56.126, 56.127, 56.128, 56.129, 56.130, 56.131, 56.132, 56.133, 56.134, 56.135, 56.136; 57.58, 57.59, 57.60, 57.61, 57.62, 57.63, 57.64, 57.65, 57.66, 57.67, 57.68, 57.69, 57.70, 57.71, 57.72, 57.73, 57.74, 57.75, 57.76, 57.77, 57.78, 57.79, 57.80, 57.81, 57.82, 57.83, 57.84, 57.85, 57.86, 57.87, 57.88, 57.89, 57.90, 57.91, 57.92, 57.93, 57.94, 57.95, 57.96, 57.97, 57.98, 57.99, 57.100, 57.101, 57.102, 57.103, 57.104, 57.105, 57.106, 57.107, 57.108, 57.109, 57.110, 57.111, 57.112, 57.113, 57.114, 57.115, 57.116, 57.117, 57.118, 57.119, 57.120, 57.121, 57.122, 57.123, 57.124, 57.125, 57.126, 57.127, 57.128, 57.129, 57.130, 57.131, 57.132, 57.133, 57.134, 57.135, 57.136; 58.59, 58.60, 58.61, 58.62, 58.63, 58.64, 58.65, 58.66, 58.67, 58.68, 58.69, 58.70, 58.71, 58.72, 58.73, 58.74, 58.75, 58.76, 58.77, 58.78, 58.79, 58.80, 58.81, 58.82, 58.83, 58.84, 58.85, 58.86, 58.87, 58.88, 58.89, 58.90, 58.91, 58.92, 58.93, 58.94, 58.95, 58.96, 58.97, 58.98, 58.99, 58.100, 58.101, 58.102, 58.103, 58.104, 58.105, 58.106, 58.107, 58.108, 58.109, 58.110, 58.111, 58.112, 58.113, 58.114, 58.115, 58.116, 58.117, 58.118, 58.119, 58.120, 58.121, 58.122, 58.123, 58.124, 58.125, 58.126, 58.127, 58.128, 58.129, 58.130, 58.131, 58.132, 58.133, 58.134, 58.135, 58.136; 59.60, 59.61, 59.62, 59.63, 59.64, 59.65, 59.66, 59.67, 59.68, 59.69, 59.70, 59.71, 59.72, 59.73, 59.74, 59.75, 59.76, 59.77, 59.78, 59.79, 59.80, 59.81, 59.82, 59.83, 59.84, 59.85, 59.86, 59.87, 59.88, 59.89, 59.90, 59.91, 59.92, 59.93, 59.94, 59.95, 59.96, 59.97, 59.98, 59.99, 59.100, 59.101, 59.102, 59.103, 59.104, 59.105, 59.106, 59.107, 59.108, 59.109, 59.110, 59.111, 59.112, 59.113, 59.114, 59.115, 59.116, 59.117, 59.118, 59.119, 59.120, 59.121, 59.122, 59.123, 59.124, 59.125, 59.126, 59.127, 59.128, 59.129, 59.130, 59.131, 59.132, 59.133, 59.134, 59.135, 59.136; 60.61, 60.62, 60.63, 60.64, 60.65, 60.66, 60.67, 60.68, 60.69, 60.70, 60.71, 60.72, 60.73, 60.74, 60.75, 60.76, 60.77, 60.78, 60.79, 60.80, 60.81, 60.82, 60.83, 60.84, 60.85, 60.86, 60.87, 60.88, 60.89, 60.90, 60.91, 60.92, 60.93, 60.94, 60.95, 60.96, 60.97, 60.98, 60.99, 60.100, 60.101, 60.102, 60.103, 60.104, 60.105, 60.106, 60.107, 60.108, 60.109, 60.110, 60.111, 60.112, 60.113, 60.114, 60.115, 60.116, 60.117, 60.118, 60.119, 60.120, 60.121, 60.122, 60.123, 60.124, 60.125, 60.126, 60.127, 60.128, 60.129, 60.130, 60.131, 60.132, 60.133, 60.134, 60.135, 60.136; 61.62, 61.63, 61.64, 61.65, 61.66, 61.67, 61.68, 61.69, 61.70, 61.71, 61.72, 61.73, 61.74, 61.75, 61.76, 61.77, 61.78, 61.79, 61.80, 61.81, 61.82, 61.83, 61.84, 61.85, 61.86, 61.87, 61.88, 61.89, 61.90, 61.91, 61.92, 61.93, 61.94, 61.95, 61.96, 61.97, 61.98, 61.99, 61.100, 61.101, 61.102, 61.103, 61.104, 61.105, 61.106, 61.107, 61.108, 61.109, 61.110, 61.111, 61.112, 61.113, 61.114, 61.115, 61.116, 61.117, 61.118, 61.119, 61.120, 61.121, 61.122, 61.123, 61.124, 61.125, 61.126, 61.127, 61.128, 61.129, 61.130, 61.131, 61.132, 61.133, 61.134, 61.135, 61.136; 62.63, 62.64, 62.65, 62.66, 62.67, 62.68, 62.69, 62.70, 62.71, 62.72, 62.73, 62.74, 62.75, 62.76, 62.77, 62.78, 62.79, 62.80, 62.81, 62.82, 62.83, 62.84, 62.85, 62.86, 62.87, 62.88, 62.89, 62.90, 62.91, 62.92, 62.93, 62.94, 62.95, 62.96, 62.97, 62.98, 62.99, 62.100, 62.101, 62.102, 62.103, 62.104, 62.105, 62.106, 62.107, 62.108, 62.109, 62.110, 62.111, 62.112, 62.113, 62.114, 62.115, 62.116, 62.117, 62.118, 62.119, 62.120, 62.121, 62.122, 62.123, 62.124, 62.125, 62.126, 62.127, 62.128, 62.129, 62.130, 62.131, 62.132, 62.133, 62.134, 62.135, 62.136; 63.64, 63.65, 63.66, 63.67, 63.68, 63.69, 63.70, 63.71, 63.72, 63.73, 63.74, 63.75, 63.76, 63.77, 63.78, 63.79, 63.80, 63.81, 63.82, 63.83, 63.84, 63.85, 63.86, 63.87, 63.88, 63.89, 63.90, 63.91, 63.92, 63.93, 63.94, 63.95, 63.96, 63.97, 63.98, 63.99, 63.100, 63.101, 63.102, 63.103, 63.104, 63.105, 63.106, 63.107, 63.108, 63.109, 63.110, 63.111, 63.112, 63.113, 63.114, 63.115, 63.116, 63.117, 63.118, 63.119, 63.120, 63.121, 63.122, 63.123, 63.124, 63.125, 63.126, 63.127, 63.128, 63.129, 63.130, 63.131, 63.132, 63.133, 63.134, 63.135, 63.136; 64.65, 64.66, 64.67, 64.68, 64.69, 64.70, 64.71, 64.72, 64.73, 64.74, 64.75, 64.76, 64.77, 64.78, 64.79, 64.80, 64.81, 64.82, 64.83, 64.84, 64.85, 64.86, 64.87, 64.88, 64.89, 64.90, 64.91, 64.92, 64.93, 64.94, 64.95, 64.96, 64.97, 64.98, 64.99, 64.100, 64.101, 64.102, 64.103, 64.104, 64.105, 64.106, 64.107, 64.108, 64.109, 64.110, 64.111, 64.112, 64.113, 64.114, 64.115, 64.116, 64.117, 64.118, 64.119, 64.120, 64.121, 64.122, 64.123, 64.124, 64.125, 64.126, 64.127, 64.128, 64.129, 64.130, 64.131, 64.132, 64.133, 64.134, 64.135, 64.136; 65.66, 65.67, 65.68, 65.69, 65.70, 65.71, 65.72, 65.73, 65.74, 65.75, 65.76, 65.77, 65.78, 65.79, 65.80, 65.81, 65.82, 65.83, 65.84, 65.85, 65.86, 65.87, 65.88, 65.89, 65.90, 65.91, 65.92, 65.93, 65.94, 65.95, 65.96, 65.97, 65.98, 65.99, 65.100, 65.101, 65.102, 65.103, 65.104, 65.105, 65.106, 65.107, 65.108, 65.109, 65.110, 65.111, 65.112, 65.113, 65.114, 65.115, 65.116, 65.117, 65.118, 65.119, 65.120, 65.121, 65.122, 65.123, 65.124, 65.125, 65.126, 65.127, 65.128, 65.129, 65.130, 65.131, 65.132, 65.133, 65.134, 65.135, 65.136; 66.67, 66.68, 66.69, 66.70, 66.71, 66.72, 66.73, 66.74, 66.75, 66.76, 66.77, 66.78, 66.79, 66.80, 66.81, 66.82, 66.83, 66.84, 66.85, 66.86, 66.87, 66.88, 66.89, 66.90, 66.91, 66.92, 66.93, 66.94, 66.95, 66.96, 66.97, 66.98, 66.99, 66.100, 66.101, 66.102, 66.103, 66.104, 66.105, 66.106, 66.107, 66.108, 66.109, 66.110, 66.111, 66.112, 66.113, 66.114, 66.115, 66.116, 66.117, 66.118, 66.119, 66.120, 66.121, 66.122, 66.123, 66.124, 66.125, 66.126, 66.127, 66.128, 66.129, 66.130, 66.131, 66.132, 66.133, 66.134, 66.135, 66.136; 67.68, 67.69, 67.70, 67.71, 67.72, 67.73, 67.74, 67.75, 67.76, 67.77, 67.78, 67.79, 67.80, 67.81, 67.82, 67.83, 67.84, 67.85, 67.86, 67.87, 67.88, 67.89, 67.90, 67.91, 67.92, 67.93, 67.94, 67.95, 67.96, 67.97, 67.98, 67.99, 67.100, 67.101, 67.102, 67.103, 67.104, 67.105, 67.106, 67.107, 67.108, 67.109, 67.110, 67.111, 67.112, 67.113, 67.114, 67.115, 67.116, 67.117, 67.118, 67.119, 67.120, 67.121, 67.122, 67.123, 67.124, 67.125, 67.126, 67.127, 67.128, 67.129, 67.130, 67.131, 67.132, 67.133, 67.134, 67.135, 67.136; 68.69, 68.70, 68.71, 68.72, 68.73, 68.74, 68.75, 68.76, 68.77, 68.78, 68.79, 68.80, 68.81, 68.82, 68.83, 68.84, 68.85, 68.86, 68.87, 68.88, 68.89, 68.90, 68.91, 68.92, 68.93, 68.94, 68.95, 68.96, 68.97, 68.98, 68.99, 68.100, 68.101, 68.102, 68.103, 68.104, 68.105, 68.106, 68.107, 68.108, 68.109, 68.110, 68.111, 68.112, 68.113, 68.114, 68.115, 68.116, 68.117, 68.118, 68.119, 68.120, 68.121, 68.122, 68.123, 68.124, 68.125, 68.126, 68.127, 68.128, 68.129, 68.130, 68.131, 68.132, 68.133, 68.134, 68.135, 68.136; 69.70, 69.71, 69.72, 69.73, 69.74, 69.75, 69.76, 69.77, 69.78, 69.79, 69.80, 69.81, 69.82, 69.83, 69.84, 69.85, 69.86, 69.87, 69.88, 69.89, 69.90, 69.91, 69.92, 69.93, 69.94, 69.95, 69.96, 69.97, 69.98, 69.99, 69.100, 69.101, 69.102, 69.103, 69.104, 69.105, 69.106, 69.107, 69.108, 69.109, 69.110, 69.111, 69.112, 69.113, 69.114, 69.115, 69.116, 69.117, 69.118, 69.119, 69.120, 69.121, 69.122, 69.123, 69.124, 69.125, 69.126, 69.127, 69.128, 69.129, 69.130, 69.131, 69.132, 69.133, 69.134, 69.135, 69.136; 70.71, 70.72, 70.73, 70.74, 70.75, 70.76, 70.77, 70.78, 70.79, 70.80, 70.81, 70.82, 70.83, 70.84, 70.85, 70.86, 70.87, 70.88, 70.89, 70.90, 70.91, 70.92, 70.93, 70.94, 70.95, 70.96, 70.97, 70.98, 70.99, 70.100, 70.101, 70.102, 70.103, 70.104, 70.105, 70.106, 70.107, 70.108, 70.109, 70.110, 70.111, 70.112, 70.113, 70.114, 70.115, 70.116, 70.117, 70.118, 70.119, 70.120, 70.121, 70.122, 70.123, 70.124, 70.125, 70.126, 70.127, 70.128, 70.129, 70.130, 70.131, 70.132, 70.133, 70.134, 70.135, 70.136; 71.72, 71.73, 71.74, 71.75, 71.76, 71.77, 71.78, 71.79, 71.80, 71.81, 71.82, 71.83, 71.84, 71.85, 71.86, 71.87, 71.88, 71.89, 71.90, 71.91, 71.92, 71.93, 71.94, 71.95, 71.96, 71.97, 71.98, 71.99, 71.100, 71.101, 71.102, 71.103, 71.104, 71.105, 71.106, 71.107, 71.108, 71.109, 71.110, 71.111, 71.112, 71.113, 71.114, 71.115, 71.116, 71.117, 71.118, 71.119, 71.120, 71.121, 71.122, 71.123, 71.124, 71.125, 71.126, 71.127, 71.128, 71.129, 71.130, 71.131, 71.132, 71.133, 71.134, 71.135, 71.136; 72.73, 72.74, 72.75, 72.76, 72.77, 72.78, 72.79, 72.80, 72.81, 72.82, 72.83, 72.84, 72.85, 72.86, 72.87, 72.88, 72.89, 72.90, 72.91, 72.92, 72.93, 72.94, 72.95, 72.96, 72.97, 72.98, 72.99, 72.100, 72.101, 72.102, 72.103, 72.104, 72.105, 72.106, 72.107, 72.108, 72.109, 72.110, 72.111, 72.112, 72.113, 72.114, 72.115, 72.116, 72.117, 72.118, 72.119, 72.120, 72.121, 72.122, 72.123, 72.124, 72.125, 72.126, 72.127, 72.128, 72.129, 72.130, 72.131, 72.132, 72.133, 72.134, 72.135, 72.136; 73.74, 73.75, 73.76, 73.77, 73.78, 73.79, 73.80, 73.81, 73.82, 73.83, 73.84, 73.85, 73.86, 73.87, 73.88, 73.89, 73.90, 73.91, 73.92, 73.93, 73.94, 73.95, 73.96, 73.97, 73.98, 73.99, 73.100, 73.101, 73.102, 73.103, 73.104, 73.105, 73.106, 73.107, 73.108, 73.109, 73.110, 73.111, 73.112, 73.113, 73.114, 73.115, 73.116, 73.117, 73.118, 73.119, 73.120, 73.121, 73.122, 73.123, 73.124, 73.125, 73.126, 73.127, 73.128, 73.129, 73.130, 73.131, 73.132, 73.133, 73.134, 73.135, 73.136; 74.75, 74.76, 74.77, 74.78, 74.79, 74.80, 74.81, 74.82, 74.83, 74.84, 74.85, 74.86, 74.87, 74.88, 74.89, 74.90, 74.91, 74.92, 74.93, 74.94, 74.95, 74.96, 74.97, 74.98, 74.99, 74.100, 74.101, 74.102, 74.103, 74.104, 74.105, 74.106, 74.107, 74.108, 74.109, 74.110, 74.111, 74.112, 74.113, 74.114, 74.115, 74.116, 74.117, 74.118, 74.119, 74.120, 74.121, 74.122, 74.123, 74.124, 74.125, 74.126, 74.127, 74.128, 74.129, 74.130, 74.131, 74.132, 74.133, 74.134, 74.135, 74.136; 75.76, 75.77, 75.78, 75.79, 75.80, 75.81, 75.82, 75.83, 75.84, 75.85, 75.86, 75.87, 75.88, 75.89, 75.90, 75.91, 75.92, 75.93, 75.94, 75.95, 75.96, 75.97, 75.98, 75.99, 75.100, 75.101, 75.102, 75.103, 75.104, 75.105, 75.106, 75.107, 75.108, 75.109, 75.110, 75.111, 75.112, 75.113, 75.114, 75.115, 75.116, 75.117, 75.118, 75.119, 75.120, 75.121, 75.122, 75.123, 75.124, 75.125, 75.126, 75.127, 75.128, 75.129, 75.130, 75.131, 75.132, 75.133, 75.134, 75.135, 75.136; 76.77, 76.78, 76.79, 76.80, 76.81, 76.82, 76.83, 76.84, 76.85, 76.86, 76.87, 76.88, 76.89, 76.90, 76.91, 76.92, 76.93, 76.94, 76.95, 76.96, 76.97, 76.98, 76.99, 76.100, 76.101, 76.102, 76.103, 76.104, 76.105, 76.106, 76.107, 76.108, 76.109, 76.110, 76.111, 76.112, 76.113, 76.114, 76.115, 76.116, 76.117, 76.118, 76.119, 76.120, 76.121, 76.122, 76.123, 76.124, 76.125, 76.126, 76.127, 76.128, 76.129, 76.130, 76.131, 76.132, 76.133, 76.134, 76.135, 76.136; 77.78, 77.79, 77.80, 77.81, 77.82, 77.83, 77.84, 77.85, 77.86, 77.87, 77.88, 77.89, 77.90, 77.91, 77.92, 77.93, 77.94, 77.95, 77.96, 77.97, 77.98, 77.99, 77.100, 77.101, 77.102, 77.103, 77.104, 77.105, 77.106, 77.107, 77.108, 77.109, 77.110, 77.111, 77.112, 77.113, 77.114, 77.115, 77.116, 77.117, 77.118, 77.119, 77.120, 77.121, 77.122, 77.123, 77.124, 77.125, 77.126, 77.127, 77.128, 77.129, 77.130, 77.131, 77.132, 77.133, 77.134, 77.135, 77.136; 78.79, 78.80, 78.81, 78.82, 78.83, 78.84, 78.85, 78.86, 78.87, 78.88, 78.89, 78.90, 78.91, 78.92, 78.93, 78.94, 78.95, 78.96, 78.97, 78.98, 78.99, 78.100, 78.101, 78.102, 78.103, 78.104, 78.105, 78.106, 78.107, 78.108, 78.109, 78.110, 78.111, 78.112, 78.113, 78.114, 78.115, 78.116, 78.117, 78.118, 78.119, 78.120, 78.121, 78.122, 78.123, 78.124, 78.125, 78.126, 78.127, 78.128, 78.129, 78.130, 78.131, 78.132, 78.133, 78.134, 78.135, 78.136; 79.80, 79.81, 79.82, 79.83, 79.84, 79.85, 79.86, 79.87, 79.88, 79.89, 79.90, 79.91, 79.92, 79.93, 79.94, 79.95, 79.96, 79.97, 79.98, 79.99, 79.100, 79.101, 79.102, 79.103, 79.104, 79.105, 79.106, 79.107, 79.108, 79.109, 79.110, 79.111, 79.112, 79.113, 79.114, 79.115, 79.116, 79.117, 79.118, 79.119, 79.120, 79.121, 79.122, 79.123, 79.124, 79.125, 79.126, 79.127, 79.128, 79.129, 79.130, 79.131, 79.132, 79.133, 79.134, 79.135, 79.136; 80.81, 80.82, 80.83, 80.84, 80.85, 80.86, 80.87, 80.88, 80.89, 80.90, 80.91, 80.92, 80.93, 80.94, 80.95, 80.96, 80.97, 80.98, 80.99, 80.100, 80.101, 80.102, 80.103, 80.104, 80.105, 80.106, 80.107, 80.108, 80.109, 80.110, 80.111, 80.112, 80.113, 80.114, 80.115, 80.116, 80.117, 80.118, 80.119, 80.120, 80.121, 80.122, 80.123, 80.124, 80.125, 80.126, 80.127, 80.128, 80.129, 80.130, 80.131, 80.132, 80.133, 80.134, 80.135, 80.136; 81.82, 81.83, 81.84, 81.85, 81.86, 81.87, 81.88, 81.89, 81.90, 81.91, 81.92, 81.93, 81.94, 81.95, 81.96, 81.97, 81.98, 81.99, 81.100, 81.101, 81.102, 81.103, 81.104, 81.105, 81.106, 81.107, 81.108, 81.109, 81.110, 81.111, 81.112, 81.113, 81.114, 81.115, 81.116, 81.117, 81.118, 81.119, 81.120, 81.121, 81.122, 81.123, 81.124, 81.125, 81.126, 81.127, 81.128, 81.129, 81.130, 81.131, 81.132, 81.133, 81.134, 81.135, 81.136; 82.83, 82.84, 82.85, 82.86, 82.87, 82.88, 82.89, 82.90, 82.91, 82.92, 82.93, 82.94, 82.95, 82.96, 82.97, 82.98, 82.99, 82.100, 82.101, 82.102, 82.103, 82.104, 82.105, 82.106, 82.107, 82.108, 82.109, 82.110, 82.111, 82.112, 82.113, 82.114, 82.115, 82.116, 82.117, 82.118, 82.119, 82.120, 82.121, 82.122, 82.123, 82.124, 82.125, 82.126, 82.127, 82.128, 82.129, 82.130, 82.131, 82.132, 82.133, 82.134, 82.135, 82.136; 83.84, 83.85, 83.86, 83.87, 83.88, 83.89, 83.90, 83.91, 83.92, 83.93, 83.94, 83.95, 83.96, 83.97, 83.98, 83.99, 83.100, 83.101, 83.102, 83.103, 83.104, 83.105, 83.106, 83.107, 83.108, 83.109, 83.110, 83.111, 83.112, 83.113, 83.114, 83.115, 83.116, 83.117, 83.118, 83.119, 83.120, 83.121, 83.122, 83.123, 83.124, 83.125, 83.126, 83.127, 83.128, 83.129, 83.130, 83.131, 83.132, 83.133, 83.134, 83.135, 83.136; 84.85, 84.86, 84.87, 84.88, 84.89, 84.90, 84.91, 84.92, 84.93, 84.94, 84.95, 84.96, 84.97, 84.98, 84.99, 84.100, 84.101, 84.102, 84.103, 84.104, 84.105, 84.106, 84.107, 84.108, 84.109, 84.110, 84.111, 84.112, 84.113, 84.114, 84.115, 84.116, 84.117, 84.118, 84.119, 84.120, 84.121, 84.122, 84.123, 84.124, 84.125, 84.126, 84.127, 84.128, 84.129, 84.130, 84.131, 84.132, 84.133, 84.134, 84.135, 84.136; 85.86, 85.87, 85.88, 85.89, 85.90, 85.91, 85.92, 85.93, 85.94, 85.95, 85.96, 85.97, 85.98, 85.99, 85.100, 85.101, 85.102, 85.103, 85.104, 85.105, 85.106, 85.107, 85.108, 85.109, 85.110, 85.111, 85.112, 85.113, 85.114, 85.115, 85.116, 85.117, 85.118, 85.119, 85.120, 85.121, 85.122, 85.123, 85.124, 85.125, 85.126, 85.127, 85.128, 85.129, 85.130, 85.131, 85.132, 85.133, 85.134, 85.135, 85.136; 86.87, 86.88, 86.89, 86.90, 86.91, 86.92, 86.93, 86.94, 86.95, 86.96, 86.97, 86.98, 86.99, 86.100, 86.101, 86.102, 86.103, 86.104, 86.105, 86.106, 86.107, 86.108, 86.109, 86.110, 86.111, 86.112, 86.113, 86.114, 86.115, 86.116, 86.117, 86.118, 86.119, 86.120, 86.121, 86.122, 86.123, 86.124, 86.125, 86.126, 86.127, 86.128, 86.129, 86.130, 86.131, 86.132, 86.133, 86.134, 86.135, 86.136; 87.88, 87.89, 87.90, 87.91, 87.92, 87.93, 87.94, 87.95, 87.96, 87.97, 87.98, 87.99, 87.100, 87.101, 87.102, 87.103, 87.104, 87.105, 87.106, 87.107, 87.108, 87.109, 87.110, 87.111, 87.112, 87.113, 87.114, 87.115, 87.116, 87.117, 87.118, 87.119, 87.120, 87.121, 87.122, 87.123, 87.124, 87.125, 87.126, 87.127, 87.128, 87.129, 87.130, 87.131, 87.132, 87.133, 87.134, 87.135, 87.136; 88.89, 88.90, 88.91, 88.92, 88.93, 88.94, 88.95, 88.96, 88.97, 88.98, 88.99, 88.100, 88.101, 88.102, 88.103, 88.104, 88.105, 88.106, 88.107, 88.108, 88.109, 88.110, 88.111, 88.112, 88.113, 88.114, 88.115, 88.116, 88.117, 88.118, 88.119, 88.120, 88.121, 88.122, 88.123, 88.124, 88.125, 88.126, 88.127, 88.128, 88.129, 88.130, 88.131, 88.132, 88.133, 88.134, 88.135, 88.136; 89.90, 89.91, 89.92, 89.93, 89.94, 89.95, 89.96, 89.97, 89.98, 89.99, 89.100, 89.101, 89.102, 89.103, 89.104, 89.105, 89.106, 89.107, 89.108, 89.109, 89.110, 89.111, 89.112, 89.113, 89.114, 89.115, 89.116, 89.117, 89.118, 89.119, 89.120, 89.121, 89.122, 89.123, 89.124, 89.125, 89.126, 89.127, 89.128, 89.129, 89.130, 89.131, 89.132, 89.133, 89.134, 89.135, 89.136; 90.91, 90.92, 90.93, 90.94, 90.95, 90.96, 90.97, 90.98, 90.99, 90.100, 90.101, 90.102, 90.103, 90.104, 90.105, 90.106, 90.107, 90.108, 90.109, 90.110, 90.111, 90.112, 90.113, 90.114, 90.115, 90.116, 90.117, 90.118, 90.119, 90.120, 90.121, 90.122, 90.123, 90.124, 90.125, 90.126, 90.127, 90.128, 90.129, 90.130, 90.131, 90.132, 90.133, 90.134, 90.135, 90.136; 91.92, 91.93, 91.94, 91.95, 91.96, 91.97, 91.98, 91.99, 91.100, 91.101, 91.102, 91.103, 91.104, 91.105, 91.106, 91.107, 91.108, 91.109, 91.110, 91.111, 91.112, 91.113, 91.114, 91.115, 91.116, 91.117, 91.118, 91.119, 91.120, 91.121, 91.122, 91.123, 91.124, 91.125, 91.126, 91.127, 91.128, 91.129, 91.130, 91.131, 91.132, 91.133, 91.134, 91.135, 91.136; 92.93, 92.94, 92.95, 92.96, 92.97, 92.98, 92.99, 92.100, 92.101, 92.102, 92.103, 92.104, 92.105, 92.106, 92.107, 92.108, 92.109, 92.110, 92.111, 92.112, 92.113, 92.114, 92.115, 92.116, 92.117, 92.118, 92.119, 92.120, 92.121, 92.122, 92.123, 92.124, 92.125, 92.126, 92.127, 92.128, 92.129, 92.130, 92.131, 92.132, 92.133, 92.134, 92.135, 92.136; 93.94, 93.95, 93.96, 93.97, 93.98, 93.99, 93.100, 93.101, 93.102, 93.103, 93.104, 93.105, 93.106, 93.107, 93.108, 93.109, 93.110, 93.111, 93.112, 93.113, 93.114, 93.115, 93.116, 93.117, 93.118, 93.119, 93.120, 93.121, 93.122, 93.123, 93.124, 93.125, 93.126, 93.127, 93.128, 93.129, 93.130, 93.131, 93.132, 93.133, 93.134, 93.135, 93.136; 94.95, 94.96, 94.97, 94.98, 94.99, 94.100, 94.101, 94.102, 94.103, 94.104, 94.105, 94.106, 94.107, 94.108, 94.109, 94.110, 94.111, 94.112, 94.113, 94.114, 94.115, 94.116, 94.117, 94.118, 94.119, 94.120, 94.121, 94.122, 94.123, 94.124, 94.125, 94.126, 94.127, 94.128, 94.129, 94.130, 94.131, 94.132, 94.133, 94.134, 94.135, 94.136; 95.96, 95.97, 95.98, 95.99, 95.100, 95.101, 95.102, 95.103, 95.104, 95.105, 95.106, 95.107, 95.108, 95.109, 95.110, 95.111, 95.112, 95.113, 95.114, 95.115, 95.116, 95.117, 95.118, 95.119, 95.120, 95.121, 95.122, 95.123, 95.124, 95.125, 95.126, 95.127, 95.128, 95.129, 95.130, 95.131, 95.132, 95.133, 95.134, 95.135, 95.136; 96.97, 96.98, 96.99, 96.100, 96.101, 96.102, 96.103, 96.104, 96.105, 96.106, 96.107, 96.108, 96.109, 96.110, 96.111, 96.112, 96.113, 96.114, 96.115, 96.116, 96.117, 96.118, 96.119, 96.120, 96.121, 96.122, 96.123, 96.124, 96.125, 96.126, 96.127, 96.128, 96.129, 96.130, 96.131, 96.132, 96.133, 96.134, 96.135, 96.136; 97.98, 97.99, 97.100, 97.101, 97.102, 97.103, 97.104, 97.105, 97.106, 97.107, 97.108, 97.109, 97.110, 97.111, 97.112, 97.113, 97.114, 97.115, 97.116, 97.117, 97.118, 97.119, 97.120, 97.121, 97.122, 97.123, 97.124, 97.125, 97.126, 97.127, 97.128, 97.129, 97.130, 97.131, 97.132, 97.133, 97.134, 97.135, 97.136; 98.99, 98.100, 98.101, 98.102, 98.103, 98.104, 98.105, 98.106, 98.107, 98.108, 98.109, 98.110, 98.111, 98.112, 98.113, 98.114, 98.115, 98.116, 98.117, 98.118, 98.119, 98.120, 98.121, 98.122, 98.123, 98.124, 98.125, 98.126, 98.127, 98.128, 98.129, 98.130, 98.131, 98.132, 98.133, 98.134, 98.135, 98.136; 99.100, 99.101, 99.102, 99.103, 99.104, 99.105, 99.106, 99.107, 99.108, 99.109, 99.110, 99.111, 99.112, 99.113, 99.114, 99.115, 99.116, 99.117, 99.118, 99.119, 99.120, 99.121, 99.122, 99.123, 99.124, 99.125, 99.126, 99.127, 99.128, 99.129, 99.130, 99.131, 99.132, 99.133, 99.134, 99.135, 99.136; 100.101, 100.102, 100.103, 100.104, 100.105, 100.106, 100.107, 100.108, 100.109, 100.110, 100.111, 100.112, 100.113, 100.114, 100.115, 100.116, 100.117, 100.118, 100.119, 100.120, 100.121, 100.122, 100.123, 100.124, 100.125, 100.126, 100.127, 100.128, 100.129, 100.130, 100.131, 100.132, 100.133, 100.134, 100.135, 100.136; 101.102, 101.103, 101.104, 101.105, 101.106, 101.107, 101.108, 101.109, 101.110, 101.111, 101.112, 101.113, 101.114, 101.115, 101.116, 101.117, 101.118, 101.119, 101.120, 101.121, 101.122, 101.123, 101.124, 101.125, 101.126, 101.127, 101.128, 101.129, 101.130, 101.131, 101.132, 101.133, 101.134, 101.135, 101.136; 102.103, 102.104, 102.105, 102.106, 102.107, 102.108, 102.109, 102.110, 102.111, 102.112, 102.113, 102.114, 102.115, 102.116, 102.117, 102.118, 102.119, 102.120, 102.121, 102.122, 102.123, 102.124, 102.125, 102.126, 102.127, 102.128, 102.129, 102.130, 102.131, 102.132, 102.133, 102.134, 102.135, 102.136; 103.104, 103.105, 103.106, 103.107, 103.108, 103.109, 103.110, 103.111, 103.112, 103.113, 103.114, 103.115, 103.116, 103.117, 103.118, 103.119, 103.120, 103.121, 103.122, 103.123, 103.124, 103.125, 103.126, 103.127, 103.128, 103.129, 103.130, 103.131, 103.132, 103.133, 103.134, 103.135, 103.136; 104.105, 104.106, 104.107, 104.108, 104.109, 104.110, 104.111, 104.112, 104.113, 104.114, 104.115, 104.116, 104.117, 104.118, 104.119, 104.120, 104.121, 104.122, 104.123, 104.124, 104.125, 104.126, 104.127, 104.128, 104.129, 104.130, 104.131, 104.132, 104.133, 104.134, 104.135, 104.136; 105.106, 105.107, 105.108, 105.109, 105.110, 105.111, 105.112, 105.113, 105.114, 105.115, 105.116, 105.117, 105.118, 105.119, 105.120, 105.121, 105.122, 105.123, 105.124, 105.125, 105.126, 105.127, 105.128, 105.129, 105.130, 105.131, 105.132, 105.133, 105.134, 105.135, 105.136; 106.107, 106.108, 106.109, 106.110, 106.111, 106.112, 106.113, 106.114, 106.115, 106.116, 106.117, 106.118, 106.119, 106.120, 106.121, 106.122, 106.123, 106.124, 106.125, 106.126, 106.127, 106.128, 106.129, 106.130, 106.131, 106.132, 106.133, 106.134, 106.135, 106.136; 107.108, 107.109, 107.110, 107.111, 107.112, 107.113, 107.114, 107.115, 107.116, 107.117, 107.118, 107.119, 107.120, 107.121, 107.122, 107.123, 107.124, 107.125, 107.126, 107.127, 107.128, 107.129, 107.130, 107.131, 107.132, 107.133, 107.134, 107.135, 107.136; 108.109, 108.110, 108.111, 108.112, 108.113, 108.114, 108.115, 108.116, 108.117, 108.118, 108.119, 108.120, 108.121, 108.122, 108.123, 108.124, 108.125, 108.126, 108.127, 108.128, 108.129, 108.130, 108.131, 108.132, 108.133, 108.134, 108.135, 108.136; 109.110, 109.111, 109.112, 109.113, 109.114, 109.115, 109.116, 109.117, 109.118, 109.119, 109.120, 109.121, 109.122, 109.123, 109.124, 109.125, 109.126, 109.127, 109.128, 109.129, 109.130, 109.131, 109.132, 109.133, 109.134, 109.135, 109.136; 110.111, 110.112, 110.113, 110.114, 110.115, 110.116, 110.117, 110.118, 110.119, 110.120, 110.121, 110.122, 110.123, 110.124, 110.125, 110.126, 110.127, 110.128, 110.129, 110.130, 110.131, 110.132, 110.133, 110.134, 110.135, and110.136;.

Using the numerical designations set forth above in a #.# format, examples of two-compound combinations comprising at least two non-cytotoxic compounds are listed below, which may or may not further comprise other compounds in the combination:
111.112, 111.113, 111.114, 111.115, 111.116, 111.117, 111.118, 111.119, 111.120, 111.121, 111.122, 111.123, 111.124, 111.125, 111.126, 111.127, 111.128, 111.129, 111.130, 111.131, 111.132, 111.133, 111.134, 111.135, 111.136; 112.113, 112.114, 112.115, 112.116, 112.117, 112.118, 112.119, 112.120, 112.121, 112.122, 112.123, 112.124, 112.125, 112.126, 112.127, 112.128, 112.129, 112.130, 112.131, 112.132, 112.133, 112.134, 112.135, 112.136; 113.114, 113.115, 113.116, 113.117, 113.118, 113.119, 113.120, 113.121, 113.122, 113.123, 113.124, 113.125, 113.126, 113.127, 113.128, 113.129, 113.130, 113.131, 113.132, 113.133, 113.134, 113.135, 113.136; 114.115, 114.116, 114.117, 114.118, 114.119, 114.120, 114.121, 114.122, 114.123, 114.124, 114.125, 114.126, 114.127, 114.128, 114.129, 114.130, 114.131, 114.132, 114.133, 114.134, 114.135, 114.136; 115.116, 115.117, 115.118, 115.119, 115.120, 115.121, 115.122, 115.123, 115.124, 115.125, 115.126, 115.127, 115.128, 115.129, 115.130, 115.131, 115.132, 115.133, 115.134, 115.135, 115.136; 116.117, 116.118, 116.119, 116.120, 116.121, 116.122, 116.123, 116.124, 116.125, 116.126, 116.127, 116.128, 116.129, 116.130, 116.131, 116.132, 116.133, 116.134, 116.135, 116.136; 117.118, 117.119, 117.120, 117.121, 117.122, 117.123, 117.124, 117.125, 117.126, 117.127, 117.128, 117.129, 117.130, 117.131, 117.132, 117.133, 117.134, 117.135, 117.136; 118.119, 118.120, 118.121, 118.122, 118.123, 118.124, 118.125, 118.126, 118.127, 118.128, 118.129, 118.130, 118.131, 118.132, 118.133, 118.134, 118.135, 118.136; 119.120, 119.121, 119.122, 119.123, 119.124, 119.125, 119.126, 119.127, 119.128, 119.129, 119.130, 119.131, 119.132, 119.133, 119.134, 119.135, 119.136; 120.121, 120.122, 120.123, 120.124, 120.125, 120.126, 120.127, 120.128, 120.129, 120.130, 120.131, 120.132, 120.133, 120.134, 120.135, 120.136; 121.122, 121.123, 121.124, 121.125, 121.126, 121.127, 121.128, 121.129, 121.130, 121.131, 121.132, 121.133, 121.134, 121.135, 121.136; 122.123, 122.124, 122.125, 122.126, 122.127, 122.128, 122.129, 122.130, 122.131, 122.132, 122.133, 122.134, 122.135, 122.136; 123.124, 123.125, 123.126, 123.127, 123.128, 123.129, 123.130, 123.131, 123.132, 123.133, 123.134, 123.135, 123.136; 124.125, 124.126, 124.127, 124.128, 124.129, 124.130, 124.131, 124.132, 124.133, 124.134, 124.135, 124.136; 125.126, 125.127, 125.128, 125.129, 125.130, 125.131, 125.132, 125.133, 125.134, 125.135, 125.136; 126.127, 126.128, 126.129, 126.130, 126.131, 126.132, 126.133, 126.134, 126.135, 126.136; 127.128, 127.129, 127.130, 127.131, 127.132, 127.133, 127.134, 127.135, 127.136; 128.129, 128.130, 128.131, 128.132, 128.133, 128.134, 128.135, 128.136; 129.130, 129.131, 129.132, 129.133, 129.134, 129.135, 129.136; 130.131, 130.132, 130.133, 130.134, 130.135, 130.136; 131.132, 131.133, 131.134, 131.135, 131.136; 132.133, 132.134, 132.135, 132.136; 133.134, 133.135, 133.136; 134.135, 134.136; and 135.136.

As Figure 7 indicates, the methods described herein provide for the observance of optima in dose response curves. In one embodiment, the methods described herein utilize a dose response curve to select drug concentrations for a patient. In another embodiment, drug concentrations are selected that induce apoptosis in greater than about 75% of the cells in the sample. In another embodiment, drug concentrations are selected that induce apoptosis in greater than about 50% of the cells in the sample. In another embodiment, drug concentrations are selected that induce apoptosis in greater than about 25% of the cells in the sample. Furthermore, standard drug concentrations, such as a drug's EC₅₀ value, may not correspond to the desired dose to administer a polytherapy treatment regimen. In another embodiment, the methods described herein utilize optima to select drug concentrations for a patient. In another embodiment, the methods described herein utilize the EC₅₀ to select drug concentrations for a patient. In another embodiment, the methods described herein utilize the EC₉₀ to select drug concentrations for a patient. In another embodiment, the methods described herein utilize the cellular response of normal cells to select the desired drug composition and concentration for a neoplastic condition.

The methods described herein can also be used to evaluate the kinetic profile of both cytotoxic and non-cytotoxic drug compositions. As Figure 8 indicates, the kinetics for an individual patient may vary for different drug compositions. In one embodiment, the methods described herein determine a drug's kinetic profile for a certain indication. In another embodiment, the methods described herein determine a drug composition's kinetic profile for a certain indication. In some embodiments, a drug regimen is selected based upon a drug's kinetic profile for a certain indication.

Figure 8 also indicates that the methods described herein are useful for measuring the ability of different drug compositions to induce apoptosis at different time periods. Furthermore, the methods described herein are useful for evaluating the differences in the induction of apoptosis between different drug compositions after different time periods have elapsed. In one embodiment, the method detects the induction of apoptosis at about 10, 12, 14, 16, 18, 20, 22, or 24 hours, or a range defined by any two of the preceding values. In another embodiment, the method detects the induction of apoptosis at about 36 or 48 hours. In another embodiment, the method detects the induction of apoptosis at about 72 hours.

A related measurement is the minimum time that a drug needs to be incubated with the cells to effectively induce programmed cell death (*i.e.,* apoptosis), as shown in Figure 28. For this measurement, a similar analysis can be made by incubating the drug compositions for 15 minutes, followed by washing the drug away, and waiting 48 hours to measure apoptosis. In one embodiment, the method detects the induction of apoptosis after incubating prior to the washing of the drug for about 30 minutes, 45 minutes, 1 hour, 2 hours, or 4 hours, or a range defined by any two of the preceding values. In another embodiment, the method detects the induction of apoptosis at about 24 or 48 hours. In another embodiment, the method detects the induction of apoptosis at about 72 hours.

### EXAMPLES

### EXAMPLE 1: Flow Cytometric Detection of Apoptotic Normal and Neoplastic Cells

An ex vivo therapeutic index can be determined by measuring the ability of a drug composition to induce apoptosis. Figures 1 and 2 depict the ability to detect apoptotic cells and differentiate between normal and tumor phenotypes using flow cytometry. In Figure 1, the reagent Annexin V coupled to Fluorescein Isothiocyanate (FITC) was used to detect phosphatidylserine expression on apoptotic cells. Fluorescein intensity is displayed on the y-axis, and cell size is displayed on the x-axis. Figure 1 illustrates the ability to identify apoptotic cells (upper left box) and live cells (lower right cluster) and demonstrates that the simultaneous use of appropriate combinations of monoclonal antibodies and multiparametric analysis strategies can allow for the discrimination of leukemic cells from residual normal cells present in samples from patients with hematological disorders. Figure 2 depicts a precursor B-ALL adult case displaying BCR/ABL gene rearrangements [t(9;22)positive]. Two cellular subsets, leukemic (light grey) and normal (dark grey), were detected among the CD19 positive cells using multiple monoclonal antibody staining analyzed by quantitative flow cytometry. The leukemic cells express a unique phenotype (homogenous expression of CD34, but low and relatively heterogeneous CD38 expression) associated with the translocation.

### EXAMPLE 2: Protocol for the ex vivo Evaluation of Drug Compositions

An ex vivo screening process for drug compositions is schematically shown in Figure 3. A sample of blood can be split into small aliquots that are distributed into well plates of any suitable size. These well plates contain individual drugs or drug combinations, each at various concentrations. To facilitate optimal assay development, a sample is diluted in RPMI media and concentrated at about 20,000 leukemic cells per well. In parallel, another aliquot is tested for immunophenotypic identification using flow cytometry for the identification of normal and pathologic cells and the detection of basal apoptosis. Control wells without any drug can be included (not shown) to identify the spontaneous level of apoptosis not associated with drug treatment.

After approximately 48 hours, each well with the sample exposed to the drugs is treated with a buffer to lyse the erythrocyte population and concentrate the leukocyte population. Each well is then incubated with Annexin V for apoptosis detection with an antibody combination to accurately detect and identify tumor cells and normal cells. It is possible to evaluate, using flow cytometry, the effect of each drug on each cell type and to quantify the level of selective cell death induced by each drug.

Results can then be evaluated and a new test can be started with an additional aliquot in order to confirm more relevant results, such as the 10 best drug compositions and concentrations identified in an earlier study. Selection of the appropriate drug or drug combination that selectively induced apoptosis on neoplastic cells, such as leukemia cells, can be made after the assay is performed for a patient sample.

### EXAMPLE 3: Individual Patient Responses Demonstrate the Cytotoxic Effects of Different Drugs Currently Approved for Chronic Lymphocytic Leukemia Treatment

The present methods have been used to analyze 30 µM concentrations of chlorambucil, cyclophosphamide, vincristine, mitoxantrone, and doxorubicin-five drugs currently approved for chronic lymphocytic leukemia (CLL)-in various patients. The results of the efficacy of individually approved cytotoxic drugs for inducing apoptosis in malignant cells of 9 ex vivo patient samples are provided in Figure 4. Figure 4 demonstrates that there is a high person-to-person variability in the drug responses, highlighting an important use for the personalized medicine tests described herein.

Regarding patient response to individual drug treatments at 30 µM concentrations, several drugs generally had poor patient response, defined as inducing less than 60% apoptosis in patient samples as measured by Annexin V positive cells. Additionally, Figure 4 indicates that some of the patients (specifically P1.0105, P2.0019, and P2.035) showed extreme resistance ex vivo to mitoxantrone. Alternatively, for the two patients denoted by a star, only doxorubicin is very effective, and the other 3 drugs are resistant, indicating that this type of test could be very helpful in guiding treatment to these patients. Although results obtained from ex vivo assays may be more accurate at predicting drug resistance than drug efficacy (e.g., as shown in Table 2), if a drug does not kill malignant cells ex vivo, it is unlikely to kill the same cells in vivo.

### EXAMPLE 4: Induction of Apoptosis by Cytotoxic and Non-cytotoxic Drugs in CLL Samples

The ability of non-cytotoxic drugs to induce apoptosis in ex vivo samples was explored using peripheral blood samples obtained from CLL (Chronic Lymphatic Leukemia) patients. Approximately 900 commercially available drugs were screened ex vivo, one by one, in 23 different patient samples. Figure 6 shows the efficacy of several clinically approved cytotoxic drugs and several non-cytotoxic drugs for the hematological neoplasms in these CLL ex vivo samples. The results are graphed as % apoptosis. As the results indicate, clinically approved drugs induce apoptosis in more than 75% of the malignant cells. From left to right, the non-cytotoxic drugs studied were paroxetine, fluoxetine, sertraline, guanabenz, and astemizole. From left to right, the cytotoxic drugs studied were fludarabine, chloramabucil, and mitoxantrone. Figure 6 demonstrates that the non-cytotoxic drugs selectively kill the same malignant cells with ex vivo efficacy similar to that of the approved cytotoxic drugs. This unexpected result indicates that these non-cytotoxic drugs could have a significant therapeutic benefit for the patients studied in Figure 6.

Additional validation studies for the induction of apoptosis by noncytotoxic drugs were performed. Figure 7 compares the differences in the cytotoxic effects of paroxetine between malignant leukemic cells and non-malignant T and NK cells. At a concentration of approximately 30 µM, paroxetine induced apoptosis in nearly 100% of the leukemic cells. However, at the same concentration of approximately 30 µM, paroxetine induced apoptosis in only 15% of the T and NK cells. Consequently, Figure 7 indicates that paroxetine selectively induces apoptosis in malignant CLL cells ex vivo and minimally affects non-malignant NK and T cells.

Non-cytotoxic drugs commonly prescribed in treatment protocols can have a highly selective apoptotic efficacy against malignant cells. One such case is shown in Figure 12 for a CLL patient, which displays the percentage of Annexin V positive cells induced by different drugs. A high variability was observed in the cytotoxic effect of different drugs used in CLL treatment (*i.e.,* vincristine, mitoxantrone, and cyclophosphamide). Surprisingly, two non-cytotoxic compounds that are usually included for treating side effects caused by chemotherapy (*i.e.,* omeprazole and acyclovir) showed similar apoptotic rates as the cytotoxic agents. Thus, personalized medicine tests that include non-cytotoxic drugs as described herein, including in the examples provided herein, may provide unexpected potential therapeutic benefits for patients. For example, adding non-cytotoxic drugs to the ex vivo tests may allow for novel and unexpected treatments that are complementary to standard treatments.

### EXAMPLE 5: 24 Hour and 48 Hour Analysis of the Ability of Selected Drugs to Induce Apoptosis

The kinetics of apoptosis induction was evaluated by observing the percentage of cells undergoing apoptosis at 24 hour and 48 hour time points. Figure 8 indicates that the non-cytotoxic drug sertraline eliminates malignant CLL cells faster than approved cytotoxics. In Figure 8, whole blood samples collected from patients diagnosed with CLL were analyzed for their response to drug treatment. The whole blood samples were incubated with either sertraline or one of three drugs (fludarabine, chlorambucil, or mitoxantrone) that are currently approved for the treatment of CLL. Following the addition of the drugs, the whole blood samples were incubated for either 24 or 48 hours prior to the analysis. As the results in Figure 8 indicate, the kinetics of apoptosis induction is faster for sertraline (more than 90% induction of apoptosis after 24 hours) than for the other 3 clinically approved drugs (approximately 40%, 45%, and 50% induction of apoptosis after 24 hours, respectively). Sertraline therefore induced almost full apoptosis in 24 hours, while the other CLL drugs required 48 hours for optimum efficacy.

Faster apoptosis ex vivo could translate to better efficacy in vivo. However, Figure 8 also indicates that the effectiveness of these four drugs is approximately equal after 48 hours. Figure 8 emphasizes the utility in evaluating multiple incubation times to select the optimal treatment for each patient. Further, Figure 8 indicates that several variables should be studied (e.g., drug compositions and incubation times) for the development of an optimal polytherapy treatment.

### EXAMPLE 6: Differential Induction of Apoptosis by Drugs in the Same Pharmacological Class

Paroxetine is a selective serotonin reuptake inhibitor (SSRI). Other members of the SSRI class of compounds were tested in order to determine if the SSRI pharmacological class of drugs has universal apoptotic induction properties. Figure 9 summarizes the ability of 6 SSRIs (paroxetine, fluoxetine, sertraline, citalopram, fluvoxamine, and zimelidine) to induce apoptosis in malignant CLL cells. As the results in Figure 9 demonstrate, of the 6 drugs, only 3 (paroxetine, fluoxetine, and sertraline) induce apoptosis similarly to clinically approved cytotoxic drugs. These differences among drugs that share similar mechanisms of action and the same pharmacological profile highlights the need for the ex vivo test methodology described herein to select among pharmacologically analogous drugs. These differences also indicate the need for ex vivo testing of each drug without regard to pharmacological class. These differences further highlight the importance of the ability to study large numbers of variables in order to develop a personalized medicine test.

### EXAMPLE 7: Frequency of Patients Exhibiting Over 80% Apoptotic Induction by the Same Drug Treatment

The apoptotic efficacy of non-cytotoxic drugs varies more from person-to-person than the apoptotic efficacy of approved cytotoxic drugs (e.g., as shown in Figure 4). This variation is also illustrated in Figure 10. An initial screen of 23 samples (combination of whole blood or bone marrow) from patients diagnosed with CLL was conducted with approximately 2,000 compounds (some samples were not sufficient to screen all compounds). The screen measured the ability of each compound to induce apoptosis selectively in the leukemic cell population of each patient. A compound was considered a "hit" for a particular patient if it induced a level of apoptosis greater than 80% in the leukemic population while having little or no effect in the normal cell population.

The results in Figure 10 indicate that only a small number of drugs were effective in a majority of patient samples (80-100%). Similarly, only 10 additional compounds were effective in 60-80% of the patient samples. 45 drugs were effective in 40-60% of samples, 66 drugs were effective in 20-40% of samples, and 229 additional drugs were effective in less than 20% of the samples. Adding these drugs means that 353 drugs were effective in inducing apoptosis ex vivo in these 23 samples. These are mostly drugs that have not been previously noted as treatments for hematological malignancies, indicating that the development of a personalized medicine test will require the screening of large numbers of drugs, both cytotoxic and non-cytotoxic, to determine an optimal drug regimen. Such unexpected data may have major clinical implications for the treatment of hematological neoplasms.

Regulatory agencies typically only approve the use of a small subset of non-cytotoxic drugs to patients with hematological neoplasms intended to palliate the effects of cytotoxic treatments. Nonetheless, the potential efficacy observed here for most other non-cytotoxic drugs and claimed herein could, in time, become part of these treatments, as the concept of personalized medicine advances further. The polytherapy personalized medicine tests described herein can identify potentially useful non-cytotoxic drugs for each individual patient, representing a novel and therapeutically beneficial approach that was previously unavailable.

### EXAMPLE 8: Potentiation of the Efficacy of an Approved Cytotoxic Drug by a Non-cytotoxic Drug

As an example of the potential benefits of a personalized medicine screening test, the compound sertraline, identified as a hit for against a CLL patient sample, can potentiate the response of chlorambucil. This is shown in Figure 11. Clorambucil is the most commonly prescribed drugs used for the frontline therapy of CLL in about a 25% of patients that cannot withstand fludarabine-based treatments. As chlorambucil is highly cytotoxic, and causes multiple severe side effects, finding a way to limit the dosage would greatly benefit patients. In this particular test, sertraline is an antidepressant that is available in a generic formulation and has been in the market for many years. Chlorambucil alone at the concentrations shown did not induce much apoptosis (lower curve), but the presence of a sub-maximal dose of sertraline greatly enhanced the level of apoptosis (upper curve). Such an example demonstrates potential concomitant therapy options that may have the ability to enhance the response of the prescribed chemotherapeutic treatment.

These results demonstrate a need for the development of personalized medicine tests using high-throughput screening, such as methods using flow cytometry, that allow for exploration of the effect of possible drugs and drug combinations, including all approved drugs and in particular non-cytotoxic concomitant used to palliate the side effects of the chemotherapeutic strategies.

### EXAMPLE 9: Design of a Polytherapy Personalized Medicine (PM) Test for CLL according to PETHEMA Treatment Protocols

A 96-well plate design for a personalized medicine test for a patient with CLL (chronic lymphocytic leukemia) is illustrated in Figure 13, without considering non-cytotoxic drugs. In each plate, column 1 contains 0.34%solution of DMSO as a negative control and column 12 contains 50 µM solution of paroxetine and 50 µM solution of staurosporin (wells E-H) as positive controls. The drugs and drug combinations in the plates are those approved for this indication in conventional treatment protocols. In each row, wells 2-6 and 7-11 include 5 point dose response of each of these drugs and drug combinations, with a dilution factor of 2:3. Columns 2 and 7 therefore contain the highest concentrations of drugs, which were established for each drug according to its therapeutic range. Chlorambucil (CH); fludarabine (F1); maphosphamide (MA); doxorubicin (DO); vincristine (VI); prednisolone (Pr); mitoxantrone (MI); 2-chlorodeoxyadenosine (2-CDA); flavopiridol (FL); melphalan (ME); me-Prednisolona (MEPR); bendamustine (BE); pentostatin (PE); rituximab (RIT); alemtuzumab (ALE).

### EXAMPLE 10: Design of a PM Test for MM according to PETHEMA Treatment Protocols

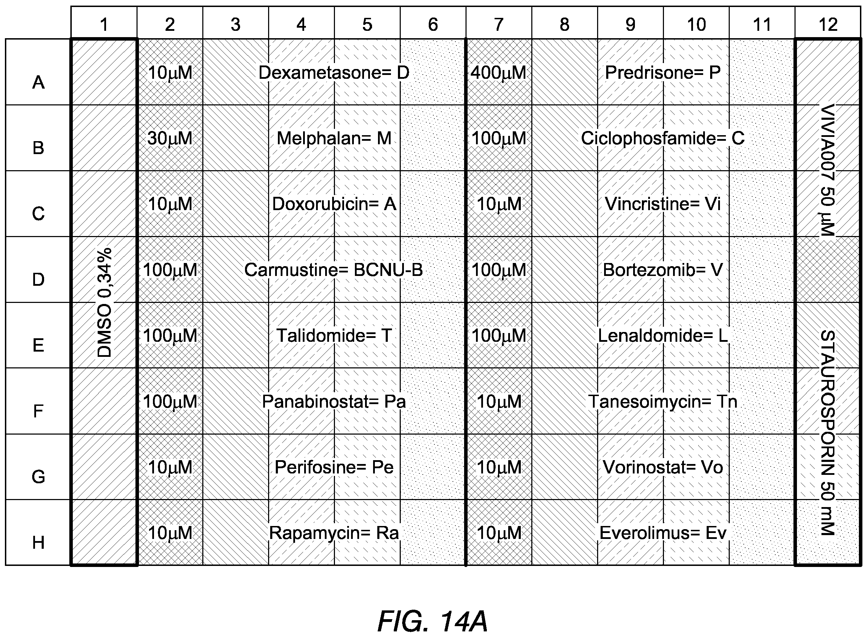

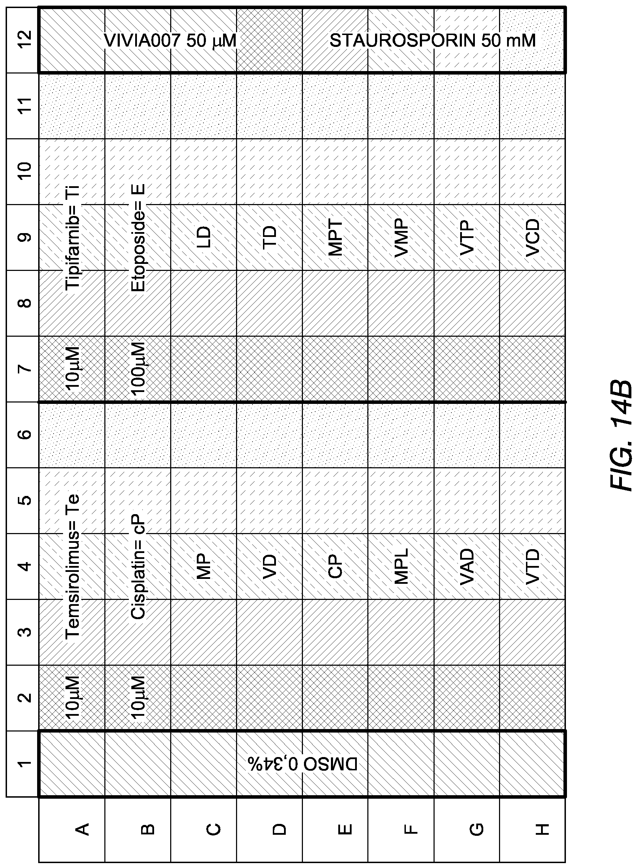

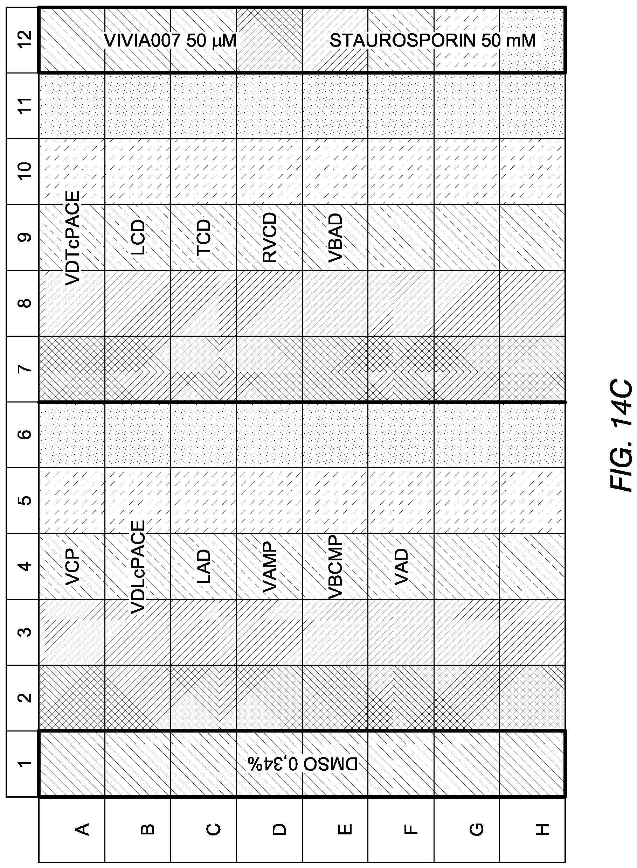

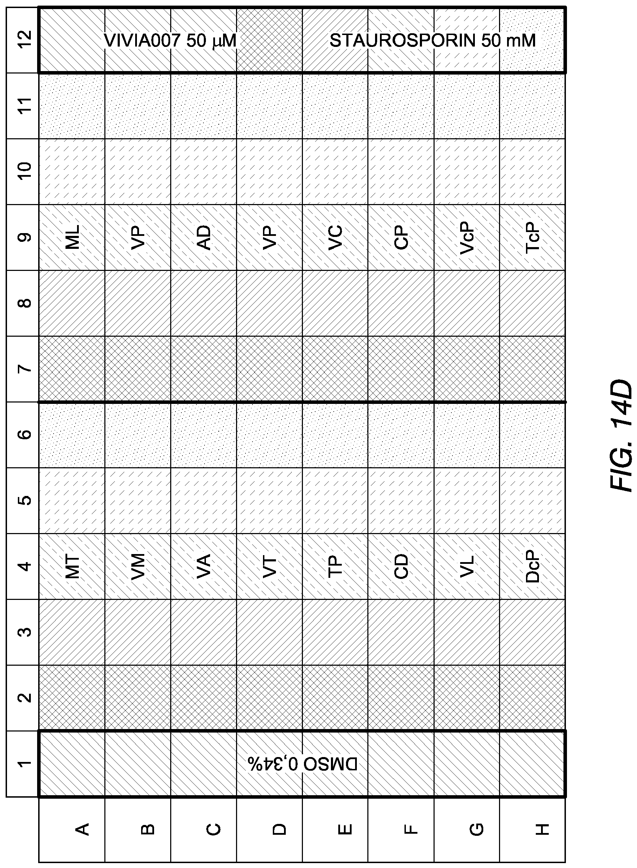

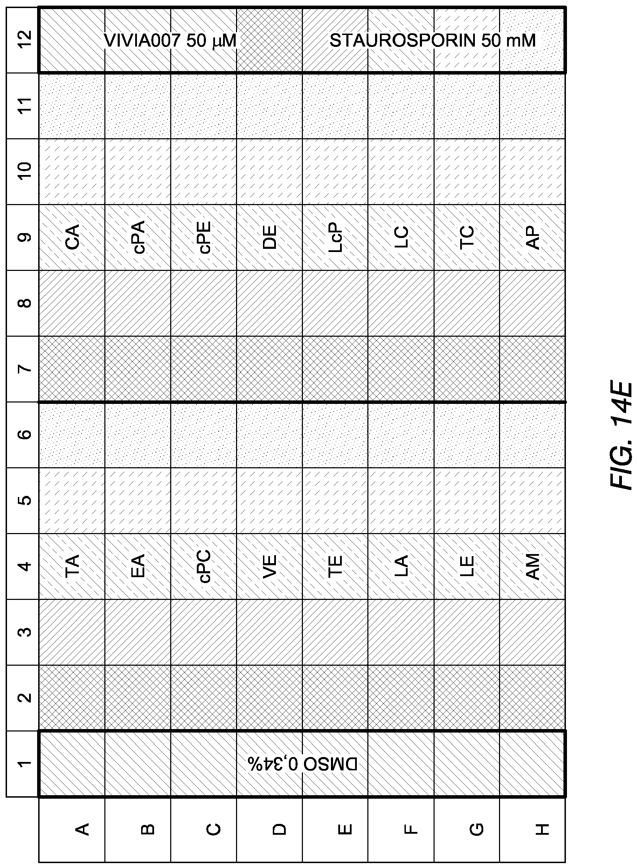

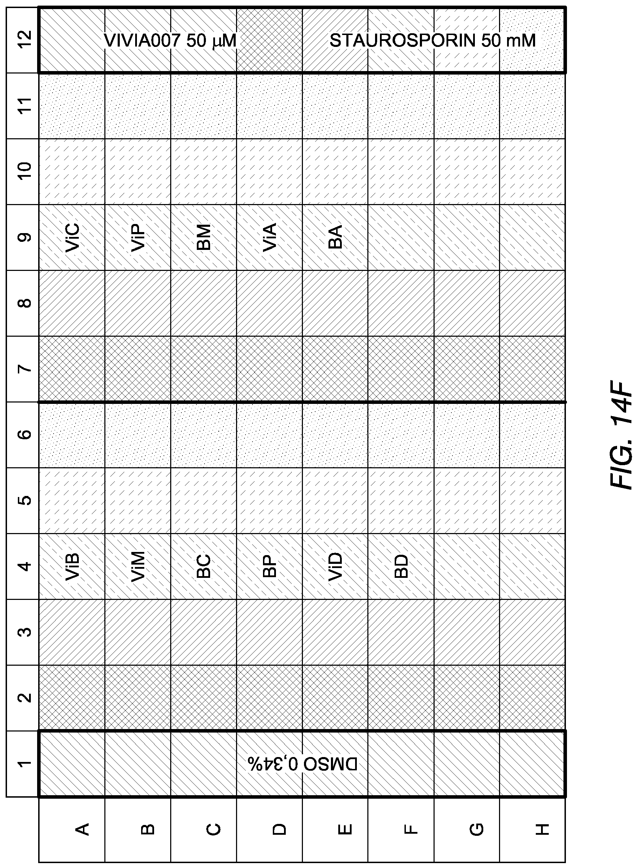

A 96-well plate design for a personalized medicine test for a patient with MM (multiple myeloma) is illustrated in Figure 14 in the six panels A to F, without considering non cytotoxic drugs. The plate layout was created according to current treatment protocols, including individual drugs. In each plate, column 1 contains 0.34%solution of DMSO as a negative control and column 12 contains 50 µM solution of paroxetine and 50 µM solution of staurosporin (wells E-H) as positive controls. The drugs and drug combinations in the plates are those approved for this indication in conventional treatment protocols. In each row, wells 2-6 and 7-11 include 5 point dose response of each of these drugs and drug combinations, with a dilution factor of 2:3. Columns 2 and 7 therefore contain the highest concentrations of drugs, which were established for each drug according to its therapeutic range. In addition, plates from 4-6 contain all possible double combinations of the approved protocols in order to clarify the synergy between all the drugs. Dexametasone (D); prednisone (P); melphalan (M); cyclophosphamide (C); doxorubicin (A); vincristine (Vi); carmustine (BCNU-B); bortezomib (V); talidomide (T); lenalidomide (L); panabinostat (Pa); tanespimycin (Tn); perifosine (Pe); vorinostat (Vo); rapamycin (Ra); everolimus (Ev); temsirolimus (Te); tipifamib (Ti); cisplatin (cP); etoposide (E).

### EXAMPLE 11: Design of a PM test for ALL according to PETHEMA Treatment Protocols

A 96-well plate design for a personalized medicine test for a patient with ALL (acute lymphoblastic leukemia) is illustrated in Figure 15. The plate layout was created according to current treatment protocols, including drugs used in monotherapy. The study design is intended to determine the ability of the following drugs to induce apoptosis in a patient sample: methotrexate (MTX), 6-mercaptopurine (6MP), cytarabine (ARA-C), daunorubicin (DNR), adriamycin, mitoxantrone (M), etoposide, teniposide (VM-26), cyclophosphamide (CF), ifosfamide (IFOS), vincristine (V), vindesine (VIND), asparaginase (L-ASA), imatinib (IMAT), rituximab (R), prednisone (P), hydrocortisone (HC), dexamethasone (DXM), leucovorin (Foli), mesna, omeprazole (Om), ondansetron (O), allopurinol (Allop), and filgrastim (GCSF). The design of this 96-well plate affords the simultaneous comparison of numerous chemotherapeutic strategies. The design also tests the effects of adjuvant drugs and drugs that are used to palliate side effects singly or in combination with monotherapy drugs.

### EXAMPLE 12: Design of a PPM Test for MDS according to PETHEMA Treatment Protocols

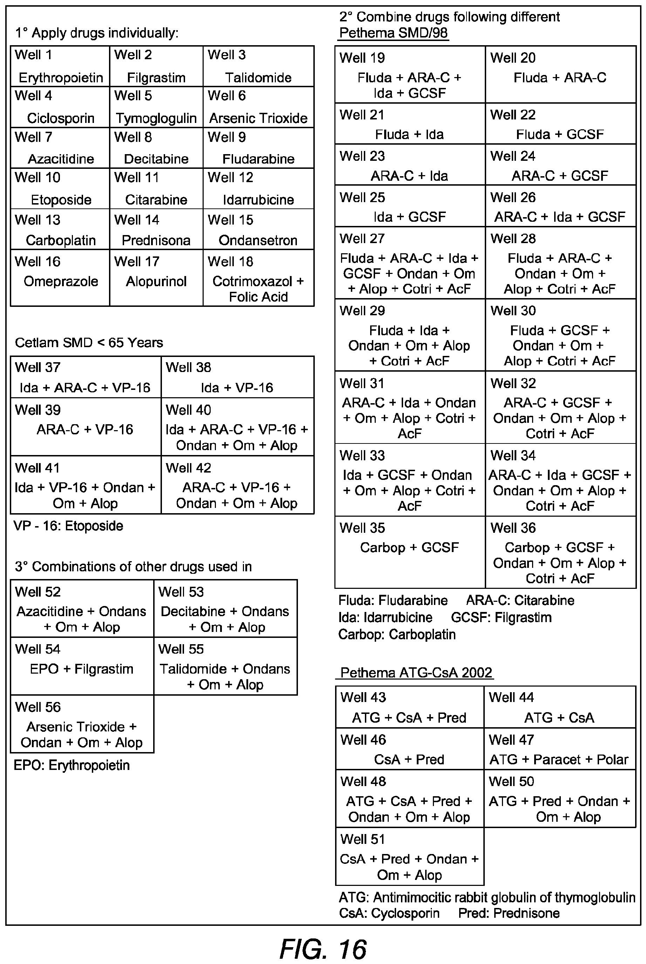

A 96-well plate design for a personalized medicine test for a patient with MDS (myelodysplastic syndrome) is illustrated in Figure 16. The plate layout was created according to current treatment protocols, including drugs used in monotherapy. The study design is intended to determine the ability of the following drugs to induce apoptosis in a patient sample: erythropoietin (EPO), filgrastim (GCSF), thalidomide, cyclosporine (CsA), thymoglobulin (ATG), arsenic trioxide, azacitidine, decitabine, fludarabine (Fluda), etoposide (VP-16), cytarabine (ARA-C), idarubicin (Ida), carboplatin (Carbop), prednisone (Pred), ondansetron (Ondans), omeprazole (Om), allopurinol (Alop), co-trimoxazole (Cotri), and folic acid (AcF). The design of this 96-well plate affords the simultaneous comparison of numerous chemotherapeutic strategies. The design also tests the effects of adjuvant drugs and drugs that are used to palliate side effects singly or in combination with monotherapy drugs.

### EXAMPLE 13: Design of a PM Test for AML according to PETHEMA Treatment Protocols

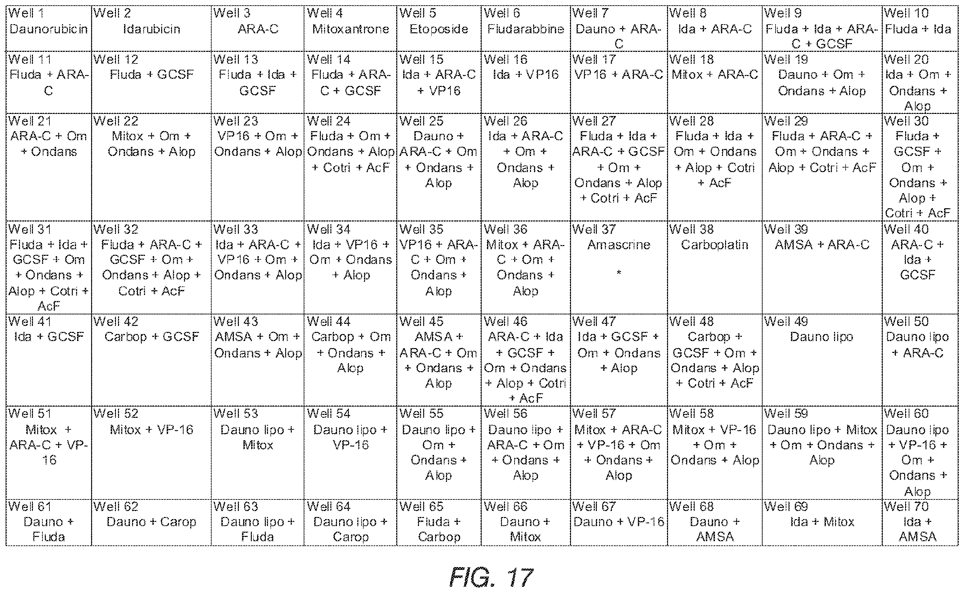

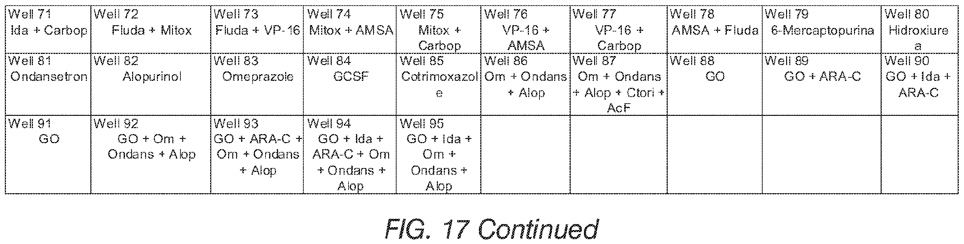

A 96-well plate design for a personalized medicine test for a patient with AML (acute myeloblastic leukemia, not M3) is illustrated in Figure 17. The plate layout was created according to current treatment protocols, including drugs used in monotherapy. The study design is intended to determine the ability of the following drugs to induce apoptosis in a patient sample: daunorubicin (Dauno), idarubicin (Ida), cytarabine (ARA-C), mitoxantrone (Mitox), etoposide (VP16), fludarabine (Fluda), filgrastim (GCSF), omeprazole (Om), ondansetron (Ondans), allopurinol (Alop), co-trimoxazole (Cotri), folic acid (AcF), amsacrine (AMSA), carboplatin (Carbop) liposomal daunorubicin (Dauno lipo), gentuzumab ozogamicina (GO), and hydroxylurea. The design of this 96-well plate affords the simultaneous comparison of numerous chemotherapeutic strategies. The design also tests the effects of adjuvant drugs and drugs that are used to palliate side effects singly or in combination with monotherapy drugs.

### EXAMPLE 14: Design of a PM Test for AML-M3 according to PETHEMA Treatment Protocols

A 96-well plate design for a personalized medicine test for a patient with AML-M3 (acute myeloblastic leukemia M3) is illustrated in Figure 18. The plate layout was created according to current treatment protocols, including drugs used in monotherapy. The study design is intended to determine the ability of the following drugs to induce apoptosis in a patient sample: tretinoin (ATRA), idarubicin (Ida), mitoxantrone (Mitox), citarabine (ARA-C), 6-mercaptopurine (6-MP), methotrexate (MTX), ondansetron (Ondans), allopurinol (Alop), omeprazole (Om), dexamethasone (Dexa), daunorubicin (Dauno), etoposide (VP-16), fludarabine (Fluda), carboplatin (Carbop), liposomal daunorubicin (Dauno lipo), co-trimoxazole (Cotri), and folic acid (FAc). The design of this 96-well plate affords the simultaneous comparison of numerous chemotherapeutic strategies. The design also tests the effects of adjuvant drugs and drugs that are used to palliate side effects singly or in combination with monotherapy drugs.

### EXAMPLE 15: MTT Proliferation Assay in Primary Origin Leukemic Cell Lines

TOM-1 cells were derived from the bone marrow cells of a patient with Ph1-positive acute lymphocytic leukemia (ALL). MOLT-4 cells were derived from a human acute lymphoblastic leukemia cell line. A standard MTT assay was performed to determine the IC₅₀ for the individual items to be tested on specific cell lines. The MTT assay is based on the cleavage of the yellow tetrazolium salt MTT to purple formazan crystal by metabolic active cells. The formazan is then solubilized, and the concentration determined by optical density at 570 nm. Six to eight different concentrations of sertraline, in triplicates, were analyzed at 24 hours post-treatment.

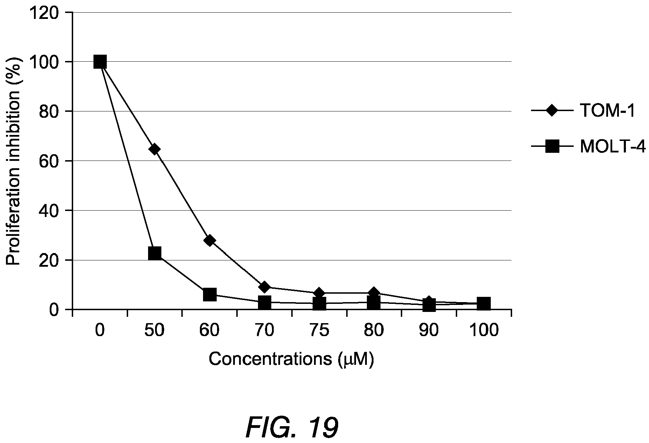

The effect of sertraline on the inhibition of cell proliferation at 24 hours in the TOM-1 and MOLT-4 primary origin cell lines was assessed. The IC₅₀ for the MOLT-4 cell line was 40 µM, and the IC₅₀ for TOM-1 cell line was 50 µM (Figure 19).

### EXAMPLE 16: Apoptosis Determination using Apoptosis ELISA

A one step sandwich ELISA was performed using the TOM-1 and MOLT-4 cells from Example 15. The one step sandwich ELISA is based in the quantification of histone-complexed DNA fragments (mono- and oligonucleosomes) out of the cytoplasm of cells after the induction of apoptosis or when released from necrotic cells.

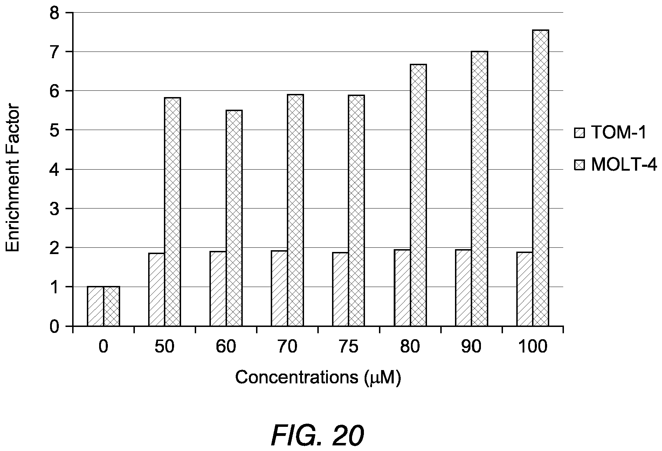

The effect of sertraline on the induction of apoptosis at 24 hours in the TOM-1 and MOLT-4 primary origin cell lines was assessed. Sertraline increased the Apoptosis Index up to 7-fold in the MOLT-4 cells (Figure 20).

### EXAMPLE 17: Active Caspase-3 Determination

Caspase-3 activation was determined using a Western blot of extracts from two acute lymphoblastic cell lines (TOM-1 and MOLT-4) exposed to increased concentrations of sertraline. Extracts were taken at 24 and 48 hours. Active caspase-3 is a protease that serves as a marker of apoptosis.

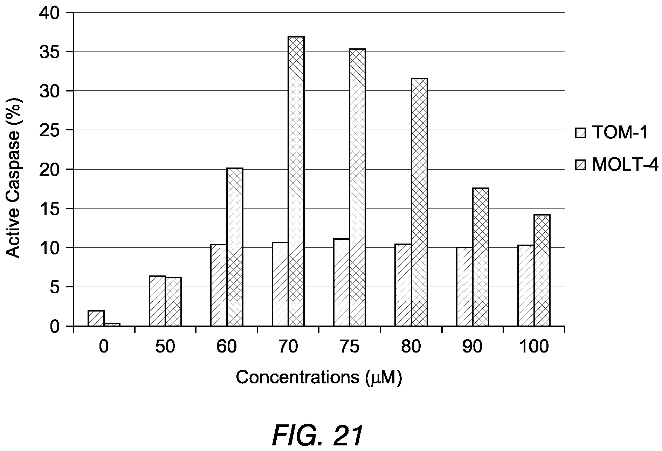

The effect of sertraline on the induction of active caspase-3 at 24 hours in TOM-1 and MOLT-4 primary origin cell lines was assessed. Sertraline dramatically induced active caspase-3 in the MOLT-4 cells, with the maximum induction occurring at the 70 um concentration (Figure 21).

### EXAMPLE 18: Ex vivo Efficacy of Polytherapy Combinations in a CLL sample

Polytherapy combinations of rituximab, fludarabine, mitoxantrone, and cyclophosphamide were tested in a CLL sample at maximum concentrations. Cyclophosphamide was not tested directly, but rather by its metabolite maphosphamide (an active compound in the human body). The four principal individual drugs were resistant (*i.e.,* rituximab, fludarabine, mitoxantrone, and cyclophosphamide) (Figure 22, left side). A polytherapy protocol with fludarabine and rituximab was also resistant (Figure 22, center). Three polytherapy protocols (*i.e.,* fludarabine and cyclophosphamide; fludarabine, cyclophosphamide, and mitoxantrone; and fludarabine, cyclophosphamide, and rituximab) were very sensitive, eliminating essentially all leukemic cells (right side) (Figure 22).

Five-point response curves were generated for the combinations of fludarabine and cyclophosphamide; fludarabine, cyclophosphamide, and mitoxantrone; and fludarabine, cyclophosphamide, and rituximab to characterize ex vivo efficacy (Figures 23-25). These curves show a significant synergistic effect for these drug combinations, highlighting the importance of evaluating drug combinations as described herein.

### EXAMPLE 19: Effect of Single Drugs at Five Different Concentrations in Two Patients

Fludarabine, maphosphamide, and the combination of fludarabine and maphosphamide were tested in two patients, P2.0144 (Figure 26, left) and P2.0149 (Figure 26, right), at five concentrations. The Combination Index (CI) was calculated using the program Calcusyn (Chou et al., Adv Enzyme Regul 1984; 22:27-55; Chou et al., Eur J Biochem 1981; 115(1):207-16) to characterize the synergy for the combinations at each concentration (shown at the top of the panels). The CI is a quantitative measure of the degree of drug interaction in terms of additive effects. Synergism occurs where CI<1, additive effect occurs where CI∼1, and antagonism occurs where CI>1. The Dose-Reduction Index (DRI) is a measure of how much the dose of each drug in synergistic combination may be reduced at a given effect level compared with the dose of each drug alone.

Figure 27 shows the Combination Index versus fractional effect based on Chou and Talalay method (top panel). Cross markers indicate observed values. The black line corresponds to a model simulation. The middle panel shows the drug interaction Isobologram based on Chou and Talalay method at three different response levels (ED₅₀, ED₇₅, and ED₉₀) based on dose response estimations. Points drawn on each axis correspond to doses estimated for these responses for each drug individually. Straight lines represent the additive effect area for combinations. Points for combined doses found below these lines denote drug synergism. Figures 26 and 27 demonstrate that the combination of fludarabine and maphosphamide enhanced cytotoxicity relative to the single drug efficacy against leukemic CLL B-cells.

### EXAMPLE 20: Effect of Incubation Time on the Efficacy of Drugs to Induce Apoptosis in Malignant Cells in CLL samples

The effect of incubation time on the efficacy of fludarabine and sertraline in inducing apoptosis in malignant cells in CLL samples was tested. Figure 28 shows curves for fludarabine (left panel) and sertraline (right panel), where apoptosis was measured at either 24 hours (top) or 48 hours (bottom). In both cases, the drugs were incubated with the sample for 30 min, 4 hours, or 8 hours before washing the drug away and waiting 24 or 48 hours to measure apoptosis. Sertraline, a non-cytotoxic drug that induces apoptosis in CLL malignant cells, demonstrated faster kinetics than fludarabine.

### EXAMPLE 21: Number of Drug Combinations

Calculations were performed to assess the number of 2 drug, 3 drug, and 4 drug combinations for 15 drugs.

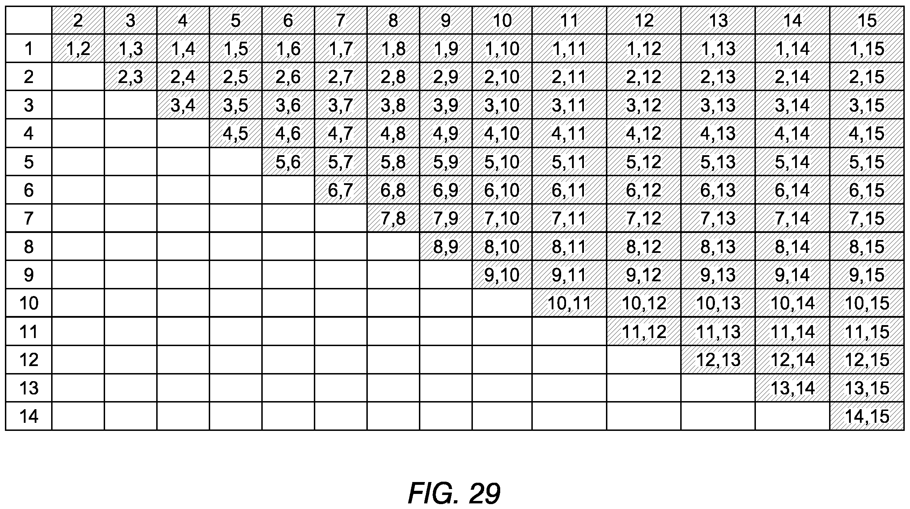

Figure 29 represents the number of unique 2 drug combinations that can be made from 15 individual drugs. Each drug is represented by a number and the shaded cells represent the 2 drug combinations. This gives a total of 105 unique combinations of 2 drugs.

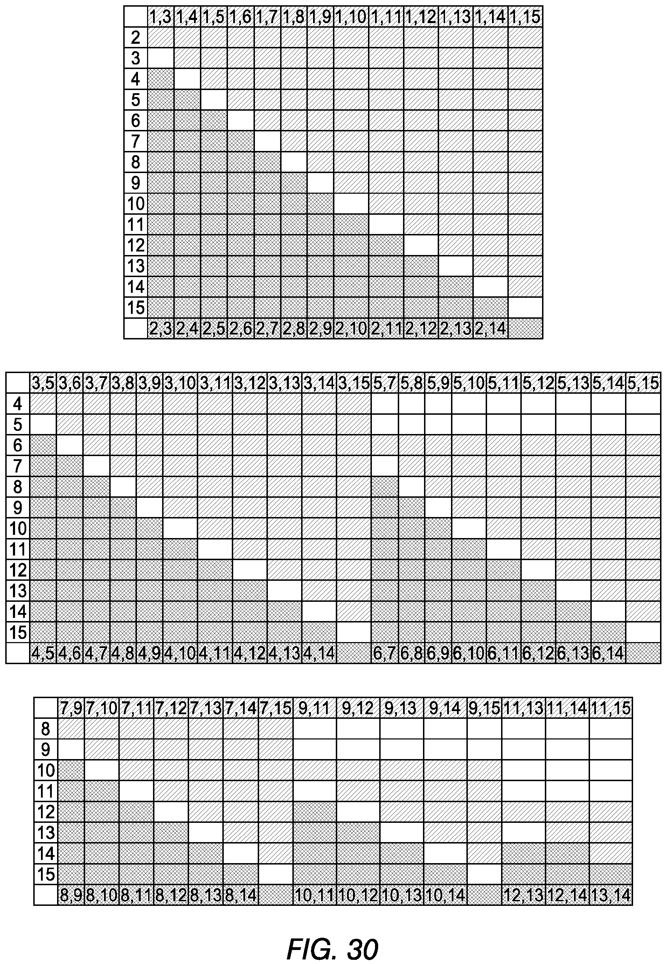

Figure 30 represents the number of unique 3 drug combinations that can be made from 15 individual drugs. All 2 drug combinations listed in the top row of each matrix will be combined with the single drugs on the left column when the boxes in the center are shaded light gray. All 2 drug combinations listed in the bottom row of each matrix will be combined with the single drugs on the left column when the boxes in the center are shaded dark gray. This gives a total of 455 unique combinations of 3 drugs.

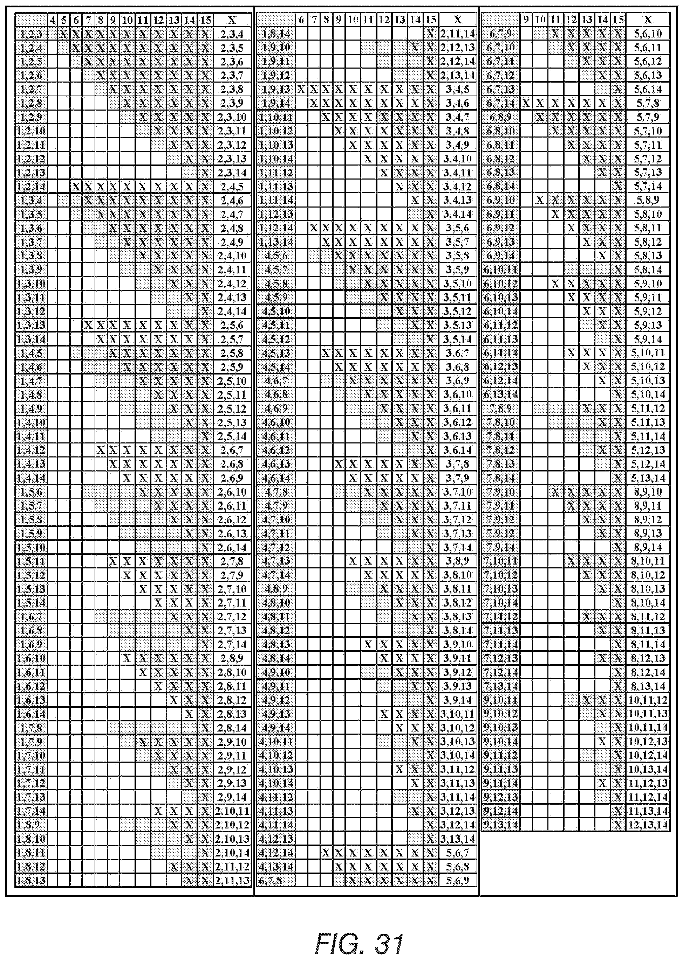

Figure 31 represents the number of unique 4 drug combinations that can be made from 15 individual drugs. The 3 drug combinations listed on the left side of each column will be combined with the individual drug listed at the top of the columns for each box that is shaded. The 3 drug combinations listed on the right side of each column will be combined with the individual drug listed at the top of the columns for each box that contains an 'X'. This gives a total of 1365 unique combinations of 4 drugs.

### EXAMPLE 22: Design of a PM Test using a Tugging System

The throughput of the ex vivo personalized medicine tests can be further increased by labeling wells containing different drug compositions with fluorescent probes. Labeled wells can be merged and passed together through a flow cytometer, saving time relative to the evaluation of each well individually. The savings achieved can approximately equal the number of wells merged. Saving time enables testing more drug compositions in less time, enabling more tests to be performed per ExviTech platform per unit time. This translates to an increase in throughput and a decrease in costs which could be very significant.

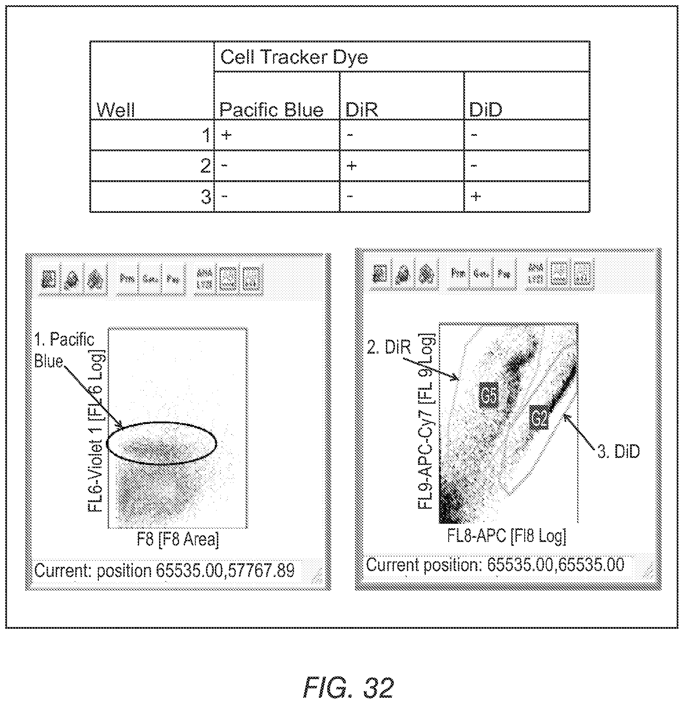

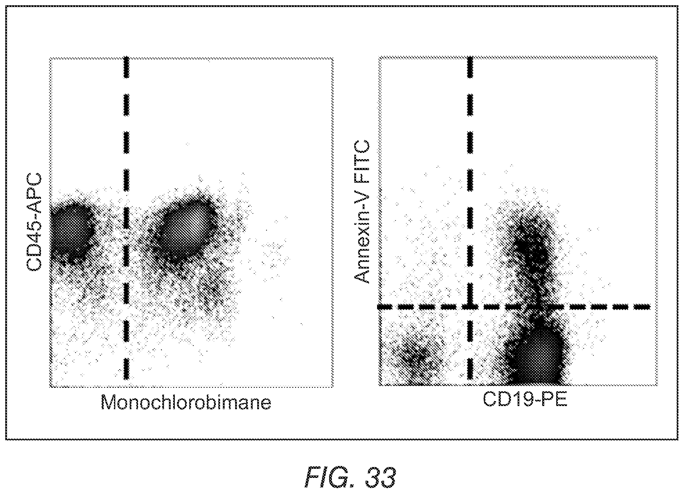

Figures 32 and 33 show examples of multiplexing using fluorochrome dyes. In an embodiment, the fluorochrome dyes are used as reagents to label cells in different drug compositions that are, *e.g.,* contained in different wells. In another embodiment, the fluorochrome dyes are used to label antibodies which are then used as reagents to label cells in the presence of different drug compositions that are, *e.g.,* contained in different wells. In another embodiment, the fluorescence reagents are quantum dots used to label cells in different drug compositions that are, *e.g.,* contained in different wells. In an embodiment, the number of drug compositions that can be evaluated in multiplexing mode is about 2, about 5, about 10, about 20, about 30, about 40, or about or more than 50, or a range defined by any two of the preceding values.

Figure 32 depicts 3 color multiplexing of peripheral blood leukocytes using different cell tracker dyes. Three consecutive wells containing lysed peripheral blood were stained individually with different cell tracker dyes. Well 1 was stained with Pacific Blue (P22652) (Invitrogen, Carlsbad, CA), well 2 was stained with DiR (D12731) (Invitrogen, Carlsbad, CA) and well 3 was stained with DiD (V-22889) (Invitrogen, Carlsbad, CA). The contents of the three wells were then mixed and acquired simultaneously. The unique excitation/emission spectra of each cell tracker dye allows for the separation of three distinct cell populations reflecting three different wells of origin.

The cells from well 1 show a stronger signal in the violet laser detector than the cells from wells 2 and 3. Conversely, the cells from wells 2 and 3 show a stronger signal in the red laser detectors compared to the cells from well 1. Finally, the cells from wells 2 and 3 show different emission peaks, allowing their separation on a bivariate plot of both red laser detectors.
1 Leone et al., Cancer Invest 1991, 9:491-503.
2. Bosanquet et al., Lancet 1991, 337: 711-716.
3. Hongo et al. et al., Cancer 1990; 65: 1263-1272.
4. Hongo et al. et al., eds. Drug resistance in leukemia and lymphoma. Langhorne, PA: Harwood Academic Publishers 1993, 319.
5. Kaspers et al., eds. Drug Resistance in Leukemia and Lymphoma. Chur: Harwood Academic Publishers 1993, 321-328.
6. Larsson et al., Int J Cancer 1992, 50:177-185.
7. Lathan et al., Haematol Blood Transfus 1990, 33:295-298.
8. Weisenthal LM et al., Cancer Treat Rep 1986, 70:1283-1295.
9. Weisenthal LM et al., Cancer Res 1983, 43:749-757.
10. Kirkpatric DL et al., Leuk Res 1990, 14:459-466.
11. Langker ST et al., eds. Drug Resistance in Leukemia and Lymphoma. Chur: Harwood Academic Publishers 1993, 279-291.
12. Staib P et al.. Adv Exper Med Biol 1999, 457:437-444.
13. Santini V, et al,, eds. Drug Resistance in Leukemia and Lymphoma. Chur: Harwood Academic Publishers, 1993, 365-368.
14. Sargent JM et al.,. Br J Cancer 1989, 60:206-210.
15. Stute N et al., Adv Exper Med Biol 1999, 457:445-452.
16. Larsson et al., eds. Drug Resistance in Leukemia and Lymphoma. Chur: Harwood Academic Publishers, 1993:399-407.
17. Beksac M et al., Med Oncol Tumor Pharmacother 1988, 5: 253-257.
18. Santini V et al., Hematol Oncol 1989, 7:287-93.
19. Tidefelt U Et al., Eur J Haematol 1989, 43:374-384.
20. Bosanquet et al., Br J Haematol 1999, 106: 474-476.
21. Silber et al., Blood 1994,84:3440-3446.
22. Bosanquet AG., eds. Drug Resistance in Leukemia and Lymphoma. Chur: Harwood Academic Publishers 1993, 373-383.
23. Sevin et al., Gynec Oncol 1988, 31:191-204.

## Claims

1. A method for analyzing cellular responsiveness to drugs, comprising:
a. obtaining a sample of whole blood, whole peripheral blood or whole bone marrow that has been withdrawn from a patient with a hematological neoplasm;
b. dividing the whole sample into at least 35 aliquots;
c. combining each of the at least 35 aliquots with a drug composition; and
d. measuring apoptosis or cell depletion in each of the at least 35 aliquots by flow cytometry.

2. The method of claim 1, wherein at least two of the drug compositions comprise the same drug at different concentrations.

3. The method of any of claims 1 or 2, wherein at least one of the drug compositions comprises a plurality of drugs.

4. The method of any of claims 1 to 3, wherein at least one of the drug compositions comprises a plurality of drugs that are non-cytotoxic.

5. The method of any of claims 1 to 4, wherein at least one of the drug compositions comprises a non-cytotoxic drug that is the same as or in the same therapeutic category as a drug already being administered to the patient.

6. The method of claim 5, wherein at least one of the drug compositions combines a non-cytotoxic drug and a cytotoxic drug.

7. The method of any of claims 1 to 6, wherein the analysis is completed within 72 hours of combining the aliquots with a drug composition.

8. The method of any of claims 1 to 7, wherein the number of aliquots combined with a drug composition is at least 96.

9. The method of any of claims claim 1 to 8, wherein the whole sample comprises cells from a hematological neoplasm selected from the group consisting of chronic lymphocytic leukemia, adult acute lymphoblastic leukemia, pediatric acute lymphoblastic leukemia, multiple myeloma, myelodysplastic syndrome, non-M3 acute myeloblastic leukemia, acute myeloblastic leukemia M3, non-Hodgkin's lymphoma, Hodgkin's lymphoma, and chronic myeloid leukemia.

10. The method of any of claims 1 to 9, wherein the drug composition comprises a compound selected from the group consisting of 5-Azacitidine, alemtuzumab, aminopterin, Amonafide, Amsacrine, Cast-8015, Bevacizumab, ARR Y520, arsenic trioxide, AS1413, Atra, AZD 6244, AZD1152, Banoxantrone, Behenoylara-C, Bendamustine, Bleomycin, Blinatumomab, Bortezomib, Busulfan, carboplatin, CEP-701, Chlorambucil, Chloro Deoxiadenosine, Cladribine, clofarabine, CPX-351, Cyclophosphamide, Cyclosporine, Cytarabine, Cytosine Arabinoside, Dasatinib, Daunorubicin, decitabine, Deglycosylated-ricin-A chain-conjugated anti-CD19/anti-CD22 immunotoxins, Dexamethasone, Doxorubicine, Elacytarabine, entinostat, epratuzumab, Erwinase, Etoposide, everolimus, Exatecan mesilate, flavopiridol, fludarabine, forodesine, Gemcitabine, Gemtuzumab-ozogamicin, Homoharringtonine, Hydrocortisone, Hydroxycarbamide, Idarubicin, Ifosfamide, Imatinib, interferon alpha 2a, iodine 1131 monoclonal antibody BC8, Iphosphamide, isotretinoin, Laromustine, L-Asparaginase, Lenalidomide, Lestaurtinib, Maphosphamide, Melphalan, Mercaptopurine, Methotrexate, Methylprednisolone, Methylprednisone, Midostaurin, Mitoxantrone, Nelarabine, Nilotinib, Oblimersen, Paclitaxel, panobinostat, Pegaspargase, Pentostatin, Pirarubicin, PKC412, Prednisolone, Prednisone, PSC-833, Rapamycin, Rituximab, Rivabirin, Sapacitabine, Dinaciclib, Sorafenib, Sorafenib, STA-9090, tacrolimus, tanespimycin, temsirolimus, Teniposide, Terameprocol, Thalidomide, Thioguanine, Thiotepa, Tipifarnib, Topotecan, Treosulfan, Troxacitabine, Vinblastine, Vincristine, Vindesine, Vinorelbine, Voreloxin, Vorinostat, Etoposide, Zosuquidar, and combinations thereof.

11. The method of any of claims 1 to 10, wherein the drug composition comprises a compound selected from the group consisting of Aluminum Oxide Hydrate, Lorazepam, Amikacine, Meropenem, Cefepime, Vancomycin, Teicoplanin, Ondansetron, Dexamethasone, Amphotericin B (liposomal), Caspofugin, Itraconazole, Fluconazole, Voriconazole, Trimetoprime, sulfamethoxazole, G-CSF, Ranitidine, Rasburicase, Paracetamol, Metamizole, Morphine chloride, Omeprazole, Paroxetine, Fluoxetine, Sertraline and combinations thereof.

12. The method of any of claims 1 to 11, wherein each of the at least 35 aliquots contains 500 or more diseased or neoplastic cells per well.

13. The method of any of claims 1 to 11, wherein each of the at least 35 aliquots contains 5.000 or more diseased or neoplastic cells per well.

14. The method of any of claims 1 to 11, wherein each of the at least 35 aliquots contains 10.000 or more diseased or neoplastic cells per well.

15. The method of any of claims 1 to 11, wherein each of the at least 35 aliquots contains 20.000 or more diseased or neoplastic cells per well.

16. The method of any of claims 1 to 11, wherein each of the at least 35 aliquots contains 40.000 or more diseased or neoplastic cells per well.

## Patentansprüche

1. Eine Methode zur Analyse der Zellreaktion auf Arzneimittel, einschließlich:
a. der Entnahme einer Vollblutprobe, einer reinen peripheren Blutprobe oder reinen Knochenmarkprobe von einem an hämatologischer Neoplasie leidenden Patienten;
b. der Aufteilung der gesamten Probe in mindestens 35 Teilproben;
c. der Beigabe einer Arzneimittelmischung zu jeder der mindestens 35 Teilproben; sowie
d. der Messung der Apoptose oder des Zellabbaus in jeder der mindestens 35 Teilproben mittels Durchflusszytometrie.

2. Das Verfahren nach Anspruch 1, wobei mindestens zwei der Arzneimittelmischungen denselben Arzneistoff in unterschiedlicher Konzentration enthalten.

3. Das Verfahren nach Anspruch 1 oder 2, wobei mindestens eine der Arzneimittelmischungen aus einer Vielzahl von Arzneimitteln besteht.

4. Das Verfahren nach Anspruch 1 bis 3, wobei mindestens eine der Arzneimittelmischungen aus einer Vielzahl nicht zytotoxischer Arzneimittel besteht.

5. Das Verfahren nach Anspruch 1 bis 4, wobei mindestens eine der Arzneimittelmischungen den selben oder der selben therapeutischen Kategorie angehörenden, nicht zytotoxischen Arzneistoff enthält, der dem Patienten bereits verabreicht wird.

6. Das Verfahren nach Anspruch 5, wobei mindestens eine der Arzneistoffmischungen aus einer Kombination aus einem nicht zytotoxischen und einem zytotoxischen Arzneistoff besteht.

7. Das Verfahren nach Anspruch 1 bis 6, wobei die Analyse innerhalb von 72 Stunden nach der Beigabe der Arzneimittelmischung zu den Teilproben abgeschlossen ist.

8. Das Verfahren nach Anspruch 1 bis 7, wobei sich die Anzahl an Teilproben zusammen mit der Arzneimittelmischung auf mindestens 96 beläuft.

9. Das Verfahren nach Anspruch 1 bis 8, wobei die gesamte Probe Zellen einer hämatologischen Neoplasie, ausgewählt aus der Gruppe bestehend aus chronischer lymphatischer Leukämie, akuter lymphatischer Leukämie in Erwachsenen, akuter lymphatischer Leukämie in Kindern, multiplen Myelomen, Myelodysplastischem Syndrom, nicht M3 akuter Myeloblastenleukämie, M3 akuter Myeloblastenleukämie, Non-Hodgkin-Lymphom, Hodgkin-Lymphom und chronischer myeloischer Leukämie beinhaltet.

10. Das Verfahren nach Anspruch 1 bis 9, wobei die Arzneimittelmischung einen Bestandteil, ausgewählt aus der Gruppe bestehend aus 5-Azacitidin, Alemtuzumab, Aminopetrin, Amonafide, Amsacrin, CAT-8015, Bevacizumab, ARR Y520, Arsen(III)-oxid, AS1413, Tretinoin, AZD 6244, AZD1152, Banoxantron, Behenoyl-ara-C, Bendamustin, Bleomycin, Blinatumomab, Bortezomib, Busulfan, Carboplatin, CEP-701, Chlorambucil, Chlorodeoxyadenosine, Cladribin, Clofarabin, CPX-351, Cyclophosphamid, Ciclosporin, Cytarabin, Arabinosylcytosin, Dasatinib, Daunorubicin, Decitabin, entglykosylierte Ricin-A konjugierte anti-CD19-/anti-CD22-Immuntoxine, Dexamethason, Doxorubicin, Elacytrabine, Entinostat, Epratuzumab, Erwinase, Etoposid, Everolimus, Exatecan Mesylate, Flavopiridol, Fludarabin, Forodesin, Gemcitabin, Gemtuzumab-Ozogamicin, Homoharringtonin, Hydrocortison, Hydroxicarbamid, Idarubicin, Ifosfamid, Imatinib, Interferon alfa-2a, Jod 1131 monoklonaler Antikörper BC8, Ifosfamid, Isotretinoin, Laromustine, L-Asparaginase, Lenalidomid, Lestaurtinib, Maphosphamid, Melphalan, Mercaptopurin, Methotrexat, Methylprednisolon, Methylprednison, Midostaurin, Mitoxantron, Nelarabin, Nilotinib, Oblimersen, Paclitaxel, Panobinostat, Pegaspargase, Pentostatin, Pirarubizin, PKC412, Prednisolon, Prednison, PSC-833, Rapamycin, Rituximab, Rivabirin, Sapacitabine, Dinaciclib, Sorafenib, Sorafenib, STA-9090, Tacrolimus, Tanespimycin, Temsirolimus, Teniposid, Terameprocol, Thalidomid, Tioguanin, Thiotepa, Tipifarnib, Topotecan, Treosulfan, Troxacitabin, Vinblastin, Vincristin, Vindesin, Vinorelbin, Voreloxin, Vorinostat, Etoposid, Zosuquidar sowie Kombinationen hieraus, beinhaltet.

11. Das Verfahren nach Anspruch 1 bis 10, wobei die Arzneimittelmischung einen Bestandteil aus der Gruppe, bestehend aus Aluminiumhydroxid, Lorazepam, Amikacin, Meropenem, Cefepim, Vancomycin, Teicoplanin, Ondansetron, Dexamethason, Amphothericin B (liposomal), Caspofungin, Itraconazol, Fluconazol, Voriconazol, Trimethoprim, Sulfamethoxazol, G-CSF, Ranitidin, Rasburicase, Paracetamol, Metamizol, Morphinchlorid, Omeprazol, Paroxetin, Fluoxetin, Sertralin sowie Kombinationen hieraus, enthält.

12. Das Verfahren nach Anspruch 1 bis 11, wobei jede der mindestens 35 Teilproben 500 oder mehr erkrankte bzw. neoplastische Zellen je Well enthält.

13. Das Verfahren nach Anspruch 1 bis 11, wobei jede der mindestens 35 Teilproben 5.000 oder mehr erkrankte bzw. neoplastische Zellen je Well enthält.

14. Das Verfahren nach Anspruch 1 bis 11, wobei jede der mindestens 35 Teilproben 10.000 oder mehr erkrankte bzw. neoplastische Zellen je Well enthält.

15. Das Verfahren nach Anspruch 1 bis 11, wobei jede der mindestens 35 Teilproben 20.000 oder mehr erkrankte bzw. neoplastische Zellen je Well enthält.

16. Das Verfahren nach Anspruch 1 bis 11, wobei jede der mindestens 35 Teilproben 40.000 oder mehr erkrankte bzw. neoplastische Zellen je Well enthält.

## Revendications

1. Un procédé pour analyser la réactivité des cellules à des médicaments, comprenant :
a. l'obtention d'un échantillon de sang total, de sang périphérique total ou de moelle osseuse totale qui a été prélevé chez un patient présentant une néoplasie hématologique ;
b. la division de la totalité de l'échantillon en 35 aliquotes au moins ;
c. associer chacune des 35 aliquotes au moins à une composition médicamenteuse ; et
d. mesurer l'apoptose ou la déplétion cellulaire dans chacune des 35 aliquotes au moins par cytométrie en flux.

2. Le procédé de la revendication 1, dans lequel au moins deux des compositions médicamenteuses comprennent le même médicament à différentes concentrations.

3. Le procédé de n'importe laquelle des revendications 1 ou 2, dans lequel au moins l'une des compositions médicamenteuses comprend une pluralité de médicaments.

4. Le procédé de n'importe laquelle des revendications 1 à 3, dans lequel au moins l'une des compositions médicamenteuses comprend une pluralité de médicaments qui sont non cytotoxiques.

5. Le procédé de n'importe laquelle des revendications 1 à 4, dans lequel au moins l'une des compositions médicamenteuses comprend un médicament non cytotoxique qui est le même ou dans la même classe thérapeutique qu'un médicament étant déjà administré au patient.

6. Le procédé de la revendication 5, dans lequel au moins l'une des compositions médicamenteuses combine un médicament non cytotoxique et un médicament cytotoxique.

7. Le procédé de n'importe laquelle des revendications 1 à 6, dans lequel l'analyse est réalisée dans les 72 heures après la combinaison des aliquotes avec une composition médicamenteuse.

8. Le procédé de n'importe laquelle des revendications 1 à 7, dans lequel le nombre d'aliquotes combinées avec une composition médicamenteuse est de 96 au moins.

9. Le procédé de n'importe laquelle des revendications 1 à 8, dans lequel la totalité de l'échantillon comprend des cellules d'une néoplasie hématologique sélectionnée dans le groupe comprenant la leucémie lymphoïde chronique, la leucémie aiguë lymphoblastique de l'adulte, la leucémie aiguë lymphoblastique de l'enfant, le myélome multiple, le syndrome myélodysplasique, la leucémie aiguë myéloblastique non-M3, la leucémie aiguë myéloblastique M3, le lymphome non hodgkinien, le lymphome hodgkinien, et la leucémie myéloïde chronique.

10. Le procédé de n'importe laquelle des revendications 1 à 9, dans lequel la composition médicamenteuse comprend un composé sélectionné dans le groupe composé de 5-Azacitidine, Alemtuzumab, aminoptérine, Amonafide, Amsacrine, CAT-8015, Bévacizumab, ARR Y520, trioxyde d'arsenic, AS1413, Atra, AZD 6244, AZD1152, Banoxantrone, Behenoylara-C, bendamustine, bléomycine, blinatumomab, bortézomib, busulfan, carboplatine, CEP-701, chlorambucil, chlorodeoxyadenosine, cladribine, clofarabine, CPX-351, cyclophosphamide, ciclosporine, cytarabine, cytosine arabinoside, dasatinib, daunorubicine, décitabine, chaîne a de ricine déglycosylée conjuguée immunotoxines anti-CD19/anti-CD22, dexaméthasone, doxorubicine,
Elacytarabine, entinostat, Epratuzumab, Erwinase, étoposide, évérolimus, exatecan mesylate, flavopiridol, fludarabine, forodésine, gemcitabine, gemtuzumab ozogamicine, Homoharringtonine, hydrocortisone, hydroxycarbamide, idarubicine, ifosfamide, imatinib, interféron alfa-2a, iode 1131 anticorps monoclonal BC8, ifosfamide, isotrétinoïne, laromustine, L-Asparaginase, lénalidomide, Lestaurtinib, maphosphamide, melphalan, mercaptopurine, méthotrexate, méthylprednisolone, méthylprednisone, midostaurine, mitoxantrone, nélarabine, nilotinib, Oblimersen, paclitaxel, panobinostat, pégaspargase, pentostatine, pirarubicine, PKC412, prednisolone, prednisone, PSC-833, rapamycine, rituximab, ribavirine, sapacitabine, Dinaciclib, sorafénib, sorafénib, STA-9090, tacrolimus, tanespimycine, temsirolimus, téniposide, terameprocol, thalidomide, Thioguanine, thiotépa, Tipifarnib, Topotécan, tréosulfan, troxacitabine, vinblastine, vincristine, vindésine, vinorelbine, Voreloxin, vorinostat, étoposide, zosuquidar, et leurs combinaisons.

11. Le procédé de n'importe laquelle des revendications 1 à 10, dans lequel la composition médicamenteuse comprend un composé sélectionné dans le groupe composé d'hydrate d'oxyde d'aluminium, lorazépam, amikacine, méropénem, céfépime, vancomycine, téicoplanine, ondansétron, dexaméthasone, amphotéricine B (liposomale), caspofungine, Itraconazole, fluconazole, voriconazole, triméthoprime, sulfaméthoxazole, G-CSF, ranitidine, rasburicase, paracétamol, métamizole, chlorure de morphine, oméprazole, paroxétine, fluoxétine, sertraline et leurs combinaisons.

12. Le procédé de n'importe laquelle des revendications 1 à 11, dans lequel chacune des 35 aliquotes au moins contient 500 ou plus de 500 cellules malades ou néoplasiques par puits.

13. Le procédé de n'importe laquelle des revendications 1 à 11, dans lequel chacune des 35 aliquotes au moins contient 5 000 ou plus de 5 000 cellules malades ou néoplasiques par puits.

14. Le procédé de n'importe laquelle des revendications 1 à 11, dans lequel chacune des 35 aliquotes au moins contient 10 000 ou plus de 10 000 cellules malades ou néoplasiques par puits.

15. Le procédé de n'importe laquelle des revendications 1 à 11, dans lequel chacune des 35 aliquotes au moins contient 20 000 ou plus de 20 000 cellules malades ou néoplasiques par puits.

16. Le procédé de n'importe laquelle des revendications 1 à 11, dans lequel chacune des 35 aliquotes au moins contient 40 000 ou plus de 40 000 cellules malades ou néoplasiques par puits.
